# EUROPEAN PATENT APPLICATION

(11) **EP 2 154 157 A2**
(43) Date of publication of application: **17.02.2010**
(21) Application number: 09155531.8
(22) Date of filing: 10.01.2005
(51) Int. Cl.: C07K 16/00, A61K 39/395

(54) **FC region variants**

(30) Priority: 12.01.2004 US 535764 P
(62) Divisional of application: 05704868.8
(71) Applicant: Applied Molecular Evolution Inc., San Diego CA 92121 (US)
(72) Inventor: Allan, Barrett, Encinitas, CA 92924 (US); Marquis, David Matthew, Encinitas, CA 92024 (US); Tang, Ying, San Diego, CA 92129 (US); Watkins, Jeffry Dean, Encinitas, CA 92024 (US)
(74) Representative: Ingham, Stephen H.

(57) **Abstract**

The present invention provides polypeptide Fc region variants and oligonucleotides encoding Fc region variants. Specifically, the present invention provides compositions comprising novel Fc region variants, methods for identifying useful Fc region variants, and methods for employing Fc region variants for treating disease.

## Description

### FIELD OF THE INVENTION

The present invention relates to polypeptide Fc region variants and oligonucleotides encoding Fc region variants. Specifically, the present invention provides compositions comprising novel Fc region variants, methods for identifying useful Fc region variants, and methods for employing Fc region variants (e.g. for treating disease).

### BACKGROUND OF THE INVENTION

There are five types of immunoglobulins in humans. These groups are known as IgG, IgM, IgD, IgA, and IgE, and are distinguished based on the isotypes of the heavy chain gene (gamma, mu, delta, alpha, and epsilon respectively). The most common isotype is IgG, and is composed of two identical heavy chain polypeptides and two identical light chain polypeptides (See, Figure 1). The two heavy chains are covalently linked to each other by disulfide bonds and each light chain is linked to a heavy chain by a disulfide bond (See, Figure 1). Each heavy chain contains approximately 445 amino acid residues, and each light chain contains approximately 215 amino acid residues.

Each heavy chain contains four distinct domains that are generally referred to as variable domain (VH), constant heavy domain 1 (CH1), constant heavy domain 2 (CH2), and constant heavy domain 3 (CH3) (See, Figure 1). The CH1 and CH2 domains are joined by a hinge region (inter-domain sections) that provides the Ig with flexibility. Each light chain contains two distinct domains that are generally referred to as the variable light (VL) and the constant light (CL).

The variable regions of the heavy and light chains directly bind antigen and are responsible for the diversity and specificity of Igs. Each VL and VH has three complementarity-determining regions (CDRs, also known as hyper variable regions). When the VL and VH come together through interactions of the heavy and light chain, the CDRs form a binding surface that contacts the antigen.

While the variable regions are involved in antigen binding, the heavy chain constant domains, primarily CH2 and CH3, are involved in non-antigen binding functions. This region, generally known as the Fc region, has many important functions. For example, the Fc region binds complement, which may trigger phagocytosis or complement dependent cytotoxicity (CDC). The Fc region also binds Fc receptors, which may trigger phagocytosis or antibody dependent cellular cytotoxicity (ADCC). The Fc region also plays a role in helping to maintain the immunoglobulin in circulation and interacts with protein A, which is commonly used to purify immunoglobulin.

There has recently been an effort to improve the immunogenic qualities and antigen binding characteristics of antibodies. For example, monoclonal, chimeric and humanized antibodies have been developed for immunotherapy. Examples of antibodies that have been approved for human immunotherapy, with the corresponding disease, include: RITUXAN (lymphoma), SYNAGIS (infectious disease), ZENEPAX (kidney transplant), REMICADE (Crohn's disease and rheumatoid arthritis), HERCEPTIN (breast carcinoma), and EDRECOLOMAB (colon cancer). However, there are many antibodies that have entered clinical trials that have failed to receive approval due to lack of efficacy or other associated problems.

What is needed, in order to improve the efficacy and speed up approval of additional therapeutic antibodies, are compositions and methods for altering Fc regions to generate variant polypeptides with improved properties.

### SUMMARY OF THE INVENTION

The present invention provides polypeptide Fc region variants and oligonucleotides encoding Fc region variants, and portions thereof. Specifically, the present invention provides compositions comprising novel Fc region variants, methods for identifying useful Fc region variants, and methods for employing Fc region variants.

In some embodiments, the present invention provides compositions comprising a variant (or a nucleic acid sequence encoding the variant) of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 247 in the Fc region. In particular embodiments, the variant comprises an antibody (e.g. an anti-CD20 antibody). In preferred embodiments, the amino acid modification is P247L.

In other embodiments, the present invention provides compositions comprising a variant (or a nucleic acid sequence encoding the variant) of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 251 in the Fc region. In particular embodiments, the variant comprises an antibody (e.g. an anti-CD20 antibody). In preferred embodiments, the amino acid modification is L251 F.

In other embodiments, the present invention provides compositions comprising a variant (or a nucleic acid sequence encoding the variant) of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 256 in the Fc region. In particular embodiments, the variant comprises an antibody (e.g. an anti-CD20 antibody). In preferred embodiments, the amino acid modification is T256M.

In other embodiments, the present invention provides compositions comprising a variant (or a nucleic acid sequence encoding the variant) of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 268 in the Fc region. In particular embodiments, the variant comprises an antibody (e.g. an anti-CD20 antibody). In preferred embodiments, the amino acid modification is H268E.

In other embodiments, the present invention provides compositions comprising a variant (or a nucleic acid sequence encoding the variant) of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 280 in the Fc region. In particular embodiments, the variant comprises an antibody (e.g. an anti-CD20 antibody). In preferred embodiments, the amino acid modification is D280A.

In other embodiments, the present invention provides compositions comprising a variant (or a nucleic acid sequence encoding the variant) of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 330 in the Fc region. In particular embodiments, the variant comprises an antibody (e.g. a anti-CD20 antibody). In preferred embodiments, the amino acid modification is A330K.

In other embodiments, the present invention provides compositions comprising a variant (or a nucleic acid sequence encoding the variant) of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 332 in the Fc region. In particular embodiments, the variant comprises an antibody (e.g. an anti-CD20 antibody). In preferred embodiments, the amino acid modification is 1332E.

In other embodiments, the present invention provides compositions comprising a variant (or a nucleic acid sequence encoding the variant) of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 339 in the Fc region. In particular embodiments, the variant comprises an antibody (e.g. an anti-CD20 antibody). In preferred embodiments, the amino acid modification is A339T.

In other embodiments, the present invention provides compositions comprising a variant (or a nucleic acid sequence encoding the variant) of a parent polypeptide having at least a portion of an Fc region, wherein the variant mediates depletion of target cells (e.g. B cells) in a whole blood assay more effectively than the parent polypeptide and comprises at least one amino acid modification at position 378 in the Fc region. In particular embodiments, the variant comprises an antibody (e.g. an anti-CD20 antibody). In preferred embodiments, the amino acid modification is A378D.

In other embodiments, the present invention provides compositions comprising a variant (or a nucleic acid sequence encoding the variant) of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 440 in the Fc region. In particular embodiments, the variant comprises an antibody (e.g. an anti-CD20 antibody). In preferred embodiments, the amino acid modification is S440Y.

In some embodiments, the present invention provides a peptide (containing the P247L amino acid modification) comprising the sequence shown in SEQ ID NO:15. In other embodiments, the present invention provides a nucleic acid sequence encoding a CH2 region with the P247L modification (e.g. SEQ ID NO:40). In some embodiments, the present invention provides an amino acid sequence encoding a CH2 region with the P247L modification comprising SEQ ID NO:15. In certain embodiments, the present invention provides a peptide (containing the L251 F modification) with the sequence shown in SEQ ID NO: 16. In additional embodiments, the present invention provides a nucleic acid sequence encoding a CH2 region with the L251 F modification (e.g. SEQ ID NO:41). In additional embodiments, the present invention provides an amino acid sequence encoding a CH2 region with the L251 F modification comprising SEQ ID NO:16.

In some embodiments, the present invention provides a peptide (containing the T256M amino acid modification) comprising the sequence shown in SEQ ID NO:17. In other embodiments, the present invention provides a nucleic acid sequence encoding a CH2 region with the T256M modification (e.g. SEQ ID NO:42). In some embodiments, the present invention provides an amino acid sequence encoding a CH2 region with the T256M modification comprising SEQ ID NO:17. In certain embodiments, the present invention provides a peptide (containing the H268E modification) comprising the sequence shown in SEQ ID NO:20. In additional embodiments, the present invention provides a nucleic acid sequence encoding a CH2 region with the H268E modification (e.g. SEQ ID NO:45). In additional embodiments, the present invention provides an amino acid sequence encoding a CH2 region with the H268E modification comprising SEQ ID NO:20.

In some embodiments, the present invention provides a peptide (containing the D280A amino acid modification) comprising the sequence shown in SEQ ID NO:21. In other embodiments, the present invention provides a nucleic acid sequence encoding a CH2 region with the D280A modification (e.g. SEQ ID NO:46). In some embodiments, the present invention provides an amino acid sequence encoding a CH2 region with the D280A modification comprising SEQ ID NO:21. In certain embodiments, the present invention provides a peptide (containing the A330K modification) comprising the sequence shown in SEQ ID NO:23. In additional embodiments, the present invention provides a nucleic acid sequence encoding a CH2 region with the A330K modification (e.g. SEQ ID NO:48). In additional embodiments, the present invention provides an amino acid sequence encoding a CH2 region with the A330K modification comprising SEQ ID NO:23.

In some embodiments, the present invention provides a peptide (containing the I332E amino acid modification) comprising the sequence shown in SEQ ID NO:26. In other embodiments, the present invention provides a nucleic acid sequence encoding a CH2 region with the I332E modification (e.g. SEQ ID NO:51). In some embodiments, the present invention provides an amino acid sequence encoding a CH2 region with the I332E modification comprising SEQ ID NO:26. In certain embodiments, the present invention provides a peptide (containing the A339T modification) comprising the sequence showin in SEQ ID NO:29. In additional embodiments, the present invention provides a nucleic acid sequence encoding a CH2 region with the A339T modification (e.g. SEQ ID NO:54). In additional embodiments, the present invention provides an amino acid sequence encoding a CH2 region with the A339T modification comprising SEQ ID NO:29.

In some embodiments, the present invention provides a peptide (containing the A378D amino acid modification) comprising the sequence shown in SEQ ID NO:30. In other embodiments, the present invention provides a nucleic acid sequence encoding a CH2 region with the A378D modification (e.g. SEQ ID NO:55). In some embodiments, the present invention provides an amino acid sequence encoding a CH2 region with the S440Y modification comprising SEQ ID NO:30.

In some embodiments, the present invention provides a peptide (containing the S440Y amino acid modification) comprising the sequence showin in SEQ ID NO:31. In other embodiments, the present invention provides a nucleic acid sequence encoding a CH2 region with the S440Y modification (e.g. SEQ ID NO:56). In some embodiments, the present invention provides an amino acid sequence encoding a CH2 region with the S440Y modification comprising SEQ ID NO:31.

In some embodiments, the present invention provides compositions comprising a variant (or a nucleic acid sequence encoding the variant) of a parent polypeptide having at least a portion of an Fc region, wherein the variant comprises at least one amino acid modification at position 247 in the Fc region selected from P247H, P247I and P247L. In other embodiments, the present invention provides compositions comprising a variant (or a nucleic acid sequence encoding the variant) of a parent polypeptide having at least a portion of an Fc region, wherein the variant comprises at least one amino acid modification at position 251 in the Fc region selected from L251 F. In other embodiments, the present invention provides compositions comprising a variant (or a nucleic acid sequence encoding the variant) of a parent polypeptide having at least a portion of an Fc region, wherein the variant comprises at least one amino acid modification at position 256 in the Fc region selected from T256M and T256P. In other embodiments, the present invention provides compositions comprising a variant (or a nucleic acid sequence encoding the variant) of a parent polypeptide having at least a portion of an Fc region, wherein the variant comprises at least one amino acid modification at position 268 in the Fc region selected from H268D and H268E. In other embodiments, the present invention provides compositions comprising a variant (or a nucleic acid sequence encoding the variant) of a parent polypeptide having at least a portion of an Fc region, wherein the variant comprises at least one amino acid modification at position 280 in the Fc region selected from D280A and D280K. In other embodiments, the present invention provides compositions comprising a variant (or a nucleic acid sequence encoding the variant) of a parent polypeptide having at least a portion of an Fc region, wherein the variant comprises at least one amino acid modification at position 330 in the Fc region selected from A330K and A330R. In other embodiments, the present invention provides compositions comprising a variant (or a nucleic acid sequence encoding the variant) of a parent polypeptide having at least a portion of an Fc region, wherein the variant comprises at least one amino acid modification at position 332 in the Fc region selected from I332D and I332E. In other embodiments, the present invention provides compositions comprising a variant (or a nucleic acid sequence encoding the variant) of a parent polypeptide having at least a portion of an Fc region, wherein the variant comprises at least one amino acid modification at position 339 in the Fc region selected from A339T. In other embodiments, the present invention provides compositions comprising a variant (or a nucleic acid sequence encoding the variant) of a parent polypeptide having at least a portion of an Fc region, wherein the variant comprises at least one amino acid modification at position 378 in the Fc region selected from A378D. In other embodiments, the present invention provides compositions comprising a variant (or a nucleic acid sequence encoding the variant) of a parent polypeptide having at least a portion of an Fc region, wherein the variant comprises at least one amino acid modification at position 440 in the Fc region selected from S440Y.

In some embodiments, the present invention provides compositions comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 247 in the Fc region. In other embodiments, the present invention provides methods comprising; a) providing; i) a composition comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 247 in the Fc region, and ii) a subject with one or more symptoms of a disease; and b) administering the composition to the subject under conditions such that at least one of the symptoms is reduced. In particular embodiments the variant comprises an antibody or immunoadhesin, and the subject has symptoms of an antibody or immunoadhesin responsive disease.

In certain embodiments, the variant comprises at least a portion of the Fc region (e.g. 40%, 50%, 60%, 80%, or 90% or more of an Fc region containing the amino acid modification). In some embodiments, the polypeptide variants comprise a CH2 or CH3 region. In further embodiments, the compositions comprise an amino acid sequence comprising SEQ ID NO:13. In some embodiments, the compositions comprise an amino acid sequence comprising SEQ ID NO: 14. In some embodiments, the compositions comprise an amino acid sequence comprising SEQ ID NO: 15. In certain embodiments, the compositions comprise a nucleic acid sequence comprising SEQ ID NO:38 and/or SEQ ID NO:39 and or SEQ ID NO: 40 or the complement thereof, or sequences that bind to SEQ ID NOs:38, 39, or 40 under conditions of high stringency. In further embodiments, the present invention provides host cells (e.g. CHO cells), and vectors comprising SEQ ID NO:38 and/or SEQ ID NO:39 and or SEQ ID NO: 40. In particular embodiments, the present invention provides a computer readable medium, wherein the computer readable medium encodes a representation of SEQ ID NO:13, 14, 15, 38, 39, or 40.

In certain embodiments, the polypeptide variant mediates antibody-dependent cell-mediated cytotoxicity (ADCC) in the presence of effector cells more effectively than the parent polypeptide. In some embodiments, the polypeptide variant comprises an antibody or antibody fragment (e.g., polyclonal antibody, monoclonal antibody, chimeric antibody, humanized antibody, or Fc fragment). In some embodiments, the parent polypeptide comprises a human IgG Fc region. In additional embodiments, the parent polypeptide comprises a human IgG1, IgG2, IgG3, or IgG4 Fc region. In other embodiments, the parent polypeptide comprises an amino acid sequence selected from SEQ ID NOS:1-12.

In preferred embodiments, the amino acid modification is P247H, P247I, or P247L. In certain embodiments, the polypeptide variant comprises a second, third, fourth, etc. amino acid modification in the Fc region (see, e.g. Table 1). In some embodiments, the variant is a CHO-expressed polypeptide.

In some embodiments, the present invention provides compositions comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 251 in the Fc region. In other embodiments, the present invention provides methods comprising; a) providing; i) a composition comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 251 in the Fc region, and ii) a subject with one or more symptoms of a disease; and b) administering the composition to the subject under conditions such that at least one of the symptoms is reduced. In particular embodiments the variant comprises an antibody or immunoadhesin, and the subject has symptoms of an antibody or immunoadhesin responsive disease.

In certain embodiments, the variant comprises at least a portion of the Fc region (e.g. 40%, 50%, 60%, 80%, or 90% or more of an Fc region containing the amino acid modification). In some embodiments, the polypeptide variants comprise a CH2 or CH3 region. In further embodiments, the compositions comprise an amino acid sequence comprising SEQ ID NO:16. In certain embodiments, the compositions comprise a nucleic acid sequence comprising SEQ ID NO:41 or the complement thereof, or sequences that bind to SEQ ID NO:41 and under conditions of high stringency. In further embodiments, the present invention provides host cells (e.g. CHO cells), and vectors comprising SEQ ID NO:41. In particular embodiments, the present invention provides a computer readable medium, wherein the computer readable medium encodes a representation of SEQ ID NO:16 or 41.

In certain embodiments, the polypeptide variant mediates antibody-dependent cell-mediated cytotoxicity (ADCC) in the presence of effector cells more effectively than the parent polypeptide. In some embodiments, the polypeptide variant comprises an antibody or antibody fragment (e.g., polyclonal antibody, monoclonal antibody, chimeric antibody, humanized antibody, or Fc fragment). In some embodiments, the parent polypeptide comprises a human IgG Fc region. In additional embodiments, the parent polypeptide comprises a human IgG1, IgG2, IgG3, or IgG4 Fc region. In other embodiments, the parent polypeptide comprises an amino acid sequence selected from SEQ ID NOs: 1-12.

In preferred embodiments, the amino acid modification is L251F. In certain embodiments, the polypeptide variant comprises a second, third, fourth, etc. amino acid modification in the Fc region (see, e.g. Table 1). In some embodiments, the variant is a CHO-expressed polypeptide.

In some embodiments, the present invention provides compositions comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 256 in the Fc region. In other embodiments, the present invention provides methods comprising; a) providing; i) a composition comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 256 in the Fc region, and ii) a subject with one or more symptoms of a disease; and b) administering the composition to the subject under conditions such that at least one of the symptoms is reduced. In particular embodiments the variant comprises an antibody or immunoadhesin, and the subject has symptoms of an antibody or immunoadhesin responsive disease.

In certain embodiments, the variant comprises at least a portion of the Fc region (e.g. 40%, 50%, 60%, 80%, or 90% or more of an Fc region containing the amino acid modification). In some embodiments, the polypeptide variants comprise a CH2 or CH3 region. In further embodiments, the compositions comprise an amino acid sequence comprising SEQ ID NO:17. In some embodiments, the compositions comprise an amino acid sequence comprising SEQ ID NO:18. In certain embodiments, the compositions comprise a nucleic acid sequence comprising SEQ ID NO:42 and/or SEQ ID NO:43, or the complement thereof, or sequences that bind to SEQ ID NOs:42 or 43 under conditions of high stringency. In further embodiments, the present invention provides host cells (e.g. CHO cells), and vectors comprising SEQ ID NO:42 and/or SEQ ID NO:43. In particular embodiments, the present invention provides a computer readable medium, wherein the computer readable medium encodes a representation of SEQ ID NO:17, 18, 42, or 43.

In certain embodiments, the polypeptide variant mediates antibody-dependent cell-mediated cytotoxicity (ADCC) in the presence of effector cells more effectively than the parent polypeptide. In some embodiments, the polypeptide variant comprises an antibody or antibody fragment (e.g., polyclonal antibody, monoclonal antibody, chimeric antibody, humanized antibody, or Fc fragment). In some embodiments, the parent polypeptide comprises a human IgG Fc region. In additional embodiments, the parent polypeptide comprises a human IgG1, IgG2, IgG3, or IgG4 Fc region. In other embodiments, the parent polypeptide comprises an amino acid sequence selected from SEQ ID NOs:1-12.

In preferred embodiments, the amino acid modification is T256M or T256P. In certain embodiments, the polypeptide variant comprises a second, third, fourth, etc. amino acid modification in the Fc region (see, e.g. Table 1). In some embodiments, the variant is a CHO-expressed polypeptide.

In some embodiments, the present invention provides compositions comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 268 in the Fc region. In other embodiments, the present invention provides methods comprising; a) providing; i) a composition comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 268 in the Fc region, and ii) a subject with one or more symptoms of a disease; and b) administering the composition to the subject under conditions such that at least one of the symptoms is reduced. In particular embodiments the variant comprises an antibody or immunoadhesin, and the subject has symptoms of an antibody or immunoadhesin responsive disease.

In certain embodiments, the variant comprises at least a portion of the Fc region (e.g. 40%, 50%, 60%, 80%, or 90% or more of an Fc region containing the amino acid modification). In some embodiments, the polypeptide variants comprise a CH2 or CH3 region. In further embodiments, the compositions comprise an amino acid sequence comprising SEQ ID NO:19. In some embodiments, the compositions comprise an amino acid sequence comprising SEQ ID NO:20. In certain embodiments, the compositions comprise a nucleic acid sequence comprising SEQ ID NO:44 and/or SEQ ID NO:45, or the complement thereof, or sequences that bind to SEQ ID NOs:44 or 45 under conditions of high stringency. In further embodiments, the present invention provides host cells (e.g. CHO cells), and vectors comprising SEQ ID NO:44 and/or SEQ ID NO:45. In particular embodiments, the present invention provides a computer readable medium, wherein the computer readable medium encodes a representation of SEQ ID NO:19, 20, 44, or 45.

In some embodiments, the present invention provides compositions comprising a variant of a parent polypeptide having at least a portion of an Fc region, wherein the variant mediates antibody-dependent cell-mediated cytotoxicity (ADCC) in the presence of effector cells less effectively than the parent polypeptide and comprises at least one amino acid modification at position 332 in the Fc region. In particular embodiments, the at least one amino acid modification is I332K or I332R.

In certain embodiments, the polypeptide variant mediates antibody-dependent cell-mediated cytotoxicity (ADCC) in the presence of effector cells more effectively than the parent polypeptide. In some embodiments, the polypeptide variant comprises an antibody or antibody fragment (e.g., polyclonal antibody, monoclonal antibody, chimeric antibody, humanized antibody, or Fc fragment). In some embodiments, the parent polypeptide comprises a human IgG Fc region. In additional embodiments, the parent polypeptide comprises a human IgG1, IgG2, IgG3, or IgG4 Fc region. In other embodiments, the parent polypeptide comprises an amino acid sequence selected from SEQ ID NOs:1-12.

In preferred embodiments, the amino acid modification is H268D or H268E. In certain embodiments, the polypeptide variant comprises a second, third, fourth, etc. amino acid modification in the Fc region (see, e.g. Table 1). In some embodiments, the variant is a CHO-expressed polypeptide.

In some embodiments, the present invention provides compositions comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 280 in the Fc region. In other embodiments, the present invention provides methods comprising; a) providing; i) a composition comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 280 in the Fc region, and ii) a subject with one or more symptoms of a disease; and b) administering the composition to the subject under conditions such that at least one of the symptoms is reduced. In particular embodiments the variant comprises an antibody or immunoadhesin, and the subject has symptoms of an antibody or immunoadhesin responsive disease.

In certain embodiments, the variant comprises at least a portion of the Fc region (e.g. 40%, 50%, 60%, 80%, or 90% or more of an Fc region containing the amino acid modification). In some embodiments, the polypeptide variants comprise a CH2 or CH3 region. In further embodiments, the compositions comprise an amino acid sequence comprising SEQ ID NO:21. In some embodiments, the compositions comprise an amino acid sequence comprising SEQ ID NO:22. In certain embodiments, the compositions comprise a nucleic acid sequence comprising SEQ ID NO:46 and/or SEQ ID NO:47, or the complement thereof, or sequences that bind to SEQ ID NOs:46 and 47 under conditions of high stringency. In further embodiments, the present invention provides host cells (e.g. CHO cells), and vectors comprising SEQ ID NO:46 and/or SEQ ID NO:47. In particular embodiments, the present invention provides a computer readable medium, wherein the computer readable medium encodes a representation of SEQ ID NO:21, 22, 46 and 47.

In certain embodiments, the polypeptide variant mediates antibody-dependent cell-mediated cytotoxicity (ADCC) in the presence of effector cells more effectively than the parent polypeptide. In some embodiments, the polypeptide variant comprises an antibody or antibody fragment (e.g., polyclonal antibody, monoclonal antibody, chimeric antibody, humanized antibody, or Fc fragment). In some embodiments, the parent polypeptide comprises a human IgG Fc region. In additional embodiments, the parent polypeptide comprises a human IgG1, IgG2, IgG3, or IgG4 Fc region. In other embodiments, the parent polypeptide comprises an amino acid sequence selected from SEQ ID NOs:1-12.

In preferred embodiments, the amino acid modification is D280A or D280K. In certain embodiments, the polypeptide variant comprises a second, third, fourth, etc. amino acid modification in the Fc region (see, e.g. Table 1). In some embodiments, the variant is a CHO-expressed polypeptide.

In some embodiments, the present invention provides compositions comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 330 in the Fc region. In other embodiments, the present invention provides methods comprising; a) providing; i) a composition comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 330 in the Fc region, and ii) a subject with one or more symptoms of a disease; and b) administering the composition to the subject under conditions such that at least one of the symptoms is reduced. In particular embodiments the variant comprises an antibody or immunoadhesin, and the subject has symptoms of an antibody or immunoadhesin responsive disease.

In certain embodiments, the variant comprises at least a portion of the Fc region (e.g. 40%, 50%, 60%, 80%, or 90% or more of an Fc region containing the amino acid modification). In some embodiments, the polypeptide variants comprise a CH2 or CH3 region. In further embodiments, the compositions comprise an amino acid sequence comprising SEQ ID NO:23. In some embodiments, the compositions comprise an amino acid sequence comprising SEQ ID NO:24. In certain embodiments, the compositions comprise a nucleic acid sequence comprising SEQ ID NO:48 and/or SEQ ID NO:49, or the complement thereof, or sequences that bind to SEQ ID NOs:48 or 49 under conditions of high stringency. In further embodiments, the present invention provides host cells (e.g. CHO cells), and vectors comprising SEQ ID NO:48 and/or SEQ ID NO:49. In particular embodiments, the present invention provides a computer readable medium, wherein the computer readable medium encodes a representation of SEQ ID NOs:23, 24, 48 and 49.

In certain embodiments, the polypeptide variant mediates antibody-dependent cell-mediated cytotoxicity (ADCC) in the presence of effector cells more effectively than the parent polypeptide. In some embodiments, the polypeptide variant comprises an antibody or antibody fragment (e.g., polyclonal antibody, monoclonal antibody, chimeric antibody, humanized antibody, or Fc fragment). In some embodiments, the parent polypeptide comprises a human IgG Fc region. In additional embodiments, the parent polypeptide comprises a human IgG1, IgG2, IgG3, or IgG4 Fc region. In other embodiments, the parent polypeptide comprises an amino acid sequence selected from SEQ ID NOs:1-12.

In preferred embodiments, the amino acid modification is A330K or A330R. In certain embodiments, the polypeptide variant comprises a second, third, fourth, etc. amino acid modification in the Fc region (see, e.g. Table 1). In some embodiments, the variant is a CHO-expressed polypeptide.

In some embodiments, the present invention provides compositions comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 332 in the Fc region. In other embodiments, the present invention provides methods comprising; a) providing; i) a composition comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 332 in the Fc region, and ii) a subject with one or more symptoms of a disease; and b) administering the composition to the subject under conditions such that at least one of the symptoms is reduced. In particular embodiments the variant comprises an antibody or immunoadhesin, and the subject has symptoms of an antibody or immunoadhesin responsive disease.

In certain embodiments, the variant comprises at least a portion of the Fc region (e.g. 40%, 50%, 60%, 80%, or 90% or more of an Fc region containing the amino acid modification). In some embodiments, the polypeptide variants comprise a CH2 or CH3 region. In further embodiments, the compositions comprise an amino acid sequence comprising SEQ ID NO:25. In some embodiments, the compositions comprise an amino acid sequence comprising SEQ ID NO:26. In certain embodiments, the compositions comprise a nucleic acid sequence comprising SEQ ID NO:50 and/or SEQ ID NO:51, or the complement thereof, or sequences that bind to SEQ ID NOs:50 or 51 under conditions of high stringency. In further embodiments, the present invention provides host cells (e.g. CHO cells), and vectors comprising SEQ ID NO:50 and/or SEQ ID NO:51. In particular embodiments, the present invention provides a computer readable medium, wherein the computer readable medium encodes a representation of SEQ ID NO:25, 26, 50, and 51.

In certain embodiments, the polypeptide variant mediates antibody-dependent cell-mediated cytotoxicity (ADCC) in the presence of effector cells more effectively than the parent polypeptide. In some embodiments, the polypeptide variant comprises an antibody or antibody fragment (e.g., polyclonal antibody, monoclonal antibody, chimeric antibody, humanized antibody, or Fc fragment). In some embodiments, the parent polypeptide comprises a human IgG Fc region. In additional embodiments, the parent polypeptide comprises a human IgGI, IgG2, IgG3, or IgG4 Fc region. In other embodiments, the parent polypeptide comprises an amino acid sequence selected from SEQ ID NOs:1-12.

In preferred embodiments, the amino acid modification is I332D or I332E. In certain embodiments, the polypeptide variant comprises a second, third, fourth, etc. amino acid modification in the Fc region (see, e.g. Table 1). In some embodiments, the variant is a CHO-expressed polypeptide.

In some embodiments, the present invention provides compositions comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 339 in the Fc region. In other embodiments, the present invention provides methods comprising; a) providing; i) a composition comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 339 in the Fc region, and ii) a subject with one or more symptoms of a disease; and b) administering the composition to the subject under conditions such that at least one of the symptoms is reduced. In particular embodiments the variant comprises an antibody or immunoadhesin, and the subject has symptoms of an antibody or immunoadhesin responsive disease.

In certain embodiments, the variant comprises at least a portion of the Fc region (e.g. 40%, 50%, 60%, 80%, or 90% or more of an Fc region containing the amino acid modification). In some embodiments, the polypeptide variants comprise a CH2 or CH3 region. In further embodiments, the compositions comprise an amino acid sequence comprising SEQ ID NO:29. In certain embodiments, the compositions comprise a nucleic acid sequence comprising SEQ ID NO:54, or the complement thereof, or sequences that bind to SEQ ID NO:54 under conditions of high stringency. In further embodiments, the present invention provides host cells (e.g. CHO cells), and vectors comprising SEQ ID NO:54. In particular embodiments, the present invention provides a computer readable medium, wherein the computer readable medium encodes a representation of SEQ ID NOs:29 or 54.

In certain embodiments, the polypeptide variant mediates antibody-dependent cell-mediated cytotoxicity (ADCC) in the presence of effector cells more effectively than the parent polypeptide. In some embodiments, the polypeptide variant comprises an antibody or antibody fragment (e.g., polyclonal antibody, monoclonal antibody, chimeric antibody, humanized antibody, or Fc fragment). In some embodiments, the parent polypeptide comprises a human IgG Fc region. In additional embodiments, the parent polypeptide comprises a human IgG1, IgG2, IgG3, or IgG4 Fc region. In other embodiments, the parent polypeptide comprises an amino acid sequence selected from SEQ ID NOs:1-12.

In preferred embodiments, the amino acid modification is A339T. In certain embodiments, the polypeptide variant comprises a second third, fourth, etc. amino acid modification in the Fc region (see, e.g. Table 1). In some embodiments, the variant is a CHO-expressed polypeptide.

In some embodiments, the present invention provides compositions comprising a variant of a parent polypeptide having at least a portion of an Fc region, wherein the variant mediates the depletion of target cells in a whole blood assay more effectively than the parent polypeptide and comprises at least one amino acid modification at position 378 in the Fc region. In other embodiments, the present invention provides methods comprising; a) providing; i) a composition comprising a variant of a parent polypeptide having at least a portion of an Fc region, wherein the variant mediates the depletion of target cells in a whole blood assay more effectively than the parent polypeptide and comprises at least one amino acid modification at position 378 in the Fc region, and ii) a subject with one or more symptoms of a disease; and b) administering the composition to the subject under conditions such that at least one of the symptoms is reduced. In particular embodiments the variant comprises an antibody or immunoadhesin, and the subject has symptoms of an antibody or immunoadhesin responsive disease.

In certain embodiments, the variant comprises at least a portion of the Fc region (e.g. 40%, 50%, 60%, 80%, or 90% or more of an Fc region containing the amino acid modification). In some embodiments, the polypeptide variants comprise a CH2 or CH3 region. In further embodiments, the compositions comprise an amino acid sequence comprising SEQ ID NO:30. In certain embodiments, the compositions comprise a nucleic acid sequence comprising SEQ ID NO:55, or the complement thereof, or sequences that bind to SEQ ID NO:55 under conditions of high stringency. In further embodiments, the present invention provides host cells (e.g. CHO cells), and vectors comprising SEQ ID NO:55. In particular embodiments, the present invention provides a computer readable medium, wherein the computer readable medium encodes a representation of SEQ ID NOs:30 or 55.

In certain embodiments, the polypeptide variant mediates mediates the depletion of target cells in a whole blood assay more effectively than the parent polypeptide. In some embodiments, the polypeptide variant comprises an antibody or antibody fragment (e.g., polyclonal antibody, monoclonal antibody, chimeric antibody, humanized antibody, or Fc fragment). In some embodiments, the parent polypeptide comprises a human IgG Fc region. In additional embodiments, the parent polypeptide comprises a human IgG1, IgG2, IgG3, or IgG4 Fc region. In other embodiments, the parent polypeptide comprises an amino acid sequence selected from SEQ ID NOs:1-12. In preferred embodiments, the amino acid modification is A378D. In certain embodiments, the polypeptide variant comprises a second, third, fourth, etc. amino acid modification in the Fc region (see, e.g. Table 1). In some embodiments, the variant is a CHO-expressed polypeptide.

In some embodiments, the present invention provides compositions comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 440 in the Fc region. In other embodiments, the present invention provides methods comprising; a) providing; i) a composition comprising a variant of a parent polypeptide having at least a portion of an Fc region, wherein the variant mediates antibody-dependent cell-mediated cytotoxicity (ADCC) in the presence of effector cells more effectively than the parent polypeptide and comprises at least one amino acid modification at position 440 in the Fc region, and ii) a subject with one or more symptoms of a disease; and b) administering the composition to the subject under conditions such that at least one of the symptoms is reduced. In particular embodiments the variant comprises an antibody or immunoadhesin, and the subject has symptoms of an antibody or immunoadhesin responsive disease.

In certain embodiments, the variant comprises at least a portion of the Fc region (e.g. 40%, 50%, 60%, 80%, or 90% or more of an Fc region containing the amino acid modification). In some embodiments, the polypeptide variants comprise a CH2 or CH3 region. In further embodiments, the compositions comprise an amino acid sequence comprising SEQ ID NO:31. In certain embodiments, the compositions comprise a nucleic acid sequence comprising SEQ ID NO:56, or the complement thereof, or sequences that bind to SEQ ID NO:56 under conditions of high stringency. In further embodiments, the present invention provides host cells (e.g. CHO cells), and vectors comprising SEQ ID NO:56. In particular embodiments, the present invention provides a computer readable medium, wherein the computer readable medium encodes a representation of SEQ ID NOs:31 or 56.

In certain embodiments, the polypeptide variant mediates antibody-dependent cell-mediated cytotoxicity (ADCC) in the presence of effector cells more effectively than the parent polypeptide. In some embodiments, the polypeptide variant comprises an antibody or antibody fragment (e.g., polyclonal antibody, monoclonal antibody, chimeric antibody, humanized antibody, or Fc fragment). In some embodiments, the parent polypeptide comprises a human IgG Fc region. In additional embodiments, the parent polypeptide comprises a human IgG1, IgG2, IgG3, or IgG4 Fc region. In other embodiments, the parent polypeptide comprises an amino acid sequence selected from SEQ ID NOs:1-12.

In preferred embodiments, the amino acid modification is S440Y. In certain embodiments, the polypeptide variant comprises a second, third, fourth, etc. amino acid modification in the Fc region (see, e.g. Table 1). In some embodiments, the variant is a CHO-expressed polypeptide.

In further embodiments, the compositions comprise a nucleic acid sequence encoding a variant of a parent polypeptide comprising at least a portion of an Fc region, wherein the variant mediates antibody-dependent cell-mediated cytotoxicity (ADCC) in the presence of effector cells more effectively that the parent polypeptide, and comprises at least one amino acid modification at position 247, 251, 256, 268, 280, 330, 332, 339, 378, or 440, or combinations thereof in the Fc region. In some embodiments, the compositions comprise a nucleic acid sequence encoding a variant Fc polypeptide which mediates antibody-dependent cell-mediated cytotoxicity (ADCC) in the presence of effector cells more effectively than the parent polypeptide, wherein the variant Fc polypeptide comprises an amino acid modification at amino acid position 247, 251, 256, 268, 280, 330, 332, 339, 378, or 440, or combinations thereof.

In further embodiments, the compositions comprise a nucleic acid sequence encoding a variant of a parent polypeptide comprising at least a portion of an Fc region, wherein the variant mediates the depletion of target cells in a whole blood assay more effectively than the parent polypeptide, and comprises at least one amino acid modification at position 247, 251, 256, 268, 280, 330, 332, 339, 378, or 440, or combinations thereof in the Fc region. In some embodiments, the compositions comprise a nucleic acid sequence encoding a variant Fc polypeptide which mediates the depletion of target cells in a whole blood assay more effectively than the parent polypeptide, wherein the variant Fc polypeptide comprises an amino acid modification at amino acid position 247, 251, 256, 268, 280, 330, 332, 339, 378, or 440, or combinations thereof.

In certain embodiments, the variants of the present invention, and the nucleic acid sequences encoding the variants, are provided with at least one other component in a kit. For example, a kit may comprise at least one type of variant, and written instructions for using the variant. The kit may also contain buffers, and other useful reagents.

In some embodiments, the present invention provides methods comprising, a) providing; i) cells expressing target antigen (CD20, for eg.), ii) a composition comprising a variant of a parent polypeptide having at least a portion of an Fc region, wherein the variant comprises at least one amino acid modification in the Fc region, and iii) effector cells (enriched PBMCs from human donor(s), for example), and b) contacting the target cells with the composition and effector cells under conditions such that the variant binds to the target cells (e.g. via a ligand expressed on the cell surface) and c) measuring killing of the target cells (release of LDH or chromium 51, for example).

In other embodiments, the present invention provides methods comprising, a) providing; i) whole blood samples containing cells expressing target antigen (B cells expressing CD20, for e.g.) and ii) a composition comprising a variant of a parent polypeptide having at least a portion of an Fc region, wherein the variant comprises at least one amino acid modification in the Fc region and b) contacting the target cells with the composition under conditions such that the variant binds to the target cells (e.g. via the CD20 ligand expressed on the cell surface) and c) measuring the disappearance of the target cells (Facs analysis with other B cell marker, such as CD19, for example).

In some embodiments, the present invention provides methods of identifying dual-species improved variants, comprising; a) providing; i) target cells, ii) a composition comprising a candidate variant of a parent polypeptide having an Fc region, wherein the candidate variant comprises at least one amino acid modification in the Fc region, and wherein the candidate variant mediates target cell cytotoxicity in the presence of a first species of effector cells more effectively than the parent polypeptide, and iii) second species effector cells, and b) incubating the composition with the target cells under conditions such that the candidate variant binds the target cells thereby generating candidate variant bound target cells, c) mixing the second species effector cells with the candidate variant bound target cells, d) measuring target cell cytotoxicity (e.g. mediated by the candidate variant), e) determining if the candidate variant mediates target cell cytotoxicity in the presence of the second species effector cells more effectively than the parent polypeptide. In some embodiments, the method further comprises screening the parent polypeptide in the same fashion with the second species effector cells. In further embodiments, steps b) and c) are performed simultaneously. In particular embodiments, the method further comprises step f) identifying a candidate variant as a dual-species improved variant that mediates target cell cytotoxicity in the presence of the second species effector cells more effectively than the parent polypeptide. In other embodiments, the method further comprises step f) identifying a candidate variant as a dual-species improved variant that mediates target cell cytotoxicity in the presence of the second species effector cells about 1.2 times (or about 1.5 times, 5 times, or about 10 times) more effectively than the parent polypeptide (e.g. about 1.2 times more target cell lysis is observed).

In other embodiments, the present invention provides methods of identifying dual-species improved variants, comprising; a) providing; i) target cells, ii) a composition comprising a candidate variant of a parent polypeptide having an Fc region, wherein the candidate variant comprises at least one amino acid modification in the Fc region, iii) first species effector cells, and iv) second species effector cells, and b) incubating the composition with the target cells under conditions such that the candidate variant binds the target cells thereby generating candidate variant bound target cells, c) mixing the first species effector cells with the candidate variant bound target cells, d) measuring target cell cytotoxicity (e.g. mediated by the candidate variant), e) determining that the candidate variant mediates target cell cytotoxicity in the presence of the first species effector cells more effectively than the parent polypeptide, f) mixing the second species effector cells with the candidate variant bound target cells (e.g. as generated in step b), g) measuring target cell cytotoxicity (e.g. mediated by the candidate variant), h) determining if the candidate variant mediates target cell cytotoxicity in the presence of the second species effector cells more effectively than the parent polypeptide.

In particular embodiments, the method further comprises a step to determine the ability of the parent polypeptide to mediate target cell cytotoxicity in the presence of the first species and/or the second species. For example, the methods may further comprise mixing the first or second species effector cells with parent polypeptide bound target cells, and then measuring target cell cytotoxicity (e.g. determining a value such that there is a value to compare the variants against).

In certain embodiments, the method further comprises step g) administering the dual-species improved variant to a test animal, wherein the test animal is a member of the second species. In other embodiments, the method further comprises, prior to step a), a step of screening the candidate variant in an Fc receptor (FcR) binding assay. In certain embodiments, the FcR binding assay is an Fc neonatal receptor (FcRn) binding assay.

In some embodiments, the method further comprises, prior to step a), a step of screening the candidate variant in a CDC assay (See, e.g., section IV below). In some embodiments, the first species of effector cells are human peripheral blood mononuclear cells (PBMCs). In other embodiments, the first species of effector cells are mouse PBMCs or rat PBMCs. In certain embodiments, the second species of effector cells are mouse PBMCs or rat PBMCs. In particular embodiments, the second species of effector cells are human PBMCs.

In some embodiments, the present invention provides methods of identifying dual-species improved variants, comprising; a) providing; i) a composition comprising a candidate variant of a parent polypeptide having an Fc region, wherein the candidate variant comprises at least one amino acid modification in the Fc region, and wherein the candidate variant mediates CDC more effectively than the parent polypeptide, and iii) a second species source of complement, and b) incubating the composition with the second species of complement; and c) determining if the candidate variant mediates CDC more effectively than the parent polypeptide. In particular embodiments, the method further comprises step d) identifying a candidate variant as a dual-species improved variant.

In some embodiments, the target cells are human cells (e.g. over-expressing one or more of the following tumor-associated antigens: CD20, CD22, CD33, CD40, CD63, EGF receptor, her-2 receptor, prostate-specific membrane antigen, Lewis Y carbohydrate, GD₂ and GD₃ gangliosides, lamp-1, CO-029, L6, and ephA2). In certain embodiments, the variant comprises an antibody, or portion thereof, specific for the target cells. In other embodiments, the candidate variant mediates target cell cytoxicity in the presence of the first species of effector cells about 1.2 times more effectively than the parent polypeptide. In some embodiments, step e) comprises performing a control reaction with the parent polypeptide. In additional embodiments, the measuring comprises quantitating target cell death or target cell lysis. In other embodiments, the target cells infected with viruses (e.g. HIV, CMV, hepatitis B, or RSV, for example) or microbial organisms (e.g. Staphylococcus, Streptococcus, Pseudomonas, etc). In certain embodiments, the target cells are microbial organisms (e.g. Staphylococcus, Streptococcus, Pseudomonas, etc). In some embodiments, the target cells are replaced instead with viruses (e.g. HIV, CMV, hepatitis B, or RSV).

In some embodiments, the present invention provides compositions comprising a CD20 binding molecule, or a nucleic acid sequence encoding a CD20 binding molecule, wherein the CD20 binding molecule comprises a light chain variable region, and wherein the light chain variable region comprises the amino acid sequence shown in SEQ ID NO:57. In other embodiments, the present invention provides compositions composition comprising a CD20 binding molecule, or a nucleic acid sequence encoding a CD20 binding molecule, wherein the CD20 binding molecule comprises a heavy chain variable region, and wherein the heavy chain variable region comprises the amino acid sequence shown in SEQ ID NO:58. In certain embodiments, the present invention provides compositions comprising a CD20 binding molecule, or a nucleic acid sequence encoding a CD20 binding molecule (See, e.g. Figure 16), wherein the CD20 binding molecule comprises a light chain variable region and a heavy chain variable region, wherein the light chain variable region comprises the amino acid sequence shown in SEQ ID NO:57 (See Figure 15), and wherein the heavy chain variable regin comprises the amino acid sequence shown in SEQ ID NO:58 (See Figure 15).

In some embodiments, the present invention provides compositions comprising a polypeptide, wherein the polypeptide comprises; i) an unmodified human framework (e.g. no alterations have been made to a naturally occurring human framework), and ii) a variant Fc region. In certain embodiments, the unmodified human framework is a human germline framework. In other embodiments, the present invention provides compositions comprising a polypeptide, wherein the polypeptide comprises: i) at least one randomized CDR sequence and ii) a variant Fc region. In further embodiments, the present invention provides compositions comprising a polypeptide, wherein the polypeptide comprises; i) an unmodified human framework (e.g. human germline framework), ii) at least one randomized CDR sequence, and iii) a variant Fc region.

### DESCRIPTION OF THE FIGURES

Figure 1 shows a schematic representation of an IgG molecule with the various regions and sections labeled.
Figure 2 shows an alignment of various parental Fc amino acid sequences, including human IgG1 (with non-a (SEQ ID NO:1) and a allotypes shown), human IgG2 (SEQ ID NO:2), human IgG3 (SEQ ID NO:3), human IgG4 (SEQ ID NO:4), murine IgG1 (SEQ ID NO:5), murine IgG2A (SEQ ID NO:6), murine IgG2B (SEQ ID NO:7), and murine IgG3 (SEQ ID NO:8).
Figure 3 shows various amino acid sequences, including the CH2 region (SEQ ID NO:9), and CH3 region (SEQ ID NO:10) of human IgG1, as well as an fallotype (SEQ ID NO:11) and a, z allotype (SEQ ID NO:12) sequences of human IgG1 that include the CH1, hinge, CH2 and CH3 regions.
Figure 4 shows the altered amino acid sequences of the variants in the context of local surrounding sequences.
Figure 5 shows nucleotide sequences encoding parental polypeptides.
Figure 6 shows the altered nucleic acid sequences of the variants in the context of local surrounding nucleic acid sequences.
Figure 7A shows the results of an ADCC assay comparing the wild type Fc region with A330K, A330R, I332E and the combination of mutations at both positions 330 and 332.
Figure 7B shows the results of an ADCC assay comparing the Fc region amino acid variants I332D and I332E to the Fc region glycan modification variant 33 (GnTIII) and the effect of amino acid plus glycan modification 33 (I332E + GnTIII) on ADCC activity.
Figure 8 shows representative ADCC data obtained using purified variant IgG.
Figure 9 shows ADCC activities of variable region variants with low affinity (6F1) and relatively high affinity (33 and 5) to CD20 antigen against Wil-2 (A) or SKW6.4 (B) target cell lines.
Figure 10 shows the titration curves of FcγRI binding to Fc-region variant IgG.
Figure 11A, B, and C show representative CDC data obtained from titration of Fc-region variants using human complement and Ramos target cells.
Figure 12 shows representative B cell depletion with AME-133 (variable region 33 with the I332E Fc variant sequence), Rituxan, and non-specific IgG using a whole blood sample containing the VV (high affinity) genotype of the FcγRIIIa receptor.
Figure 13 shows representative B cell depletion with AME-133 (variable region 33 with the I332E Fc variant sequence), Rituxan, and non-specific IgG using a whole blood sample containing the FF (low affinity) genotype of the FcγRIIIa receptor.
Figure 14 shows representative B cell depletion with Rituxan, non-specific IgG and with variants A378D and T256P, I332E using a whole blood sample.
Figure 15 shows the amino acid sequence of the light (SEQ ID NO:57) and the heavy (SEQ ID NO:58) chains of AME-133 (variable region 33 with the I332E Fc variant sequence).
Figure 16 shows the nucleotide sequence of the light (SEQ ID NO:59) and the heavy (SEQ ID NO:60) chains of AME-133 (variable region 33 with the I332E Fc variant sequence).

### DEFINITIONS

To facilitate an understanding of the invention, a number of terms are defined below.

As used herein, the terms "subject" and "patient" refer to any animal, such as a mammal like a dog, cat, bird, livestock, and preferably a human (e.g. a human with a disease).

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

DNA molecules are said to have "5' ends" and "3' ends" because mononucleotides are reacted to make oligonucleotides or polynucleotides in a manner such that the 5' phosphate of one mononucleotide pentose ring is attached to the 3' oxygen of its neighbor in one direction via a phosphodiester linkage. Therefore, an end of an oligonucleotides or polynucleotide, referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide or polynucleotide, also may be said to have 5' and 3' ends. In either a linear or circular DNA molecule, discrete elements are referred to as being "upstream" or 5' of the "downstream" or 3' elements. This terminology reflects the fact that transcription proceeds in a 5' to 3' fashion along the DNA strand. The promoter and enhancer elements that direct transcription of a linked gene are generally located 5' or upstream of the coding region. However, enhancer elements can exert their effect even when located 3' of the promoter element and the coding region. Transcription termination and polyadenylation signals are located 3' or downstream of the coding region.

As used herein, the term "codon" or "triplet" refers to a tuplet of three adjacent nucleotide monomers which specify one of the twenty naturally occurring amino acids found in polypeptide biosynthesis. The term also includes nonsense codons which do not specify any amino acid.

As used herein, the terms "an oligonucleotide having a nucleotide sequence encoding a polypeptide", "polynucleotide having a nucleotide sequence encoding a polypeptide", and "nucleic acid sequence encoding a polypeptide" means a nucleic acid sequence comprising the coding region of a particular polypeptide. The coding region may be present in a cDNA, genomic DNA, or RNA form. When present in a DNA form, the oligonucleotide or polynucleotide may be single-stranded (*i.e.*, the sense strand) or double-stranded. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, *etc.* or a combination of both endogenous and exogenous control elements.

As used herein, the terms "complementary" or "complementarity" are used in reference to polynucleotides (*i.e.*, a sequence of nucleotides) related by the base-pairing rules. For example, for the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

As used herein, the term "the complement of' a given sequence is used in reference to the sequence that is completely complementary to the sequence over its entire length. For example, the sequence A-G-T-A is "the complement" of the sequence T-C-A-T.

The term "homology" (when in reference to nucleic acid sequences) refers to a degree of complementarity. There may be partial homology or complete homology (*i.e.*, identity). A partially complementary sequence is one that at least partially inhibits a completely complementary sequence from hybridizing to a target nucleic acid and is referred to using the functional term "substantially homologous." The term "inhibition of binding," when used in reference to nucleic acid binding, refers to inhibition of binding caused by competition of homologous sequences for binding to a target sequence. The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (*i.e.*, the hybridization) of a completely homologous sequence to a target under conditions of low stringency. This is not to say that conditions of low stringency are such that non-specific binding is permitted; low stringency conditions require that the binding of two sequences to one another be a specific (*i.e.,* selective) interaction. The absence of non-specific binding may be tested by the use of a second target that lacks even a partial degree of complementarity (*e.g.*, less than about 30% identity); in the absence of non-specific binding the probe will not hybridize to the second non-complementary target.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (*e.g.,* the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (*e.g.,* increasing the temperature of the hybridization and/or wash steps, the use of formamide in the hybridization solution, etc.).

A nucleic acid sequence (*e.g.* encoding a variant Fc region or portion thereof) may produce multiple RNA species that are generated by differential splicing of the primary RNA transcript. cDNAs that are splice variants of the same gene will contain regions of sequence identity or complete homology (representing the presence of the same exon or portion of the same exon on both cDNAs) and regions of complete non-identity (for example, representing the presence of exon "A" on cDNA 1 wherein cDNA 2 contains exon "B" instead). Because the two cDNAs contain regions of sequence identity they will both hybridize to a probe derived from the entire gene or portions of the gene containing sequences found on both cDNAs; the two splice variants are therefore substantially homologous to such a probe and to each other.

When used in reference to a single-stranded nucleic acid sequence, the term "substantially homologous" refers to any probe that can hybridize (*i.e.*, it is the complement of) the single-stranded nucleic acid sequence under conditions of low stringency.

As used herein, the term "hybridization" is used in reference to the pairing of complementary nucleic acids. Hybridization and the strength of hybridization (*i.e.,* the strength of the association between the nucleic acids) is impacted by such factors as the degree of complementary between the nucleic acids, stringency of the conditions involved, the Tₘ of the formed hybrid, and the G:C ratio within the nucleic acids.

As used herein, the term "Tₘ" is used in reference to the "melting temperature." The melting temperature is the temperature at which a population of double-stranded nucleic acid molecules becomes half dissociated into single strands. The equation for calculating the Tₘ of nucleic acids is well known in the art. As indicated by standard references, a simple estimate of the Tₘ value may be calculated by the equation: Tₘ = 81.5 + 0.41(% G + C), when a nucleic acid is in aqueous solution at 1 M NaCl (*See e.g.,* Anderson and Young, Quantitative Filter Hybridization, *in Nucleic Acid Hybridization* [1985]). Other references include more sophisticated computations that take structural as well as sequence characteristics into account for the calculation of Tₘ, and in some cases the Tₘ may be determined empirically by beginning with the calculated Tₘ and testing small increases or decreases of temperature and examining the effect on the population of nucleic acid molecules.

As used herein the term "stringency" is used in reference to the conditions of temperature, ionic strength, and the presence of other compounds such as organic solvents, under which nucleic acid hybridizations are conducted. Those skilled in the art will recognize that "stringency" conditions may be altered by varying the parameters just described either individually or in concert. With "high stringency" conditions, nucleic acid base pairing will occur only between nucleic acid fragments that have a high frequency of complementary base sequences (*e.g.*, hybridization under "high stringency" conditions may occur between homologs with about 85-100% identity, preferably about 70-100% identity). With medium stringency conditions, nucleic acid base pairing will occur between nucleic acids with an intermediate frequency of complementary base sequences (*e.g.*, hybridization under "medium stringency" conditions may occur between homologs with about 50-70% identity). Thus, conditions of "weak" or "low" stringency are often required with nucleic acids that are derived from organisms that are genetically diverse, as the frequency of complementary sequences is usually less.

"High stringency conditions" when used in reference to nucleic acid hybridization comprise conditions equivalent to binding or hybridization at 42 °C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 0.1X SSPE, 1.0% SDS at 42 C when a probe of about 500 nucleotides in length is employed.

"Medium stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 1.0X SSPE, 1.0% SDS at 42 C when a probe of about 500 nucleotides in length is employed.

"Low stringency conditions" comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.1 % SDS, 5X Denhardt's reagent [50X Denhardt's contains per 500 ml: 5 g Ficoll (Type 400, Pharmacia), 5 g BSA (Fraction V; Sigma)] and 100 g/ml denatured salmon sperm DNA followed by washing in a solution comprising 5X SSPE, 0.1 % SDS at 42 C when a probe of about 500 nucleotides in length is employed.

The following terms are used to describe the sequence relationships between two or more polynucleotides: "reference sequence", "sequence identity", and "percentage of sequence identity". A "reference sequence" is a defined sequence used as a basis for a sequence comparison; a reference sequence may be a subset of a larger sequence, for example, as a segment of a full-length cDNA sequence given in a sequence listing or may comprise a complete gene sequence. Generally, a reference sequence is at least 20 nucleotides in length, frequently at least 25 nucleotides in length, and often at least 50 nucleotides in length (*e.g*.any one of SEQ ID NOs: 32-37 may be used as a reference sequence). Since two polynucleotides may each (1) comprise a sequence (*i.e.*, a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window", as used herein, refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (*i.e.,* gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman [Smith and Waterman, Adv. Appl. Math. 2: 482 (1981)] by the homology alignment algorithm of Needleman and Wunsch [Needleman and Wunsch, J. Mol. Biol. 48:443 (1970)], by the search for similarity method of Pearson and Lipman [Pearson and Lipman, Proc. Natl. Acad. Sci. (U.S.A.) 85:2444 (1988)], by computerized implementations of these algorithms (GAP, BESTFIT, FASTA, and TFASTA in the Wisconsin Genetics Software Package Release 7.0, Genetics Computer Group, 575 Science Dr., Madison, Wis.), or by inspection, and the best alignment (*i.e.*, resulting in the highest percentage of homology over the comparison window) generated by the various methods is selected. The term "sequence identity" means that two polynucleotide sequences are identical (*i.e.*, on a nucleotide-by-nucleotide basis) over the window of comparison. The term "percentage of sequence identity" is calculated by comparing two optimally aligned sequences over the window of comparison, determining the number of positions at which the identical nucleic acid base (*e.g.*, A, T, C, G, U, or I) occurs in both sequences to yield the number of matched positions, dividing the number of matched positions by the total number of positions in the window of comparison (*i.e.,* the window size), and multiplying the result by 100 to yield the percentage of sequence identity. The window of comparison, as used in the present application, is the entire length of the recited reference sequence (i. e. if SEQ ID NO:33 is recited as the reference sequence, percentage of sequence identity is compared over the entire length of SEQ ID NO:33).

As used herein, the term "probe" refers to an oligonucleotide (*i.e.,* a sequence of nucleotides), whether occurring naturally as in a purified restriction digest or produced synthetically, recombinantly or by PCR amplification, that is capable of hybridizing to another oligonucleotide of interest. A probe may be single-stranded or double-stranded. Probes are useful in the detection, identification and isolation of particular gene sequences. It is contemplated that any probe used in the present invention may be labeled with any "reporter molecule," so that is detectable in any detection system, including, but not limited to enzyme (*e.g.*, ELISA, as well as enzyme-based histochemical assays), fluorescent, radioactive, and luminescent systems. It is not intended that the present invention be limited to any particular detection system or label.

As used herein, the term "polymerase chain reaction" ("PCR") refers to the method described in U.S. Patent Nos. 4,683,195, 4,683,202, and 4,965,188, hereby incorporated by reference, that describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing, and polymerase extension can be repeated many times (*i.e.*, denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified."

With PCR, it is possible to amplify a single copy of a specific target sequence in genomic DNA to a level detectable by several different methodologies (*e.g.*, hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

The term "isolated" when used in relation to a nucleic acid, as in "an isolated oligonucleotide" or "isolated polynucleotide" refers to a nucleic acid sequence that is identified and separated from at least one contaminant nucleic acid with which it is ordinarily associated in its natural source. Isolated nucleic acid is present in a form or setting that is different from that in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the polypeptide where, for example, the nucleic acid molecule is in a chromosomal location different from that of natural cells. The isolated nucleic acid, oligonucleotide, or polynucleotide may be present in single-stranded or double-stranded form. When an isolated nucleic acid, oligonucleotide or polynucleotide is to be utilized to express a protein, the oligonucleotide or polynucleotide will contain at a minimum the sense or coding strand (*i.e.*, the oligonucleotide or polynucleotide may single-stranded), but may contain both the sense and anti-sense strands (*i.e.*, the oligonucleotide or polynucleotide may be double-stranded).

As used herein the terms "portion" when used in reference to a nucleotide sequence (as in "a portion of a given nucleotide sequence") refers to fragments of that sequence. The fragments may range in size from ten nucleotides to the entire nucleotide sequence minus one nucleotide (*e.g.*, 10 nucleotides, 20, 30, 40, 50, 100, 200, etc.).

As used herein the term "portion" when in reference to an amino acid sequence (as in "a portion of a given amino acid sequence") refers to fragments of that sequence. The fragments may range in size from six amino acids to the entire amino acid sequence minus one amino acid (*e.g.*, 6 amino acids, 10, 20, 30, 40, 75, 200, etc.).

As used herein, the term "purified" or "to purify" refers to the removal of contaminants from a sample. For example, antigen specific antibodies may be purified by removal of contaminating non-immunoglobulin proteins; they are also purified by the removal of immunoglobulin that does not bind to the same antigen. The removal of non-immunoglobulin proteins and/or the removal of immunoglobulins that do not bind a particular antigen results in an increase in the percent of antigen specific immunoglobulins in the sample. In another example, recombinant antigen specific polypeptides are expressed in bacterial host cells and the polypeptides are purified by the removal of host cell proteins; the percent of recombinant antigen specific polypeptides is thereby increased in the sample.

The term "recombinant DNA molecule" as used herein refers to a DNA molecule that is comprised of segments of DNA joined together by means of molecular biological techniques.

The term "recombinant protein" or "recombinant polypeptide" as used herein refers to a protein molecule that is expressed from a recombinant DNA molecule.

The term "native protein" as used herein to indicate that a protein does not contain amino acid residues encoded by vector sequences; that is the native protein contains only those amino acids found in the protein as it commonly occurs in nature. A native protein may be produced by recombinant means or may be isolated from a naturally occurring source.

The term "Southern blot," refers to the analysis of DNA on agarose or acrylamide gels to fractionate the DNA according to size followed by transfer of the DNA from the gel to a solid support, such as nitrocellulose or a nylon membrane. The immobilized DNA is then probed with a labeled probe to detect DNA species complementary to the probe used. The DNA may be cleaved with restriction enzymes prior to electrophoresis. Following electrophoresis, the DNA may be partially depurinated and denatured prior to or during transfer to the solid support. Southern blots are a standard tool of molecular biologists (J. Sambrook et al., Molecular Cloning: A Laboratory Manual, Cold Spring Harbor Press, NY, pp 9.31-9.58 [1989]).

The term "Northern blot," as used herein refers to the analysis of RNA by electrophoresis of RNA on agarose gels to fractionate the RNA according to size followed by transfer of the RNA from the gel to a solid support, such as nitrocellulose or a nylon membrane. The immobilized RNA is then probed with a labeled probe to detect RNA species complementary to the probe used. Northern blots are a standard tool of molecular biologists (J. Sambrook, *et al., supra,* pp 7.39-7.52 [1989]).

The term "Western blot" refers to the analysis of protein(s) (or polypeptides) immobilized onto a support such as nitrocellulose or a membrane. The proteins are run on acrylamide gels to separate the proteins, followed by transfer of the protein from the gel to a solid support, such as nitrocellulose or a nylon membrane. The immobilized proteins are then exposed to antibodies with reactivity against an antigen of interest. The binding of the antibodies may be detected by various methods, including the use of radiolabelled antibodies.

The term "antigenic determinant" as used herein refers to that portion of an antigen that makes contact with a particular antibody (i. e., an epitope). When a protein or fragment of a protein is used to immunize a host animal, numerous regions of the protein may induce the production of antibodies that bind specifically to a given region or three-dimensional structure on the protein; these regions or structures are referred to as antigenic determinants. An antigenic determinant may compete with the intact antigen (*i.e.,* the "immunogen" used to elicit the immune response) for binding to an antibody.

The term "transgene" as used herein refers to a foreign, heterologous, or autologous gene that is placed into an organism by introducing the gene into newly fertilized eggs or early embryos. The term "foreign gene" refers to any nucleic acid (*e.g.*, gene sequence) that is introduced into the genome of an animal by experimental manipulations and may include gene sequences found in that animal so long as the introduced gene does not reside in the same location as does the naturally-occurring gene. The term "autologous gene" is intended to encompass variants (*e.g.*, polymorphisms or mutants) of the naturally occurring gene. The term transgene thus encompasses the replacement of the naturally occurring gene with a variant form of the gene.

As used herein, the term "vector" is used in reference to nucleic acid molecules that transfer DNA segment(s) from one cell to another. The term "vehicle" is sometimes used interchangeably with "vector."

The term "expression vector" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

As used herein, the term "host cell" refers to any eukaryotic or prokaryotic cell (*e.g.*, bacterial cells such as *E. coli,* CHO cells, yeast cells, mammalian cells, avian cells, amphibian cells, plant cells, fish cells, and insect cells), whether located *in vitro* or *in vivo.* For example, host cells may be located in a transgenic animal.

The terms "transfection" and "transformation" as used herein refer to the introduction of foreign DNA into cells (e.g. eukaryotic and prokaryotic cells). Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

The term "stable transfection" or "stably transfected" refers to the introduction and integration of foreign DNA into the genome of the transfected cell. The term "stable transfectant" refers to a cell that has stably integrated foreign DNA into the genomic DNA.

The term "transient transfection" or "transiently transfected" refers to the introduction of foreign DNA into a cell where the foreign DNA fails to integrate into the genome of the transfected cell. The foreign DNA persists in the nucleus of the transfected cell for several days. During this time the foreign DNA is subject to the regulatory controls that govern the expression of endogenous genes in the chromosomes. The term "transient transfectant" refers to cells that have taken up foreign DNA but may have failed to integrate this DNA.

The term "calcium phosphate co-precipitation" refers to a technique for the introduction of nucleic acids into a cell. The uptake of nucleic acids by cells is enhanced when the nucleic acid is presented as a calcium phosphate-nucleic acid co-precipitate. The original technique of Graham and van der Eb (Graham and van der Eb, Virol., 52:456 [1973]), has been modified by several groups to optimize conditions for particular types of cells. The art is well aware of these numerous modifications.

A "composition comprising a given polynucleotide sequence" as used herein refers broadly to any composition containing the given polynucleotide sequence. The composition may comprise an aqueous solution. Compositions comprising polynucleotide sequences encoding, for example, a variant Fc region or fragments thereof may be employed as hybridization probes. In this case, variant Fc region encoding polynucleotide sequences are typically employed in an aqueous solution containing salts (*e.g.,* NaCl), detergents (*e.g.*, SDS), and other components (*e.g.*, Denhardt's solution, dry milk, salmon sperm DNA, etc.).

The term "test compound" or "candidate compound" refer to any chemical entity, pharmaceutical, drug, and the like that can be used to treat or prevent a disease, illness, sickness, or disorder of bodily function, or otherwise alter the physiological or cellular status of a sample. Test compounds comprise both known and potential therapeutic compounds. A test compound can be determined to be therapeutic by screening using the screening methods of the present invention. A "known therapeutic compound" refers to a therapeutic compound that has been shown (*e.g.*, through animal trials or prior experience with administration to humans) to be effective in such treatment or prevention.

As used herein, the term "response," when used in reference to an assay, refers to the generation of a detectable signal (*e.g.*, accumulation of reporter protein, increase in ion concentration, accumulation of a detectable chemical product).

As used herein, the term "reporter gene" refers to a gene encoding a protein that may be assayed. Examples of reporter genes include, but are not limited to, luciferase (*See, e.g.,* deWet et al., Mol. Cell. Biol. 7:725 [1987] and U.S. Pat Nos., 6,074,859, incorporated herein by reference), green fluorescent protein (*e.g.*, GenBank Accession Number U43284; a number of GFP variants are commercially available from CLONTECH Laboratories, Palo Alto, CA), chloramphenicol acetyltransferase, β-galactosidase, alkaline phosphatase, and horseradish peroxidase.

As used herein, the terms "computer memory" and "computer memory device" refer to any storage media readable by a computer processor. Examples of computer memory include, but are not limited to, RAM, ROM, computer chips, digital video disc (DVDs), compact discs (CDs), hard disk drives (HDD), and magnetic tape.

As used herein, the term "computer readable medium" refers to any device or system for storing and providing information (*e.g.*, data and instructions) to a computer processor. Examples of computer readable media include, but are not limited to, DVDs, CDs, hard disk drives, magnetic tape and servers for streaming media over networks.

As used herein, the phrase "computer readable medium encodes a representation" of a nucleic acid or amino acid sequence, refers to computer readable medium that has stored thereon information, that when delivered to a processor, allows the sequence of the nucleic or amino acid sequence to be displayed to a user (*e.g.* printed out or presented on a display screen).

As used herein, the terms "processor" and "central processing unit" or "CPU" are used interchangeably and refer to a device that is able to read a program from a computer memory (*e.g.*, ROM or other computer memory) and perform a set of steps according to the program.

As used herein, the numbering of amino acid residues in an immunoglobulin heavy chain uses the EU index format as in Kabat et al., Sequences of Proteins of Immunological Interest, 5th Ed. Public Health Service, National Institutes of Health, Bethesda, MD (1991), expressly incorporated herein by reference. The "EU index format as in Kabat" refers to the residue numbering of the human IgG1 EU antibody.

As used herein a "parent polypeptide" is a polypeptide comprising an amino acid sequence that may be changed or altered (e.g. an amino acid substitution, addition or deletion is made) to produce a variant. In preferred embodiments, the parent polypeptide comprises at least a portion of a naturally occurring Fc region or an Fc region with amino acid sequence modifications (e.g., additions, deletions, and/or substitutions). In some embodiments, variants that are shorter or longer than the parent polypeptide are specifically contemplated. In particularly preferred embodiments, the parent polypeptide differs in function (e.g. effector function, binding, etc.) as compared to a variant.

As used herein, the term "variant of a parent polypeptide" refers to a peptide comprising an amino acid sequence that differs from that of the parent polypeptide by at least one amino acid modification. In certain embodiments, the variant comprises at least a portion of an Fc region (e.g. at least 40%, 50%, 75%, or 90% or an Fc region). In preferred embodiments, the variant comprises an Fc region of a parent polypeptide with at least one amino acid modification.

As used herein, the term "Fc region" refers to a C-terminal region of an immunoglobulin heavy chain (e.g., as shown in Figure 1). The "Fc region" may be a native sequence Fc region or a variant Fc region. Although the generally accepted boundaries of the Fc region of an immunoglobulin heavy chain might vary, the human IgG heavy chain Fc region is usually defined to stretch from an amino acid residue at position Cys226, or from Pro230, to the carboxyl-terminus thereof. In some embodiments, variants comprise only portions of the Fc region and can include or not include the carboxyl-terminus. The Fc region of an immunoglobulin generally comprises two constant domains, CH2 and CH3, as shown, for example, in Figure 1. In some embodiments, variants having one or more of the constant domains are contemplated. In other embodiments, variants without such constant domains (or with only portions of such constant domains) are contemplated.

As used herein, the "CH2 domain" (also referred to as"Cγ2"domain) generally comprises the stretch of residues that extends from about amino acid 231 to about amino acid 340 in an Fc region (e.g. in the human IgG Fc region). The CH2 domain is unique in that it is not closely paired with another domain. Rather, two N-linked branched carbohydrate chains are interposed between the two CH2 domains of an intact native IgG molecule.

As used herein, the "CH3 domain" (also referred to as"Cγ3 "domain) generally comprises the stretch of residues C-terminal to a CH2 domain in an Fc region (e.g., from about amino acid residue 341 to about amino acid residue 447 of a human IgG Fc region).

As used herein, an Fc region may possess "effector functions" that are responsible for activating or diminishing a biological activity (e.g. in a subject). Examples of effector functions include, but are not limited to: C1q binding; complement dependent cytotoxicity (CDC); Fc receptor binding; antibody-dependent cell-mediated cytotoxicity (ADCC); phagocytosis; down regulation of cell surface receptors (e.g., B cell receptor; BCR), etc. Such effector functions may require the Fc region to be combined with a binding domain (e.g. an antibody variable domain) and can be assessed using various assays (e.g. Fc binding assay, ADCC assays, CDC assays, target cell depletion from whole or fractionated blood samples, etc.).

As used herein the term "native sequence Fc region" or "wild type Fc region" refers to an amino acid sequence that is identical to the amino acid sequence of an Fc region commonly found in nature. Exemplary native sequence human Fc regions are shown in Figure 2 and include a native sequence human IgG1 Fc region (f and a,z allotypes); native sequence human IgG2 Fc region; native sequence human IgG3 Fc region; and native sequence human IgG4 Fc region as well as naturally occurring variants thereof. Native sequence murine Fc regions are also shown in Figure 2. Other sequences are contemplated and are readily obtained from various web sites (e.g., NCBI's web site).

As used herein, the term "variant Fc region" refers to amino acid sequence that differs from that of a native sequence Fc region (or portions thereof) by virtue of at least one amino acid modification (e.g., substitution, insertion, or deletion), including heterodimeric variants in which the heavy chain subunit sequences may differ from one another. In preferred embodiments, the variant Fc region has at least one amino acid substitution compared to a native sequence Fc region (e.g. from about one to about ten amino acid substitutions, and preferably from about one to about five amino acid substitutions in a native sequence Fc region). In preferred embodiments, variant Fc regions will possess at least about 80% homology with a native sequence Fc region, preferably at least about 90% homology, and more preferably at least about 95% homology.

As used herein, the term "homology", when used in reference to amino acid sequences, refers to the percentage of residues in an amino acid sequence variant that are identical with the native amino acid sequence after aligning the sequences and introducing gaps, if necessary, to achieve the maximum percent homology.

The term "Fc region-containing polypeptide" refers to a polypeptide, such as an antibody or immunoadhesin (see definitions below), which comprises an Fc region.

The terms "Fc receptor" and "FcR" are used to describe a receptor that binds to an Fc region (e.g. the Fc region of an antibody or antibody fragment). The term includes the neonatal receptor, FcRn, which is responsible for the transfer of maternal IgGs to the fetus.

As used herein, the phrase "antibody-dependent cell-mediated cytotoxicity" and "ADCC" refer to a cell-mediated reaction in which cytotoxic cells (e.g. nonspecific) that express FcRs (e.g. Natural Killer (NK) cells, neutrophils, and macrophages) recognize bound antibody on a target cell and subsequently cause lysis of the target cells. The primary cells for mediating ADCC, NK cells, express FcγRIII, whereas monocytes express FcγRI, FcγRII and FcγRIII.

As used herein, the phrase "effector cells" refers to leukocytes which express one or more FcRs and perform effector functions. Preferably, the cells express at least FcγRIII and perform an ADCC effector function. Examples of leukocytes which mediate ADCC include peripheral blood mononuclear cells (PBMC), natural killer (NK) cells, monocytes, cytotoxic T cells and neutrophils. The effector cells may be isolated from a native source (e.g. from blood).

As used herein, the phrase "whole blood" refers to unfractionated blood samples.

As used herein, a polypeptide variant with "altered" FcR binding affinity or ADCC activity is one which has either enhanced (i.e. increased) or diminished (i.e. reduced) FcR binding activity and/or ADCC activity compared to a parent polypeptide or to a polypeptide comprising a native sequence Fc region. A polypeptide variant which "displays increased binding" to an FcR binds at least one FcR with better affinity than the parent polypeptide. A polypeptide variant which "displays decreased binding" to an FcR, binds at least one FcR with worse affinity than a parent polypeptide. Such variants which display decreased binding to an FcR may possess little or no appreciable binding to an FcR, e.g., 0-20% binding to the FcR compared to a parent polypeptide. A polypeptide variant which binds an FcR with "better affinity" than a parent polypeptide, is one which binds any one or more of the above identified FcRs with higher binding affinity than the parent antibody, when the amounts of polypeptide variant and parent polypeptide in a binding assay are essentially the same, and all other conditions are identical. For example, a polypeptide variant with improved FcR binding affinity may display from about 1.10 fold to about 100 fold (more typically from about 1.2 fold to about 50 fold) improvement (i.e. increase) in FcR binding affinity compared to the parent polypeptide, where FcR binding affinity is determined, for example, in an ELISA assay.

As used herein, an "amino acid modification" refers to a change in the amino acid sequence of a given amino acid sequence. Exemplary modifications include, but are not limited to, an amino acid substitution, insertion and/or deletion. In preferred embodiments, the amino acid modification is a substitution (e.g. in an Fc region of a parent polypeptide).

As used herein, an "amino acid modification at" a specified position (e.g. in the Fc region) refers to the substitution or deletion of the specified residue, or the insertion of at least one amino acid residue adjacent the specified residue. By insertion "adjacent" a specified residue is meant insertion within one to two residues thereof. The insertion may be N-terminal or C-terminal to the specified residue.

As used herein, an "amino acid substitution" refers to the replacement of at least one existing amino acid residue in a given amino acid sequence with another different "replacement" amino acid residue. The replacement residue or residues may be "naturally occurring amino acid residues" (i.e. encoded by the genetic code) and selected from: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val). Substitution with one or more non-naturally occurring amino acid residues is also encompassed by the definition of an amino acid substitution herein. A "non-naturally occurring amino acid residue" refers to a residue, other than those naturally occurring amino acid residues listed above, which is able to covalently bind adjacent amino acid residues (s) in a polypeptide chain. Examples of non-naturally occurring amino acid residues include norleucine, ornithine, norvaline, homoserine and other amino acid residue analogues such as those described in Ellman et al. Meth. Enzym. 202: 301-336 (1991), herein incorporated by reference.

As used herein, the term "amino acid insertion" refers to the incorporation of at least one amino acid into a given amino acid sequence. In preferred embodiments, an insertion will usually be the insertion of one or two amino acid residues. In other embodiments, the insertion includes larger peptide insertions (e.g. insertion of about three to about five or even up to about ten amino acid residues.

As used herein, the term "amino acid deletion" refers to the removal of at least one amino acid residue from a given amino acid sequence.

The term "assay signal" refers to the output from any method of detecting protein-protein interactions, including but not limited to, absorbance measurements from colorimetric assays, fluorescent intensity, or disintegrations per minute. Assay formats could include ELISA, facs, or other methods. A change in the "assay signal" may reflect a change in cell viability and/or a change in the kinetic off-rate, the kinetic on-rate, or both. A "higher assay signal" refers to the measured output number being larger than another number (e.g. a variant may have a higher (larger) measured number in an ELISA assay as compared to the parent polypeptide). A "lower" assay signal refers to the measured ouput number being smaller than another number (e.g. a variant may have a lower (smaller) measured number in an ELISA assay as compared to the parent polypeptide).

The term "binding affinity" refers to the equilibrium dissociation constant (expressed in units of concentration) associated with each Fc receptor-Fc binding interaction. The binding affinity is directly related to the ratio of the kinetic off-rate (generally reported in units of inverse time, e.g. seconds⁻¹) divided by the kinetic on-rate (generally reported in units of concentration per unit time, e.g. molar /second). In general it is not possible to unequivocally state whether changes in equilibrium dissociation constants are due to differences in on-rates, off-rates or both unless each of these parameters are experimentally determined (e.g., by BIACORE or SAPIDYNE measurements).

As used herein, the term "hinge region" refers to the stretch of amino acids in human IgG1 stretching from Glu216 to Pro230 of human IgG1. Hinge regions of other IgG isotypes may be aligned with the IgG1 sequence by placing the first and last cysteine residues forming inter-heavy chain S-S bonds in the same positions.

As used herein, the term "lower hinge region" of an Fc region refers to the stretch of amino acid residues immediately C-terminal to the hinge region (e.g. residues 233 to 239 of the Fc region of IgG1).

"C1q" is a polypeptide that includes a binding site for the Fc region of an immunoglobulin. C1q together with two serine proteases, C1r and C1s, forms the complex C1, the first component of the complement dependent cytotoxicity (CDC) pathway.

As used here, the term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

As used herein, the term "antibody fragments" refers to a portion of an intact antibody. Examples of antibody fragments include, but are not limited to, linear antibodies; single-chain antibody molecules; Fc or Fc' peptides, Fab and Fab fragments, and multispecific antibodies formed from antibody fragments. The antibody fragments preferably retain at least part of the hinge and optionally the CH1 region of an IgG heavy chain. In other preferred embodiments, the antibody fragments comprise at least a portion of the CH2 region or the entire CH2 region.

As used herin, the term "functional fragment", when used in reference to a monoclonal antibody, is intended to refer to a portion of the monoclonal antibody that still retains a functional activity. A functional activity can be, for example, antigen binding activity or specificity. Monoclonal antibody functional fragments include, for example, individual heavy or light chains and fragments thereof, such as VL, VH and Fd; monovalent fragments, such as Fv, Fab, and Fab'; bivalent fragments such as F(ab')₂; single chain Fv (scFv); and Fc fragments. Such terms are described in, for example, Harlowe and Lane, Antibodies: A Laboratory Manual, Cold Spring Harbor Laboratory, New York (1989); Molec. Biology and Biotechnology: A Comprehensive Desk Reference (Myers, R.A. (ed.), New York: VCH Publisher, Inc.); Huston et al., Cell Biophysics, 22:189-224 (1993); Pluckthun and Skerra, Meth. Enzymol., 178:497-515 (1989) and in Day, E.D., Advanced Immunochemistry, Second Ed., Wiley-Liss, Inc., New York, NY (1990), all of which are herein incorporated by reference. The term functional fragment is intended to include, for example, fragments produced by protease digestion or reduction of a monoclonal antibody and by recombinant DNA methods known to those skilled in the art.

As used herein, "humanized" forms of non-human (e.g., murine) antibodies are antibodies that contain minimal sequence, or no sequence, derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues that are not found in the recipient antibody or in the donor antibody. These modifications are generally made to further refine antibody performance. In general, the humanized antibody will comprise substantially all of at least one, and typically two, variable domains, in which all or substantially all of the hypervariable loops correspond to those of a nonhuman immunoglobulin and all or substantially all of the FR residues are those of a human immunoglobulin sequence. The humanized antibody may also comprise at least a portion of an immunoglobulin constant region (Fc), typically that of a human immunoglobulin. Examples of methods used to generate humanized antibodies are described in U.S. Pat. 5,225,539 to Winter et al. (herein incorporated by reference).

As used herein, the term "hypervariable region" refers to the amino acid residues of an antibody which are responsible for antigen-binding. The hypervariable region comprises amino acid residues from a "complementarity determining region" or "CDR" (i.e. residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (i.e. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196: 901-917 (1987)). "Framework" or "FR" residues are those variable domain residues other than the hypervariable region residues as defined herein.

As used herein, the term "immunoadhesin" designates antibody-like molecules which combine the binding domain of a heterologous "adhesin" protein (e.g. a receptor, ligand or enzyme) with an immunoglobulin constant domain. Structurally, immunoadhesins comprise a fusion of the adhesin amino acid sequence with the desired binding specificity which is other than the antigen recognition and binding site (antigen combining site) of an antibody (i.e. is "heterologous") with an immunoglobulin constant domain sequence.

As used herein, the term "ligand binding domain" refers to any native receptor or any region or derivative thereof retaining at least a qualitative ligand binding ability of a corresponding native receptor. In certain embodiments, the receptor is from a cell-surface polypeptide having an extracellular domain that is homologous to a member of the immunoglobulin supergenefamily. Other receptors, which are not members of the immunoglobulin supergenefamily but are nonetheless specifically covered by this definition, are receptors for cytokines, and in particular receptors with tyrosine kinase activity (receptor tyrosine kinases), members of the hematopoietin and nerve growth factor receptor superfamilies, and cell adhesion molecules (e.g. E-, L-, and P- selectins).

As used herein, the term "receptor binding domain" refers to any native ligand for a receptor, including cell adhesion molecules, or any region or derivative of such native ligand retaining at least a qualitative receptor binding ability of a corresponding native ligand.

As used herein, the term "antibody-immunoadhesin chimera" comprises a molecule that combines at least one binding domain of an antibody with at least one immunoadhesin. Examples include, but are not limited to, the bispecific CD4-IgG chimeras described in Berg et al., PNAS (USA) 88:4723-4727 (1991) and Charnow et al., J. Immunol., 153:4268 (1994), both of which are hereby incorporated by reference.

As used herein, an "isolated" polypeptide is one that has been identified and separated and/or recovered from a component of its natural environment. Contaminant components of its natural environment are materials that would interfere with diagnostic or therapeutic uses for the polypeptide, and may include enzymes, hormones, and other proteinaceous or non-proteinaceous solutes. In certain embodiments, the isolated polypeptide is purified (1) to greater than 95% by weight of polypeptides as determined by the Lowry method, and preferably, more than 99% by weight, (2) to a degree sufficient to obtain at least 15 residues of N-terminal or internal amino acid sequence by use of a spinning cup sequenator, or (3) to homogeneity by SDS-page under reducing or nonreducing conditions using Coomassie blue, or silver stain. Isolated polypeptide includes the polypeptide in situ within recombinant cells since at least one component of the polypeptide's natural environment will not be present. Ordinarily, however, isolated polypeptide will be prepared by a least one purification step.

As used herein, the term "treatment" refers to both therapeutic treatment and prophylactic or preventative measures. Those in need of treatment include those already with the disorder as well as those in which the disorder is to be prevented.

As used herein, the term "disorder" refers to any condition that would benefit from treatment with a polypeptide variant, including chronic and acute disorders or diseases (e.g. pathological conditions that predispose a patient to a particular disorder). In certain embodiments, the disorder is cancer.

As used herein, the terms "cancer" and "cancerous" refer to or describe the physiological condition in mammals that is typically characterized by unregulated cell growth. Examples of cancer include, but are not limited to, carcinoma, lymphoma, blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma and various types of head and neck cancer.

As used herein, the phrase "HER2-expressing cancer" is one comprising cells which have HER2 receptor protein (e.g., Genebank accession number X03363) present at their cell surface, such that an anti-HER2 antibody is able to bind to the cancer.

As used herein, the term "label" refers to a detectable compound or composition that is conjugated directly or indirectly to a polypeptide. The label may itself be detectable (e.g., radioisotope labels or fluorescent labels) or, in the case of an enzymatic label, may catalyze chemical alteration of a substrate compound or composition which is detectable.

As used herein, the terms "control element", "control sequence" and "regulatory element" refers to a genetic element that controls some aspect of the expression of nucleic acid sequences. For example, a promoter is a regulatory element that facilitates the initiation of transcription of an operably linked coding region. Other regulatory elements include splicing signals, polyadenylation signals, termination signals, etc. Control elements that are suitable for prokaryotes, for example, include a promoter, optionally an operator sequence, and a ribosome binding site. Eukaryotic cells are known to utilize promoters, polyadenylation signals, and enhancers.

As used herein, nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. In preferred embodiments, "operably linked" means that the DNA sequences being linked are contiguous, and, in the case of a secretory leader, contiguous and in reading frame. However enhancers, for example, do not have to be contiguous. Linking may be accomplished, for example, by ligation at convenient restriction sites. If such sites do not exist, synthetic oligonucleotide adaptors or linkers may be used in accordance with conventional practice.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content due to deliberate or inadvertent mutations. Mutant progeny that have the same function or biological activity as screened for in the originally transformed cell are included. Where distinct designations are intended, it will be clear from the context.

As used herein, "analyte" refers to a substance that is to be analyzed. The preferred analyte is an Fc region containing polypeptide that is to be analyzed for its ability to bind to an Fc receptor.

As used herein, the term "receptor" refers to a polypeptide capable of binding at least one ligand. The preferred receptor is a cell-surface or soluble receptor having an extracellular ligand-binding domain and, optionally, other domains (e.g. transmembrane domain, intracellular domain and/or membrane anchor). A receptor to be evaluated in an assay described herein may be an intact receptor or a fragment or derivative thereof (e.g. a fusion protein comprising the binding domain of the receptor fused to one or more heterologous polypeptides). Moreover, the receptor to be evaluated for its binding properties may be present in a cell or isolated and optionally coated on an assay plate or some other solid phase or labeled directly and used as a probe.

As used herein, the phrase "CHO-expressed polypeptide" refers to a polypeptide that has been recombinantly expressed in Chinese Hamster Ovary (CHO) cells.

As used herein, the term "antibody responsive disease" refers to any disease or medical condition that is shown to be treatable, at least in part, with antibody therapy. Examples of such diseases and medical conditions include, but are not limited to, lymphoma (shown to be treatable with RITUXAN), infectious disease (shown to be treatable with SYNAGIS), kidney transplant (ZENAPAX has shown to be helpful), Crohn's disease and rheumatoid arthritis (shown to be treatable with REMICADE), breast carcinoma (shown to be treatable with HERCEPTIN), and colon cancer (shown to be treatable with EDRECOLOMAB). As used herein, the term "immunoadhesin responsive disease" refers to any disease or medical condition that is shown to be treatable, at least in part, with immunoadhesin therapy.

As used herein a polypeptide variant that "mediates antibody-dependent cell-mediated cytotoxicity (ADCC) in the presence of human effector cells more effectively" than a parent antibody is one which in vitro or in vivo is substantially more effective at mediating ADCC, when the amounts of polypeptide variant and parent antibody used in the assay are essentially the same. For example, such a variant causes a higher amount of target cell lysis in a given ADCC assay than the parent polypeptide in an identical ADCC assay. Such variants may be identified, for example, using an ADCC assay, but other assays or methods for determining ADCC activity may also be employed (e.g. animal models). In preferred embodiments, the polypeptide variant is from about 1.2 fold, 1.5 fold, 50 fold, 100 fold, about 500 fold, or about 1000 fold more effective at mediating ADCC than the parent polypeptide.

As used herein a polypeptide variant that "mediates antibody-dependent B cell depletion from whole blood more effectively" than a parent antibody is one which in vitro or in vivo is substantially more effective at mediating depleting B cells, when the amounts of polypeptide variant and parent antibody used in the assay are essentially the same. For example, such a variant causes a higher degree of B cell depletion in a given assay than the parent polypeptide in an identical assay. Also, such a variant may deplete B cells to the same extent but at lower concentrations than are required of the parent polypeptide in an identical assay. Such variants may be identified, for example, using an ADCC assay, but other assays or methods for determining ADCC activity may also be employed (e.g. animal models). In preferred embodiments, the enhancement in depletion relative to the parent polypeptide is independent of the genotype of the FcRIIIa genotype at position 158 (V or F) and the FcRIIa genotype at position 131 (H or R), and the polypeptide variant is from about 1.2 fold, 1.5 fold, 50 fold, 100 fold, about 500 fold, or about 1000 fold more effective at mediating B cell depletion than the parent polypeptide.

The term "symptoms of an antibody or immunoadhesin responsive disease" refers to those symptoms generally associated with a particular disease. For example, the symptoms normally associated with Crohn's disease include: abdominal pain, diarrhea, rectal bleeding, weight loss, fever, loss of appetite, dehydration, anemia, distention, fibrosis, inflamed intestines and malnutrition.

The phrase "under conditions such that the symptoms are reduced" refers to any degree of qualitative or quantitative reduction in detectable symptoms of any antibody or immunoadhesin responsive disease, including but not limited to, a detectable impact on the rate of recovery from disease (*e.g*. rate of weight gain), or the reduction of at least one of the symptoms normally associated with the particular disease (e.g., if the antibody or immunoadhesin responsive disease were Crohn's disease, a reduction in at least one of the following symptoms: abdominal pain, diarrhea, rectal bleeding, weight loss, fever, loss of appetite, dehydration, anemia, distention, fibrosis, inflamed intestines and malnutrition).

### DESCRIPTION OF THE INVENTION

The present invention provides polypeptide Fc region variants and oligonucleotides encoding Fc region variants. Specifically, the present invention provides compositions comprising novel Fc region variants, methods for identifying useful Fc region variants, and methods for employing Fc region variants for treating disease. The description of the invention is provided below in the following sections: I.) Antibody Fc Regions; II.) Variant Fc Regions; III.) Combination Variants; IV.) Variant Polypeptide Assays; V.) Exemplary Variant Fc Region Containing Molecules; VI.) Nucleic Sequences Encoding Fc Region Variants; VII.) Therapeutic Uses and Formulations; VIII.) Additional Variant Fc Region Uses.

### I. Antibody Fc Regions

As described above, antibodies have regions, primarily the CH2 and CH3 regions, that are involved in non-antigen binding functions. Together, these regions are generally known as the Fc region, and have several effector functions mediated by binding of effector molecules.

The effector functions mediated by the antibody Fc region can be divided into two categories: (1) effector functions that operate after the binding of antibody to an antigen (these functions involve, for example, the participation of the complement cascade or Fc receptor (FcR)-bearing cells); and (2) effector functions that operate independently of antigen binding (these functions confer, for example, persistence in the circulation and the ability to be transferred across cellular barriers by transcytosis). For example, binding of the Cl component of complement to antibodies activates the complement system. Following opsonization, activation of complement is important in the lysis of cell pathogens. The activation of complement also stimulates the inflammatory response and may also be involved in autoimmune hypersensitivity. Further, antibodies bind to cells via the Fc region, with an Fc receptor binding site on the antibody Fc region binding to a Fc receptor (FcR) on a cell. There are a number of Fc receptors which are specific for different classes of antibody, including IgG (gamma receptors), IgE (eta receptors), IgA (alpha receptors) and IgM (mu receptors). While the present invention is not limited to any particular mechanism, binding of antibody to Fc receptors on cell surfaces triggers a number of important and diverse biological responses including engulfment and destruction of antibody-coated particles, clearance of immune complexes, lysis of antibody-coated target cells by killer cells (called antibody-dependent cell-mediated cytotoxicity, or ADCC), release of inflammatory mediators, placental transfer and control of immunoglobulin production.

Several antibody effector functions are mediated by Fc receptors (FcRs), which bind the Fc region of an antibody. FcRs are defined by their specificity for immunoglobulin isotypes; Fc receptors for IgG antibodies are referred to as FcγR, for IgE as FcεR, for IgA as FcαR and so on. Three subclasses of FcγR have been identified: FcγRI (CD64), FcγRII (CD32) and FcγRIII (CD16).

Because each FcγR subclass is encoded by two or three genes, and alternative RNA spicing leads to multiple transcripts, a broad diversity in FcγR isoforms exists. The three genes encoding the FcγRI subclass (FcγRIA, FcγRIB and FcγRIC) are clustered in region 1q21.1 of the long arm of chromosome 1; the genes encoding FcγRII isoforms (FcγRIIA, FcγRIIB and FcγRIIC) and the two genes encoding FcγRIII (FcγRIIIA and FcγRIII1B) are all clustered in region 1q22. These different FcR subtypes are expressed on different cell types (see, e.g., Ravetch and Kinet, Annu. Rev. Immunol. 9: 457-492 (1991)). For example, in humans, FcγRIIIB is found only on neutrophils, whereas FcγRIIIA is found on macrophages, monocytes, natural killer (NK) cells, and a subpopulation of T-cells. Notably, FcγRIIIA is present on NK cells, one of the cell types implicated in ADCC.

Human FcγRIIIA (CD16) receptor has a common polymorphism at position 158 in its extracellular domain encoding either a phenylalanine or valine at this position. The V allele of FcγRIIIA has higher affinity to human IgG 1 than the F allele. The V158 allele also mediates ADCC more efficiently. Clinical data have shown a correlation between the genotype of FcγRIIIA receptor in patients undergoing Rituxan treatment and therapeutic response. Both clinical and molecular responses and time to progression were shown to be superior in patients homozygous for the FcγRIIIA-158V genotype (approximately 20% of population). In contrast, patients heterozygous or homozygous for the lower affinity FcγRIIIA-158F genotype (approximately 80% of population) respond more poorly. These data suggest that Fc mutations that enhance ADCC activity of the 158F carriers might enhance the clinical efficacy of antibody-based therapy of cancer. A genetic polymorphism is also present in human FcγRIIA (CD32) receptor at position 131 in its extracellular domain encoding either a histidine (H) or arginine (R) at this position. The polymorphism at position 131 has been found to affect its ability to bind to human IgG. Recent data also show a correlation between the FcγRIIA position 131 polymorphism and clinical response to Rituxan. Patients homozygous for the H131 allele had a significantly higher response rate than the other 2 groups.

FcγRI, FcγRII and FcγRIII are immunoglobulin superfamily (IgSF) receptors; FcγRI has three IgSF domains in its extracellular domain, while FcγRII and FcγRIII have only two IgSF domains in their extracellular domains.

Another type of Fc receptor is the neonatal Fc receptor (FcRn). FcRn is structurally similar to major histocompatibility complex (MHC) and consists of an α-chain noncovalently bound to β2-microglobulin.

### II. Variant Fc Regions

The present invention provides polypeptide variants, nucleic acid sequences encoding the polypeptide variants, and methods for generating polypeptide variants. Preferably, the polypeptide variants of the present invention differ from a parent polypeptide by at least one amino acid modification. The "parent", "wild type", "starting" or "nonvariant" polypeptide preferably comprises at least a portion of an antibody Fc region, and may be prepared using techniques available in the art for generating polypeptides comprising an Fc region or portion thereof. In preferred embodiments, the parent polypeptide is an antibody. The parent polypeptide may, however, be any other polypeptide comprising at least a portion of an Fc region (e.g. an immunoadhesin). In certain embodiments, a variant Fc region may be generated (e.g. according to the methods disclosed herein) and can be fused to a heterologous polypeptide of choice, such as an antibody variable domain or binding domain of a receptor or ligand.

In preferred embodiments, the parent polypeptide comprises an Fc region or functional portion thereof. Generally, the Fc region of the parent polypeptide will comprise a native sequence Fc region, and preferably a human native sequence Fc region. However, the Fc region of the parent polypeptide may have one or more pre-existing amino acid sequence alterations or modifications from a native sequence Fc region. For example, the C1q binding activity of the Fc region may have been previously altered or the FcγR binding affinity of the Fc region may have been altered. In further embodiments, the parent polypeptide Fc region is conceptual (e.g. mental thought or a visual representation on a computer or on paper) and, while it does not physically exist, the antibody engineer may decide upon a desired variant Fc region amino acid sequence and generate a polypeptide comprising that sequence or a DNA encoding the desired variant Fc region amino acid sequence. However, in preferred embodiments, a nucleic acid encoding an Fc region of a parent polypeptide is available and this nucleic acid sequence is altered to generate a variant nucleic acid sequence encoding the Fc region variant.

Nucleic acid encoding a variant of the parent polypeptide may be prepared by methods known in the art using the guidance of the present specification for particular sequences. These methods include, but are not limited to, preparation by site-directed (or oligonucleotide-mediated) mutagenesis, PCR mutagenesis, and cassette mutagenesis of an earlier prepared nucleic acid encoding the polypeptide. Site-directed mutagenesis is a preferred method for preparing variants. This technique is well known in the art (see, e.g., Carter et al. Nucleic Acids Res. 13: 4431-4443 (1985) and Kunkel et. al., Proc. Natl. Acad. Sci. USA 82: 488 (1987), both of which are hereby incorporated by reference). Briefly, in carrying out site directed mutagenesis of DNA, the starting DNA is altered by first simultaneously hybridizing one or multiple oligonucleotide(s) encoding the desired mutation(s) to a single strand of such starting DNA. After hybridization, a DNA polymerase is used to synthesize an entire second strand, using the hybridized oligonucleotide(s) as a primer, and using the single strand of the starting DNA as a template and a DNA ligase is used to ligate the second strand to form a circular, double-stranded DNA. Thus, the oligonucleotide encoding the desired mutation is incorporated in the resulting double-stranded DNA.

PCR mutagenesis is also suitable for making amino acid sequence variants of the starting polypeptide (see, e.g., Vallette et al., Nuc. Acids Res. 17: 723-733 (1989), hereby incorporated by reference). Briefly, when small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template.

Another method for preparing variants, cassette mutagenesis, is based on the technique described by Wells et al., Gene 34: 315-323 (1985), hereby incorporated by reference. The starting material is the plasmid (or other vector) comprising the starting polypeptide DNA to be mutated. The codon(s) in the starting DNA to be mutated are identified. There must be a unique restriction endonuclease site on each side of the identified mutation site(s). If no such restriction sites exist, they may be generated using the above-described oligonucleotide-mediated mutagenesis method to introduce them at appropriate locations in the starting polypeptide DNA. The plasmid DNA is cut at these sites to linearize it. A double-stranded oligonucleotide encoding the sequence of the DNA between the restriction sites but containing the desired mutation(s) is synthesized using standard procedures, wherein the two strands of the oligonucleotide are synthesized separately and then hybridized together using standard techniques. This double-stranded oligonucleotide is referred to as the cassette. This cassette is designed to have 5' and 3' ends that are compatible with the ends of the linearized plasmid, such that it can be directly ligated to the plasmid. This plasmid now contains the mutated DNA sequence.

Alternatively, or additionally, the desired amino acid sequence encoding a polypeptide variant can be determined, and a nucleic acid sequence encoding such amino acid sequence variant can be generated synthetically.

The amino acid sequence of the parent polypeptide may be modified in order to generate a variant Fc region with altered Fc receptor binding affinity or activity in vitro and/or in vivo and/or altered antibody-dependent cell-mediated cytotoxicity (ADCC) activity in vitro and/or in vivo. The amino acid sequence of the parent polypeptide may also be modified in order to generate a variant Fc region with altered complement binding properties and/or circulation half-life.

Substantial modifications in the biological properties of the Fc region may be accomplished by selecting substitutions that differ significantly in their effect on altering (a) the structure of the polypeptide backbone in the area of the substitution, for example, as a sheet or helical conformation, (b) the charge or hydrophobicity of the molecule at the target site, or the bulk of the side chain, (d) interaction with carbohydrate, or (e) flexibility of domain movement. Naturally occurring residues are divided into classes based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.
Non-conservative substitutions will entail exchanging a member of one of these classes for a member of another class. Conservative substitutions will entail exchanging a member of one of these classes for another member of the same class.

As is demonstrated in the Examples below, one can engineer an Fc region variant with altered activity (effector function(s) and pharmacokinetics). One may, for example, modify one or more amino acid residues of the Fc region in order to alter (e.g. increase or decrease) ADCC activity. In preferred embodiments, a modification comprises one or more of the Fc region residues identified herein (See, e.g, Example 2, and WO0042072, herein incorporated by reference for all purposes). Generally, one will make an amino acid substitution at one or more of the Fc region residues identified herein as effecting ADCC activity in order to generate such an Fc region variant. In preferred embodiments, no more than one to about ten Fc region residues will be deleted or substituted. The Fc regions herein comprising one or more amino acid modifications (e.g. substitutions) will preferably retain at least about 80%, and preferably at least about 90%, and most preferably at least about 95%, of the parent Fc region sequence or of a native sequence human Fc region.

One may also make amino acid insertion Fc region variants, which variants have altered effector function. For example, one may introduce at least one amino acid residue (e.g. one to two amino acid residues and generally no more than ten residues) adjacent to one or more of the Fc region positions identified herein as impacting FcR binding. By "adjacent" is meant within one to two amino acid residues of a Fc region residue identified herein. Such Fc region variants may display enhanced or diminished FcR binding and/or ADCC activity relative to the partent molecule. In order to generate such insertion variants, one may evaluate a co-crystal structure of a polypeptide comprising a binding region of an FcR (e.g. the extracellular domain of the FcR of interest) and the Fc region into which the amino acid residue(s) are to be inserted (see, e.g., Sondermann et al. Nature 406:267 (2000); Deisenhofer, Biochemistry 20 (9): 2361-2370 (1981); and Burmeister et al., Nature 342: 379-383, (1994), all of which are herein incorporated by reference) in order to rationally design an Fc region variant with, e.g., improved FcR binding ability. In preferred embodiments, such insertion(s) are made in an Fc region loop, but not in the secondary structure (i.e. in a β-strand) of the Fc region.

By introducing the appropriate amino acid sequence modifications in a parent Fc region, one can generate a variant Fc region which (a) mediates antibody-dependent cell-mediated cytotoxicity (ADCC) in the presence of human effector cells more or less effectively and/or (b) mediates complement-dependent cytotoxicity (CDC) in the presence of human complement more or less effectively and/or (c) binds an Fc gamma receptor (FcγR) or Fc neonatal receptor (FcRn) with the desired affinity at different pHs than the parent polypeptide. Such Fc region variants will generally comprise at least one amino acid modification in the Fc region.

In preferred embodiments, the parent polypeptide Fc region is a human Fc region, e.g. a native human Fc region human IgG1 (f and a,z allotypes), IgG2, IgG3, IgG4, and all allotypes known or discovered from any species.. Such regions have sequences such as those shown in Figure 2 (SEQ ID NOs:1-8), Figure 3 (SEQ ID NOs:9-12), and Figure 5 (SEQ ID NOs:32-37).

In certain embodiments, in order to generate an Fc region with improved ADCC activity, the parent polypeptide preferably has pre-existing ADCC activity (e.g., the parent polypeptide comprises a human IgG1 or human IgG3 Fc region). In some embodiments, a variant with improved ADCC mediates ADCC substantially more effectively than an antibody with a native sequence IgG1 or IgG3 Fc region (e.g. P247L and I332E variants).

In preferred embodiments, amino acid modification(s) are introduced into the CH2 and/or CH3 domains of a Fc region. Useful amino acid positions for modification in order to generate a variant IgG Fc region with altered ADCC activity include any one or more of amino acid positions: 247, 251, 256, 268, 269, 270, 272, 276, 278, 280, 283, 285, 286, 289, 290, 292, 293, 294, 295, 296, 298, 300 301, 303, 305, 307, 309, 330, 331, 332, 333, 334, 335, 337, 338, 339, 340, 360, 373, 376, 416, 419, 430, 434, 435, 437, 438 ,439, or 440 of the Fc region. In preferred embodiments, the parent Fc region used as the template to generate such variants comprises a human IgG Fc region.

In certain embodiments, the present invention provides compositions comprising a variant of a parent polypeptide having an Fc region, wherein the variant mediates antibody-dependent cell-mediated cytotoxicity (ADCC) in the presence of effector cells, and comprises at least one amino acid modification at position 247 in the Fc region. In certain embodiments, the amino acid modification is P247L. In other embodiments, the amino acid modification is P247I or P247H

In some embodiments, the present invention provides compositions comprising a variant of a parent polypeptide having an Fc region, wherein the variant mediates antibody-dependent cell-mediated cytotoxicity (ADCC) in the presence of effector cells, and comprises at least one amino acid modification at position 251 in the Fc region. In certain embodiments, the amino acid modification is L251 F.

In particular embodiments, the present invention provides compositions comprising a variant of a parent polypeptide having an Fc region, wherein the variant mediates antibody-dependent cell-mediated cytotoxicity (ADCC) in the presence of effector cells, and comprises at least one amino acid modification at position 256 in the Fc region. In certain embodiments, the amino acid modification isT256M or T256P.

In particular embodiments, the present invention provides compositions comprising a variant of a parent polypeptide having an Fc region, wherein the variant mediates antibody-dependent cell-mediated cytotoxicity (ADCC) in the presence of effector cells, and comprises at least one amino acid modification at position 268 in the Fc region. In certain embodiments, the amino acid modification is H268D or H268E.

In particular embodiments, the present invention provides compositions comprising a variant of a parent polypeptide having an Fc region, wherein the variant mediates antibody-dependent cell-mediated cytotoxicity (ADCC) in the presence of effector cells, and comprises at least one amino acid modification at position 280 in the Fc region. In certain embodiments, the amino acid modification is D280A or D280K.

In particular embodiments, the present invention provides compositions comprising a variant of a parent polypeptide having an Fc region, wherein the variant mediates antibody-dependent cell-mediated cytotoxicity (ADCC) in the presence of effector cells, and comprises at least one amino acid modification at position 330 in the Fc region. In certain embodiments, the amino acid modification is A330K or A330R.

In particular embodiments, the present invention provides compositions comprising a variant of a parent polypeptide having an Fc region, wherein the variant mediates antibody-dependent cell-mediated cytotoxicity (ADCC) in the presence of effector cells, and comprises at least one amino acid modification at position 332 in the Fc region. In certain embodiments, the amino acid modification is I332D, I332E, I332K, or I332R.

In particular embodiments, the present invention provides compositions comprising a variant of a parent polypeptide having an Fc region, wherein the variant mediates antibody-dependent cell-mediated cytotoxicity (ADCC) in the presence of effector cells, and comprises at least one amino acid modification at position 339 in the Fc region. In certain embodiments, the amino acid modification is A339T.

In particular embodiments, the present invention provides compositions comprising a variant of a parent polypeptide having an Fc region, wherein the variant mediates antibody-dependent cell-mediated cytotoxicity (ADCC) in the presence of effector cells, and comprises at least one amino acid modification at position 378 in the Fc region. In certain embodiments, the amino acid modification is A378D.

In particular embodiments, the present invention provides compositions comprising a variant of a parent polypeptide having an Fc region, wherein the variant mediates antibody-dependent cell-mediated cytotoxicity (ADCC) in the presence of effector cells, and comprises at least one amino acid modification at position 440 in the Fc region. In certain embodiments, the amino acid modification is S440Y.

The polypeptide variants described above may be subjected to further modifications, depending on the desired or intended use of the polypeptide. Such modifications may involve, for example, further alteration of the amino acid sequence (substitution, insertion and/or deletion of amino acid residues), carbohydrate modifications, fusion to heterologous polypeptide(s) and/or covalent modifications. Such further modifications may be made prior to, simultaneously with, or following, the amino acid modification(s) disclosed above which result in an alteration of Fc receptor binding and/or ADCC activity.

Alternatively or additionally, it may be useful to combine amino acid modifications with one or more further amino acid modifications that alter C1q binding and/or complement dependent cytoxicity function of the Fc region. The starting polypeptide of particular interest herein is one that binds to C1q and displays complement dependent cytotoxicity (CDC). Amino acid substitutions described herein may serve to alter the ability of the starting polypeptide to bind to C1q and/or modify its complement dependent cytotoxicity function (e.g. to reduce and preferably abolish these effector functions). However, polypeptides comprising substitutions at one or more of the described positions with improved C1q binding and/or complement dependent cytotoxicity (CDC) function are contemplated herein. For example, the starting polypeptide may be unable to bind C1q and/or mediate CDC and may be modified according to the teachings herein such that it acquires these further effector functions. Moreover, polypeptides with pre-existing C1q binding activity, optionally further having the ability to mediate CDC may be modified such that one or both of these activities are enhanced. Amino acid modifications that alter C1q and/or modify its complement dependent cytotoxicity function are described, for example, in WO0042072, which is hereby incorporated by reference.

As disclosed above, one can design an Fc region or portion thereof with altered effector function, e.g., by modifying CDC activity and/or ADCC activity. For example, one can generate a variant Fc region with improved CDC activity and improved ADCC activity (e.g. having both improved ADCC activity and improved CDC activity). Alternatively, where one desires that effector function be reduced or ablated, one may engineer a variant Fc region with reduced CDC activity and/or reduced ADCC activity. In other embodiments, one may increase only one of these activities, and optionally also reduce the other activity, e.g. to generate an Fc region variant with improved ADCC activity, but reduced CDC activity and vice versa. Additionally, one can engineer a variant Fc region with modified binding affinity to FcRn, protein A, and/or other Fc binding proteins.

Another type of amino acid substitution serves to alter the glycosylation pattern of the polypeptide. This may be achieved, for example, by deleting one or more carbohydrate moieties found in the polypeptide, and/or adding one or more glycosylation sites that are not present in the polypeptide. Alternatively, this may be achieved indirectly, by altering amino acids other than the glycosylation site or by engineering the cells. Glycosylation of polypeptides is typically either N-linked or O-linked. N-linked refers to the attachment of the carbohydrate moiety to the side chain of an asparagine residue. The peptide sequences asparagine-X-serine and asparagine-X-threonine, where X is any amino acid except proline, are the recognition sequences for enzymatic attachment of the carbohydrate moiety to the asparagine side chain. Thus, the presence of either of these peptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the polypeptide is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original polypeptide (for O-linked glycosylation sites). An exemplary glycosylation variant has an amino acid substitution of residue Asn 297 of the heavy chain.

In some embodiments, the present invention provides compositions comprising a variant of a parent polypeptide having an Fc region, wherein the variant comprises at least one surface residue amino acid modification (See, e.g., Deisenhofer, Biochemistry, 28;20(9):2361-70, April 1981, and WO0042072, both of which are hereby incorporated by reference). In other embodiments, the present invention provides compositions comprising a variant of a parent polypeptide having an Fc region, wherein the variant comprises at least one non-surface residue amino acid modification. In further embodiments, the present invention comprises a variant of a parent polypeptide having an Fc region, wherein the variant comprises at least one surface amino acid modification and at least one non-surface amino acid modification.

### III. Combination Variants

In some embodiments, the variants of the present comprise two or more amino acid modifications (e.g. substitutions). Such combination variants may be produced, for example, by selecting two or more of the amino acid modifications detailed above. Table 1 below provides exemplary combinations of two or more amino acid substitutions. For example, the first row of Table 1 shows possible combinations of P247H with other amino acid substitutions at postions 251, 256, 268, 280, 330, 332, 339, 378 and 440 (e.g. this row shows combinations of two, three, four, five, six, seven, eight, nine, and ten amino acid modifications).

**Table 1.**

| | **Exemplary Combination Variants** | | | | | | | | | |
|---|---|---|---|---|---|---|---|---|---|---|
| | Position | | | | | | | | | |
| Mutation | 247 | 251 | 256 | 268 | 280 | 330 | 332 | 339 | 378 | 440 |
| P247H | | F | M, P | D, E | A, K | K, R | D, E | T | D | Y |
| P247I | | F | M, P | D, E | A, K | K, R | D, E | T | D | Y |
| P247L | | F | M, P | D, E | A, K | K, R | D, E | T | D | Y |
| L251 F | H, I, L | | M, P | D, E | A, K | K, R | D, E | T | D | Y |
| T256M | H, I, L | F | | D, E | A, K | K, R | D, E | T | D | Y |
| T256P | H, I, L | F | | D, E | A, K | K, R | D, E | T | D | Y |
| H268D | H, I, L | F | M, P | | A, K | K, R | D, E | T | D | Y |
| H268E | H, I, L | F | M, P | | A, K | K, R | D, E | T | D | Y |
| D280A | H, I, L | F | M, P | D, E | | K, R | D, E | T | D | Y |
| D280K | H, I, L | F | M, P | D, E | | K, R | D, E | T | D | Y |
| A330K | H, I, L | F | M, P | D, E | A, K | | D, E | T | D | Y |
| A330R | H, I, L | F | M, P | D, E | A, K | | D, E | T | D | Y |
| I332E | H, I, L | F | M, P | D, E | A, K | K, R | | T | D | Y |
| I332D | H,I,L | F | M, P | D, E | A, K | K, R | | T | D | Y |
| A339T | H,I,L | F | M, P | D, E | A, K | K, R | D, E | | D | Y |
| A378D | H,I,L | F | M,P | D,E | A,K | K,R | D,E | T | | Y |
| S440Y | H, I, L | F | M, P | D, E | A, K | K, R | D, E | T | D | |

| | | | | | | | | | | |
|---|---|---|---|---|---|---|---|---|---|---|
| ^{**} Note that table uses EU numbering as in Kabat. | | | | | | | | | | |

The combination variants shown in Table 1 and other combination variants (such as those disclosed in WO0042072) may be tested for a given activity (e.g. FcR binding activity, ADCC activity, and CDC activity) in a variety of assays (See, Section IV. below). In this regard, useful combination variants may be identified.

In certain preferred embodiments, the combination variants of the present invention have one amino acid modification that increases ADCC activity, and one amino acid modification that increases neonatal Fc receptor (FcRn) binding affinity (e.g., at pH 6.0, but not at pH 7.0 or 7.4). In other embodiments, the combination variants of the present invention have one surface amino acid modification, and one non-surface amino acid modification. Additional combination variants may be generated by combining two or more of the amino acid modifications described herein, or at least one of the amino acid modifications described herein with those described in WO0042072.

### IV. Variant Polypeptide Assays

The present invention provides various assays for screening Fc region variants. Screening assays may be used to find or confirm useful variants. For example, combination variants (See Table 1) may be screened to find variants with altered FcR binding, and/or altered ADCC and/or altered CDC activity (e.g. increased or decreased ADCC or CDC activity) and/or modified ability to deplete target cells (B cells, for eg.) from whole blood. Also, as described below, the assays of the present invention may be employed to find or confirm variants that have beneficial therapeutic activity in a subject (e.g. such as a human with symptoms of an antibody or immunoadhesin responsive disease). A variety of assay types may be employed to evaluate any change in a variant compared to the parent polypeptide (See, screening assays provided in WO0042072, herein incorporated by reference). Further exemplary assays are described below.

In preferred embodiments, the variants of the present invention are antibodies that essentially retain the ability to bind antigen (via an unmodified antigen binding region or modified antigen binding region) compared to the nonvariant (parent) polypeptide (e.g. the binding capability is preferably no worse than about 20 fold or no worse than about 5 fold of that of the nonvariant polypeptide). The binding capability of the polypeptide variant to antigen may be determined using techniques such as ELISA, fluorescence activated cell sorting (FACS) analysis, or radioimmunoprecipitation (RIA), for example.

Fc receptor (FcR) binding assays may be employed to evaluate the variants of the present invention. For example, binding of Fc receptors such as FcγRI, FcγRIIa, FcγRIIb, FcγRIII, FcRn, etc., can be measured by titrating polypeptide variant and measuring bound polypeptide variant using an antibody which specifically binds to the polypeptide variant in an ELISA format (see Examples below). For example, a variant that comprises an antibody may be screened in a standard ELISA assay to determine binding to an FcRn at pH 6.0 and pH 7.0 or 7.4. A solid surface coated with streptavidin or Neutravidin may be used to capture biotin labeled FcRn from any species, such as mouse or human. After blocking, the capture receptor can be incubated with variant polypeptides (antibodies) diluted in buffers at pH 6.0 or pH 7.0. In the following step a molecule specific for human antibodies is added (e.g. goat (Fab')2 anti-human-Fab conjugated to an enzyme). Thereafter a substrate may be added in order to determine the amount of binding of the variant polypeptide to the immobilized FcRn at pH 6.0 or pH 7.0 or 7.4. The results of this assay can be compared to the parent (non-variant) polypeptide's ability to bind the same FcR. In other preferred embodiments, the components for carrying out an ELISA (e.g. with FcRn ) to screen variants are packaged in a kit (e.g. with instructions for use).

An antibody dependent cellular cytotoxicity (ADCC) assay may also be employed to screen the variants of the present invention. ADCC assays may be performed in vitro or in vivo. To assess ADCC activity of a polypeptide variant an in vitro ADCC assay may be performed using varying effector:target ratios. An exemplary ADCC assay could use a target cell line expressing any of the following target antigens: CD20, CD22, CD33, CD40, CD63, EGF receptor, her-2 receptor, prostate-specific membrane antigen, Lewis Y carbohydrate, GD₂ and GD₃ gangliosides, lamp-1, CO-029, L6, and ephA2. Effector cells may be obtained from a healthy donor (e.g. on the day of the experiment) and PBMC purified using Histopaque (Sigma). Target cells are then preincubated with an IgG variant at, for example, 0.1 - 1,000 ng/ml for about 30 minutes prior to mixing with effector cells at effector:target ratios of, for example, 40:1, 20:1 and 10:1. ADCC activity may then be measured colorimetrically using a Cytotoxicity Detection Kit (Roche Molecular Biochemicals) for the quantitation of cell death and lysis based upon the measurement of lactate dehydrogenase (LDH) activity released from the cytosol of damaged cells into the supernatant. ADCC activity may also be measured, for Chromium 51 loaded target cell assays, by measuring the resulting Chromium 51 released. Antibody independent cellular cytoxicity can be determined by measuring the LDH activity from target and effector cells in the absence of antibody. Total release may be measured following the addition of 1% Triton X-100 to the mixture of target and effector cells. Incubation of the target and effector cells may be performed for an optimized period of time (0.54-18 hours) at 37 C in 5.0 % CO₂ and then be followed by centrifugation of the assay plates. The supernatants may then be transferred to 96 well plates and incubated with LDH detection reagent for 30 minutes at 25°C. The sample absorbance may then be measured at 490 nm using a microplate reader. The percent cytotoxicity can then be calculated using the following equation: % cytotoxicity = experimental value - low control / high control - low control X 100%. The percent cytoxicity of anti-CD20 and variants can then be compared directly with equal amount of RITUXAN to provide a measurement of relative effectiveness. An exemplary ADCC assay could employ SKW6.4 cells over-expressing the CD20 antigen (e.g. purchased from the American Type Culture Collection) as the source of target cells. Many variations of this assay are known in the art (See, e.g., Zuckerman et al., CRC Crit Rev Microbiol 1978;7(1):1-26, herein incorporated by reference).

Useful effector cells for such assays includes, but is not limited to, natural killer (NK) cells, macrophages, and other peripheral blood mononuclear cells (PBMC). Alternatively, or additionally, ADCC activity of the polypeptide variants of the present invention may be assessed in vivo, e.g., in a animal model such as that disclosed in Clynes et al. PNAS (USA) 95:652-656 (1998), herein incorporated by reference). The variants of the present invention may also be screened for complement activation. To assess complement activation, a complement dependent cytotoxicity (CDC) assay may be performed (See, e.g. Gazzano-Santoro et al., J. Immunol. Methods, 202:163 (1996), herein incorporated by reference). For example, various concentrations of the polypeptide variant and human complement may be diluted with buffer. Cells which express the antigen to which the polypeptide variant binds may be diluted to a density of ∼1 x 10⁶ cells/ml. Mixtures of polypeptide variant, diluted human complement and cells expressing the antigen may be added to a flat bottom tissue culture 96 well plate and allowed to incubate for 2 hours at 37°C and 5% CO₂ to facilitate complement mediated cell lysis. 50 ul of alamar blue (Accumed International) may then be added to each well and incubated overnight at 37°C. The absorbance may be measured using a 96-well fluorimeter with excitation at 530 nm and emission at 590 nm. The results may be expressed in relative fluorescence units (RFU). The sample concentrations may be computed from a standard curve and the percent activity as compared to nonvariant polypeptide may be reported for the polypeptide variant of interest.

In certain embodiments, the variants of the present invention do not activate complement. For example, a polypeptide variant displays about 0-10% CDC activity in this assay compared to a control antibody having a nonmutated IgG1 Fc region. Preferably the variant does not appear to have any CDC activity (e.g. above background) in the above CDC assay. In other embodiments, the variants of the present invention are found to have enhanced CDC compared to a parent polypeptide (e.g., displaying about two-fold to about 100-fold (or greater) improvement in CDC activity in vitro or in vivo when the IC50 values are compared).

The variants of the present invention may also be screened for depletion of target cells in a whole blood assay. For example, various concentrations of the polypeptide variant with CD20 target specificity can be screened for the depletion of B cells in a whole blood assay using facs (Vugmeyster et al., 2003 Cytometry 52A, 101-109). Freshly drawn blood is incubated with varying concentrations of polypeptide variant at 37°C and 5% CO₂ for 4 hours (time can be varied). Following the incubation red blood cells are lysed per manufacturer's directions with an ammonium chloride reagent (Beckton-Dickinson cat. #555899) and B cells are detected by facs using a fluorescent-labeled antibody specific for B cells (anti-CD19, for example). The results may be expressed as % depletion of B cells relative to either an untreated sample or a sample incubated with an irrelevant (non-depleting) antibody.

In preferred embodiments, the variant depletes B cells more effectively than the parental polypeptide. A variant may deplete B cells, for example, to about two-fold or greater extent, and preferably about five-fold or more. Also, a variant may display greater potency in depleting B cells. For example, a variant may deplete the same percentage of B cells relative to parental polypeptide, but utilize about five-fold less, and preferably about 10-fold less antibody. Target cell depletion mediated by variants may be about 5-fold to about 100-fold, and preferably from about 5-fold to about 1000-fold improved compared to the parent polypeptide.

The variants of the present invention may also be *screened in vivo.* Any type of *in vivo* assay may be employed. A particular example of one type of assay is provided below. This exemplary assay allows for preclinical evaluation of Fc variants *in vivo.* A variant to be tested may be incorporated into the Fc region of a particular antibody known to have some activity. For example, a variant may be incorporated into the Fc region of an anti-CD20 IgG by mutagenesis. This allows a parental IgG and Fc variant IgG to be compared directly with RITUXAN (known to promote tumor regression). The preclinical evaluation may be done in 2 phases (a pharmacokinetic and pharmacodynamic phase). The goal of the Phase I pharmacokinetic studies is to determine if there are differences in the clearance rate between an Fc variant IgG and the antibody with known in vivo activity (e.g. RITUXAN). Differences in clearance rate may cause differences in the steady-state level of IgG in serum. As such, if differences in steady-state concentrations are detected these should be normalized to enable accurate comparisons to be made. The goal of the Phase II pharmacodynamic studies is to determine the effect of the Fc mutations upon, in this case, tumor growth. Previous studies with RITUXAN used a single dose which completely inhibited tumor growth. Because this does not allow quantitative differences to be measured, a dose range should be employed.

Phase I pharmacokinetic comparison of an Fc variant, the wild type parental Fc, and RITLJXAN may be performed in the following manner. First, 40 µg per animal may be injected intravenously and the plasma level of the IgG quantitated at 0, .25, .5, 1, 24, 48, 72, 96, 120, 168, and 336 hrs. The data may be fitted, for example, using a pharmacokinetic program (WinNonLin) using a zero lag two compartment pharmacokinetic model to obtain the clearance rate. Clearance rate may be used to define steady state plasma level with the following equation: C= Dose/(Clearance rate X τ), where τ ts the interval between doses and C is the plasma level at steady state. Pharmacokinetic experiments may be performed in non-tumor bearing mice with, for example, a minimum of 5 mice per time point.

An animal model may be employed for the next phase in the following manner. The right flank of CB17-SCID mice may be implanted with 10⁶ Raji cells subcutaneously. Intravenous bolus of the Fc variant, the wild type Fc, and RITUXAN may be commenced immediately after implantation and continued until the tumor size is greater than 2 cm in diameter. Tumor volume may be determined every Monday, Wednesday and Friday by measuring the length, width, and depth of the tumor using a caliper (tumor volume= W x L x D). A plot of tumor volume versus time will give the tumor growth rate for the pharmakodynamic calculation. A minimum of about 10 animals per group should be used.

Phase II pharmacodynamic comparison of the Fc variant, the wild type Fc, and RITUXAN may be performed in the following manner. Based on published data, RITUXAN at 10 µg/g weekly completely inhibited tumor growth in vivo (Clynes et al., Nat. Med. 2000 Apr;6(4):443-6, 2000, herein incorporated by reference). Therefore, a weekly dose range of 10 µg/g, 5 µg/g, 1 µg/g, 0.5 µg/g, and 0 µg/g may be tested. The steady state plasma level at which tumor growth rate is inhibited by 50% may be graphically determined by the relationship between steady state plasma level and effectiveness. The steady state plasma level may be calculated as described above. If necessary, τ may be adjusted accordingly for each Fc variant and the Fc wild type depending on their pharmacokinetic properties to achieve comparable steady state plasma level as RITUXAN. Statistical improved pharmakodynamic values of the Fc variant in comparison to the parental polypeptide (e.g. Fc wild type) and RITUXAN will generally indicate that Fc variant confers improved activity *in vivo.*

Additional pharmacodynamic comparison of the Fc variants, the wild type Fc, and RITUXAN may be performed in cynomolgous monkeys as described previously (Reff et al., Blood 83, 435-445, 1994). A dose response for depletion of peripheral B cells and lymph node B cells may be used to compare the relative potencies of the Fc variants with wild type Fc and Rituxan administered intravenously and/or subcutaneously. Statistical improved pharmakodynamic values of the Fc variant in comparison to the parental polypeptide (e.g. Fc wild type) and RITUXAN will generally indicate that Fc variant confers improved activity *in vivo.*

In further embodiments, the variants of the present invention are screened such that variants that are useful for therapeutic use in at least two species are identified. Such variants are referred to herein as "dual-species improved variants", and are particularly useful for identifying variants that are therapeutic in humans, and also demonstrate (or are likely to demonstrate) efficacy in an animal model. In this regard, the present invention provides methods for identifying variants that have a strong chance of being approved for human clinical testing since animal model data will likely support any human testing applications made to governmental regulatory agencies (e.g. U.S. Food and Drug Administration).

In certain embodiments, dual-species improved variants are identified by first performing an ADCC assay using human effector cells to find improved variants, and then performing a second ADCC assay using mouse, rat, or non-human primate effector cells to identify a sub-set of the improved variants that are dual-species improved variants. In some embodiments, the present invention provides methods for identifying dual-species improved variants, comprising; a) providing; i) target cells, ii) a composition comprising a candidate variant of a parent polypeptide having at least a portion of an Fc region, wherein the candidate variant comprises at least one amino acid modification in the Fc region, and wherein the candidate variant mediates target cell cytotoxicity in the presence of a first species (e.g. human) of effector cells more effectively than the parent polypeptide, and iii) second species (e.g. mouse, rat, or non-human primate) effector cells, and b) incubating the composition with the target cells under conditions such that the candidate variant binds the target cells thereby generating candidate variant bound target cells, c) mixing the second species effector cells with the candidate variant bound target cells, and d) measuring target cell cytotoxicity mediated by the candidate variant. In certain embodiments, the method further comprises step e) determining if the candidate variant mediates target cell cytotoxicity in the presence of the second species effector cells more effectively than the parent polypeptide. In some embodiments, the method further comprises step f) identifying a candidate variant as a dual-species improved variant that mediates target cell cytotoxicity in the presence of the second species effector cells more effectively than the parent polypeptide. In preferred embodiments, the dual-species variants identified are then screened *in vivo* in one or more animal assays.

In certain embodiments, dual-species improved variants are identified by first performing a whole blood assay using human blood to find improved variants, and then performing a second whole blood assay using mouse, rat, or non-human primate blood to identify a sub-set of the improved variants that are dual-species improved variants. In some embodiments, the present invention provides methods for identifying dual-species improved variants, comprising; a) providing; i) target cells, ii) a composition comprising a candidate variant of a parent polypeptide having at least a portion of an Fc region, wherein the candidate variant comprises at least one amino acid modification in the Fc region, and wherein the candidate variant mediates target cell depletion in the presence of a first species (e.g. human) blood more effectively than the parent polypeptide, and iii) second species (e.g. mouse, rat, or non-human primate) blood, and b) incubating the composition with the target cells under conditions such that the candidate variant binds the target cells thereby generating candidate variant bound target cells, c) mixing the second species blood with the candidate variant bound target cells, and d) measuring target cell depletion mediated by the candidate variant. In certain embodiments, the method further comprises step e) determining if the candidate variant mediates target cell depletion in the presence of the second species blood more effectively than the parent polypeptide. In some embodiments, the method further comprises step f) identifying a candidate variant as a dual-species improved variant that mediates target cell depletion in the presence of the second species blood more effectively than the parent polypeptide. In preferred embodiments, the dual-species variants identified are then screened *in vivo* in one or more animal assays.

In certain embodiments, dual-species improved variants are identified by performing any of the assays above using human components (e.g. human cells, human Fc receptors, etc.) to identify improved variants, and then running the same assay (or a different assay) with non-human animal components (e.g. mouse cells, mouse Fc receptors, etc.). In this regard, a sub-set of variants that perform well according to a given criteria in both human based assays and a second species based assays can be identified.

An exemplary process for identifying dual-species improved variants is a follows. First, a nucleic acid sequence encoding at least a portion of an IgG Fc region is mutated such that the amino acid sequence expressed has at least one amino acid change, thereby generating a variant. This expressed IgG variant is then characterized in an ADCC assay using human PBMCs or a subset (NK cells or macrophages, for example). If enhanced ADCC activity is found, then the variant is screened in a second ADCC assay using mouse or rat PBMCs. Alternatively, or in addition, an assay can be performed with the variant for binding to cloned rodent receptors or cell lines. Finally, if the variant is found to be improved in the second assay, making it a dual-improved variant, then the variant is screened *in vivo* in mice or rats.

### V. Exemplary Variant Fc Region Containing Molecules

The variant Fc regions of the present invention may be part of larger molecules. The larger molecules may be, for example, monoclonal antibodies, polyclonal antibodies, chimeric antibodies, humanized antibodies, bispecific antibodies, immunoadhesins, etc. As such, it is evident that there is a broad range of applications for the variant Fc regions of the present invention.

### A. Antibodies Containing Variant Fc Regions

In preferred embodiments, the variant Fc region containing molecule (e.g. polypeptide) is an antibody. Techniques for producing antibodies are described below.

### (i) Antigen selection and preparation

Generally, when the variant Fc region containing molecule is an antibody, the antibody is directed against an antigen of interest. Preferably, the antigen is a polypeptide and administration of the antibody to a mammal suffering from a disease or disorder can result in a therapeutic benefit in that mammal. However, antibodies directed against nonpolypeptide antigens (such as tumor associated glycolipid antigens; see US Patent 5,091,178, herein incorporated by reference), may also be employed.

Exemplary antigens include, but are not limited to, molecules such as renin; a growth hormone, including human growth hormone and bovine growth hormone; growth hormone releasing factor; parathyroid hormone; thyroid stimulating hormone; lipoproteins; alpha-1- antitrypsin; insulin A-chain; insulin B-chain; proinsulin; follicle stimulating hormone; calcitonin; luteinizing hormone; glucagon; clotting factors such as factor VIIIC, factor IX, tissue factor (TF), and von Willebrands factor; anti-clotting factors such as Protein C; atrial natriuretic factor; lung surfactant; a plasminogen activator, such as urokinase or human urine or tissue-type plasminogen activator (t-PA); bombesin; thrombin; hemopoietic growth factor; tumor necrosis factor-alpha and -beta; enkephalinase; RANTES (regulated on activation normally T-cell expressed and secreted); human macrophage inflammatory protein (MIP-1-alpha); a serum albumin such as human serum albumin; Muellerian-inhibiting substance; relaxin A-chain; relaxin B-chain; prorelaxin; mouse gonadotropin-associated peptide; a microbial protein, such as beta-lactamase; DNase; IgE; a cytotoxic T-lymphocyte associated antigen (CTLA), such as CTLA-4; inhibin; activin; vascular endothelial growth factor (VEGF); receptors for hormones or growth factors; protein A or D; rheumatoid factors; a neurotrophic factor such as bone-derived neurotrophic factor (BDNF), neurotrophin-3,-4,-5, or-6 (NT-3, NT-4, NT-5, or NT-6), or a nerve growth factor; platelet-derived growth factor (PDGF); fibroblast growth factor such as αFGF and βEGF; epidermal growth factor (EGF); transforming growth factor (TGF) such as TGF-alpha and TGF beta, including TGF- 1, TGF- 2, TGF- 3, TGF- 4, or TGF- 5; insulin-like growth factor-I and-II (IGF-I and IGF-II); des (1-3)-IGF-I (brain IGF-I), insulin-like growth factor binding proteins; CD proteins such as CD3, CD4, CD8, CD19 and CD20; erythropoietin; osteoinductive factors; immunotoxins; a bone morphogenetic protein (BMP); an interferon such as interferon-alpha,-beta, and-gamma; colony stimulating factors (CSFs), e. g., M-CSF, GM-CSF, and G-CSF; interleukins (ILs), e.g., IL-1 to IL-10; superoxide dismutase; T-cell receptors; surface membrane proteins; decay accelerating factor; viral antigen such as, for example, a portion of the AIDS envelope; transport proteins; homing receptors; addressins; regulatory proteins; integrins such as CD11 a, CD11 b, CD11c, CD18, an ICAM, VLA-4 and VCAM; a tumor associated antigen such as HER2, HER3 or HER4 receptor; a member of an apoptosis pathway; and fragments of any of the above-listed polypeptides.

Preferred antigens include, but are not limited to, CD proteins such as CD3, CD4, CD8, CD19, CD20 and CD34; members of the ErbB receptor family such as the EGF receptor, HER2, HER3 or HER4 receptor; cell adhesion molecules such as LFA-1, Mac1, p150.95, VLA-4, ICAM-1, VCAM, a4/p7 integrin, and (Xv/p3 integrin including either a or subunits thereof (e.g. anti-CD11a, anti-CD18 or anti-CD11b antibodies); growth factors such as VEGF; tissue factor (TF); alpha interferon (a-IFN); an interleukin, such as IL-8; IgE; blood group antigens; flk2/flt3 receptor; obesity (OB) receptor; mpl receptor; CTLA-4; protein C etc.

Soluble antigens or fragments thereof, optionally conjugated to other molecules, can be used as immunogens for generating antibodies. For transmembrane molecules, such as receptors, fragments of these (e.g. the extracellular domain of a receptor) can be used as the immunogen. Alternatively, cells expressing the transmembrane molecule can be used as the immunogen. Such cells can be derived from a natural source (e.g. cancer cell lines) or may be cells which have been transformed by recombinant techniques to express the transmembrane molecule. Other antigens and forms thereof useful for preparing antibodies will be apparent to those in the art.

### (ii) Polyclonal Antibodies

The present invention provides polyclonal antibodies with variant Fc regions. For example, a human immunoglobulin repertoire containing modified G1 constant regions may be transplanted into immunoglobulin-inactivated mice, resulting in mice expressing an IgG repertoire containing modified Fc regions (see e.g. Mendez, MJ *et al.,* Nature Genetics 15:146 (1997), herein incorporated by reference). Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized (e.g. keyhole limpet hemocyanin, serum albumin, bovine thyroglobulin, or soybean tyrpsin inhibitor) using a bifunctional or derivitizing agent (e.g. maleimidobenzoyl sulfosuccinimide ester for conjugation through cystein residues, N-hydroxysuccinimide for conjugation through lysine residues, glutaraldehyde, succinic anhydride, SOCl₂, or R1N=C=NR, where R and R1 are different alkyl groups.

Examples of a general immunization protocol for a rabbit and mouse are as follows. Animals are immunized against the antigen, immunogenic conjugates, or derivatives by combining, for example, 100 µg or 5 µg of the protein or conjugate (e.g. for a rabbit or mouse respectively) with 3 volumes of Freund's complete adjuvant and injecting the solution intradermally at multiple sites. One month later the animals are boosted with 1/5 or 1/10 the original amount of peptide or conjugate in Freund's complete adjuvant by subcutaneous injection at multiple sites. Seven to fourteen days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. In addition, aggregating agents such as alum are suitably used to enhance the immune response.

### (iii) Monoclonal antibodies

The present invention provides monoclonal antibodies with variant Fc regions. Monoclonal antibodies may be made in a number of ways, including using the hybridoma method (e.g. as described by Kohler et al., Nature, 256: 495, 1975, herein incorporated by reference), or by recombinant DNA methods (e.g., U. S. Patent No. 4,816,567, herein incorporated by reference).

In the hybridoma method, a mouse or other appropriate host animal, such as a hamster or macaque monkey, is immunized to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell. The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (e.g., Kozbor, J. Immunol., 133: 3001 (1984), herein incorporated by reference).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). After hybridoma cells are identified that produce antibodies of the desired specificity, affinity, and/or activity, the clones may be subcloned by limiting dilution procedures and grown by standard methods. Suitable culture media for this purpose include, for example, D-MEM or RPMI-1640 medium. In addition, the hybridoma cells may be grown in vivo as ascites tumors in an animal. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. Recombinant production of antibodies is described in more detail below.

In some embodiments, antibodies or antibody fragments are isolated from antibody phage libraries generated using the techniques described in, for example, McCafferty et al., Nature, 348: 552554 (1990). Clackson et al., Nature, 352:624-628 (1991) and Marks et al., J. Mol. Biol., 222: 581-597 (1991) describe the isolation of murine and human antibodies, respectively, using phage libraries. Subsequent publications describe the production of high affinity (nM range) human antibodies by chain shuffling (Marks et al., BioTechnology, 10: 779-783 (1992)), as well as combinatorial infection and in vivo recombination as a strategy for constructing very large phage libraries (e.g., Waterhouse et al., Nuc. Acids. Res., 21: 2265-2266 (1993)). Thus, these techniques, and similar techniques, are viable alternatives to traditional monoclonal antibody hybridoma techniques for isolation of monoclonal antibodies.

Also, the DNA may be modified, for example, by substituting the coding sequence for human heavy-and light-chain constant domains in place of the homologous murine sequences (e.g., U. S. Patent No. 4,816,567, and Morrison, et al., Proc. Nat. Acad. Sci USA, 81: 6851 (1984), both of which are hereby incorporated by reference), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### (iv) Humanized and human antibodies

The present invention provides humanized and human antibodies with variant Fc regions. In preferred embodiments, a humanized antibody comprises human antibody amino acid sequences together with amino acid residues that are not from a human antibody. In some embodiments, the human sequences in a humanized antibody comprise the framework regions (FRs) and the sequences or residues that are not from a human antibody comprise one or more complementarity-determining regions (CDRs).

It is worth noting that FRs and CDRs can be defined based on amino acid residue numbering in the heavy and light chain variable regions. The term complementarity determining region, or CDR is intended to mean the non-contiguous antigen combining sites found within the variable region of both heavy and light chain polypeptides. These regions have been defined by Kabat et al. (J. Biol. Chem. 252:6609-6616 (1977) and Kabat et al. Sequences of Proteins of Immunological Interest (1991); "Kabat"), Chothia et al. (J. Mol. Biol. 196:901-917 (1987); "Chothia") and MacCallum et al. (J. Mol. Biol. 262:732-745 (1996); "MacCallum"), where the definitions include overlapping or subsets of amino acid residues when compared against each other. Nevertheless, the application of any of these definitions, alone (for example, the Kabat definition) or in combination (by way of example only, the combined definition of Kabat and Chothia) to refer to a CDR of an antibody (including a humanized antibody) is intended to be within the scope of the term as defined and used herein. The amino acid residues which encompass the CDRs as defined by each of the above cited references are set forth below in Table 6 as a comparison.

**Table 6: CDR Definitions:**

| | Kabat | Chothia | MacCallum |
|---|---|---|---|
| V_{H} CDR1 | 31-35 | 26-32 | 30-35 |
| V_{H} CDR2 | 50-65 | 53-55 | 47-58 |
| V_{H} CDR3 | 95-102 | 96-101 | 93-101 |
| V_{L} CDR1 | 24-34 | 26-32 | 30-36 |
| V_{L} CDR2 | 50-56 | 50-52 | 46-55 |
| V_{L} CDR3 | 89-97 | 91-96 | 89-96 |

Also, the term "framework" when used in reference to an antibody variable region is intended to mean all amino acid residues outside the CDR regions within the variable region of an antibody. Therefore, a variable region framework is between about 100-120 amino acids in length but is intended to reference only those amino acids outside of the CDRs. The term "framework region" is intended to mean each domain of the framework that is separated by the CDRs. Therefore, for the specific example of a heavy chain variable region and for the CDRs as defined by Kabat, framework region 1 (FR1) corresponds to the domain of the variable region encompassing amino acids 1-30; region 2 (FR2) corresponds to the domain of the variable region encompassing amino acids 36-49; region 3 (FR3) corresponds to the domain of the variable region encompassing amino acids 66-94, and region 4 (FR4) corresponds to the domain of the variable region from amino acid 103 to the end of the variable region. The framework regions for the light chain are similarly separated by each of the light chain variable region CDRs. Similarly, using the definition of CDRs by Chothia or MacCallum, or any combination of CDR definitions, the framework boundaries are separated by the respective CDR termini as described above. Notwithstanding the multiple definitions of CDRs, in some embodiments, it is preferred to use the Kabat definition to define CDRs.

The residues in a humanized antibody that are not from a human antibody may be residues or sequences imported from or derived from another species (including but not limited to mouse), or these sequences may be random amino acid sequences (e.g. generated from randomized nucleic acid sequences), which are inserted into the humanized antibody sequence. As noted above, the human amino acid sequences in a humanized antibody are preferably the framework regions, while the residues which are not from a human antibody (whether derived from another species or random amino acid sequences) preferably correspond to the CDRs. However, in some embodiments, one or more framework regions may contain one or more non-human amino acid residues. In cases of alterations or modifications (e.g. by introduction of a non-human residue) to an otherwise human framework, it is possible for the altered or modified framework region to be adjacent to a modified CDR from another species or a random CDR sequence, while in other embodiments, an altered framework region is not adjacent to an altered CDR sequence from another species or a random CDR sequence. In some embodiments, the framework sequences of a humanized antibody are entirely human (i.e. no framework changes are made to the human framework). In preferred embodiments, the framework sequences of a humanized antibody are entirely human germline (i.e. no framework changes are made to the human germline framework).

Non-human amino acid residues from another species, or a random sequence, are often referred to as "import" residues, which are typically taken from an "import" variable domain. Humanization can be essentially performed following the method of Winter and co-workers (e.g., Jones et al., Nature, 321: 522-525 (1986); Riechmann et al., Nature, 332: 323-327 (1988); Verhoeyen et al., Science, 239: 1534-1536 (1988), all of which are hereby incorporated by reference), by substituting rodent (or other mammal) CDRs or CDR sequences for the corresponding sequences of a human antibody. Also, antibodies wherein substantially less than an intact human variable domain has been substituted by the corresponding sequence from a non-human species may also be generated (e.g. 4,816,567, hereby incorporated by reference). In practice, humanized antibodies are typically human antibodies in which some CDR residues and possibly some FR residues are substituted by residues from analogous sites in rodent antibodies, or, as noted above, in which CDR sequences have been substituted by random sequences. By way of nonlimiting example only, methods for conferring donor CDR binding affinity onto an antibody acceptor variable region framework are described in WO 01/27160 A1, herein incorporated by reference and in US applications 09/434,870 and 09/982,464, all of which are herein incorporated by reference.

The choice of human variable domains, both light and heavy, to be used in making the humanized antibodies is important to reduce antigenicity. According to the so-called "best-fit" method, the sequence of the variable domain of a rodent antibody to be humanized is screened against the entire library of known human variable-domain sequences. The human sequence which is closest to that of the rodent is then accepted as the human framework (FR) for the humanized antibody (e.g., Sims et al., J. Immunol., 151: 2296 (1993), and Chothia et al., J. Mol. Biol., 196: 901 (1987), both of which are hereby incorporated by reference). Another method uses a particular framework derived from the consensus sequence of all human antibodies of a particular subgroup of light or heavy chains. The same framework may be used for several different humanized antibodies (e.g., Carter et al., Proc. Natl. Acad. Sci. USA, 89: 4285 (1992); Presta et al., J. Immunol., 151: 2623 (1993), both of which are hereby incorporated by reference).

In other embodiments, there is no need to "pre-select" a particular human antibody framework (i. e. there is no need to select a human framework with the closest homology or sequence identity to a given candidate antibody to be humanized). In these embodiments, a common or universal human framework may be used to accept one or more non-human CDRs. In the preferred embodiment, a single universal, fully human framework is used as the framework for all antibodies to be humanized, regardless of its homology to the framework sequence(s) of the candidate antibodies. In this regard, humanized antibodies may be generated without making any changes in the framework region. This universal, fully human framework can then accept one or more CDR sequences. In one embodiment, the one or more CDR sequences are CDR sequences from an antibody from another species (*e.g*. mouse or rat) which have been modified in comparison to the corresponding CDR in the intact antibody from the other species (*i.e.* there is simultaneous introduction of the CDR and modification of the CDR being introduced into the universal human framework). The modification corresponds to one or more amino acid changes (in the modified CDR) in comparison to the corresponding CDR in the intact antibody from the other species. In one embodiment, all amino acid residues in the CDR are included in a library, while in other embodiments, not all of the CDR amino acid residues are included in a library. In another embodiment, the one or more CDR sequences are random sequences, which substitute for CDR sequences.

In preferred embodiments, antibodies are humanized with retention of high affinity for the antigen and other favorable biological properties. In some embodiments, the affinity of the humanized antibody for the antigen is higher than the affinity of the corresponding non-humanized, intact antibody or fragment or portion thereof (*e*.*g*. the candidate rodent antibody). In this regard, in some embodiments, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen (s), is achieved. In general, the CDR residues are directly and most substantially involved in influencing antigen binding.

A variety of specific methods, well known to one of skill in the art, may be employed to introduce antibody CDRs (or random sequences substituting for antibody CDRs) into antibody frameworks (see, for example, US applications 09/434,879 and 09/982,464). In some embodiments, overlapping oligos may be used to synthesize an antibody gene, or portion thereof (for example, a gene encoding a humanized antibody). In other embodiments, mutagenesis of an antibody template may be carried out using the methods of Kunkel (infra), for example to introduce a modified CDR or a random sequence to substitute for a CDR. In some embodiments, light and heavy chain variable regions are humanized separately, and then co-expressed as a humanized variable region. In other embodiments, humanized variable regions make-up the variable region of an intact antibody. In some embodiments, the Fc region of the intact antibody comprising a humanized variable region has been modified (e.g. at least one amino acid modification has been made in the Fc region). For example, an antibody that has been humanized with randomized CDR and no framework changes may comprise at least one amino acid modification in the Fc region.

In other embodiments, transgenic animals (e.g., mice) that are capable, upon immunization, of producing a full repertoire of human antibodies in the absence of endogenous immunoglobulin production are employed. For example, it has been described that the homozygous deletion of the antibody heavy-chain joining region (JH) gene in chimeric and germ- line mutant mice results in complete inhibition of endogenous antibody production. Transfer of the human germ-line immunoglobulin gene array in such germ-line mutant mice will result in the production of human antibodies upon antigen challenge (See, e.g., Jakobovits et al., Proc. Natl. Acad. Sci. USA, 90: 2551 (1993), and Jakobovits et al., Nature, 362: 255-258 (1993), both of which are hereby incorporated by reference). Human antibodies can also be derived from phage-display libraries (e.g., Hoogenboorn et al., J. Mol. Biol., 227: 381 (1991), and Vaughan et al. Nature Biotech 14: 309 (1996), both of which are hereby incorporated by reference).

The present invention provides methods for generating humanized antibodies (and antibody fragments) that comprise at least one amino acid modification in the Fc region (as compared to a parental polypeptide having an Fc region). Discussed below are additional methods for generating such humanized antibodies. The present invention also provides compositions comprising the antibodies and antibody fragments generated by these methods. Importantly, the humanization methods discussed below, and other huminization methods (e.g. discussed above), may be combined with the Fc variants of the present invention. In this regard, humanized antibodies with altered, unique Fc regions may be constructed according to the present invention.

In some embodiments, a method of constructing a population of altered heavy chain variable region encoding nucleic acids is provided, comprising: a) providing a representation of first and second reference amino acid sequences, the first reference sequence comprising the sequence of a donor heavy chain variable region, the donor variable region comprising i) framework regions and ii) three complementarity-determining regions as defined by the combined definitions of Kabat and Chothia; the second reference sequence comprising the sequence of an acceptor heavy chain variable region comprising framework regions; b) synthesizing a) first oligonucleotides encoding portions of the framework regions of the acceptor heavy chain variable region, wherein the portions of the framework regions when compared to the second reference sequence are unmodified; and b) a population of second oligonucleotides, each encoding i) at least a portion of a first complementarity-determining region that has been modified, the first complementarity-determining region selected from the group consisting of HCDR1, HCDR2 and HCDR3, wherein the modified first complementarity-determining region comprises a different amino acid at one or more positions when compared to the corresponding donor complementarity determining regions of the first reference sequence and ii) one or more portions of unmodified framework regions which are capable of hybridizing to the first oligonucleotides; c) mixing the first oligonucleotides with the population of second oligonucleotides as to create overlapping oligonucleotides; and d) treating the overlapping oligonucleotides under conditions such that a population of altered heavy chain variable region encoding nucleic acids is constructed, wherein the framework regions encoded by the altered heavy chain variable region encoding nucleic acids are unmodified with respect to the second reference sequence.

In some embodiments, the representation of first and second reference sequences is in electronic form. In some embodiments, the method further comprises the step of (E) coexpressing the population of altered heavy chain variable region encoding nucleic acids with a light chain variable region encoding nucleic acid so as to produce a diverse population of altered heteromeric variable regions. In some embodiments, the synthesizing comprises chemically synthesizing. In some embodiments, the acceptor is human. In some embodiments, the treating of step D) comprises extension by a polymerase.

In other embodiments, a method of constructing a population of altered light chain variable region encoding nucleic acids is provided, comprising: a) providing a representation of first and second reference amino acid sequences, the first reference sequence comprising the sequence of a donor light chain variable region, the donor variable region comprising i) framework regions and ii) three complementarity-determining regions as defined by the combined definitions of Kabat and Chothia; the second reference sequence comprising the sequence of an acceptor light chain variable region comprising framework regions; b) synthesizing a) first oligonucleotides encoding portions of the framework regions of the acceptor light chain variable region, wherein the portions of the framework regions when compared to the second reference sequence are unmodified; and b) a population of second oligonucleotides, each encoding i) at least a portion of a first complementarity-determining region that has been modified, the first complementarity-determining region selected from the group consisting of LCDR1, LCDR2 and LCDR3, wherein the modified first complementarity-determining region comprises a different amino acid at one or more positions when compared to the corresponding donor complementarity determining regions of the first reference sequence and ii) one or more portions of unmodified framework regions which are capable of hybridizing to the first oligonucleotides; c) mixing the first oligonucleotides with the population of second oligonucleotides as to create overlapping oligonucleotides; and d) treating the overlapping oligonucleotides under conditions such that a population of altered light chain variable region encoding nucleic acids is constructed, wherein the framework regions encoded by the altered light chain variable region encoding nucleic acids are unmodified with respect to the second reference sequence.

In some embodiments, the representation of first and second reference sequences is in electronic form. In some embodiments, the method further comprises the step of (E) co-expressing the population of altered light chain variable region encoding nucleic acids with a heavy chain variable region encoding nucleic acid so as to produce a diverse population of altered heteromeric variable regions. In some embodiments, the synthesizing comprises chemically synthesizing. In some embodiments, the acceptor is human. In some embodiments, the treating of step D) comprises extension by a polymerase.

In some embodiments, a method of constructing a population of altered heavy chain variable region encoding nucleic acids is contemplated, comprising: A) providing a representation of first and second reference amino acid sequences, the first reference sequence comprising the sequence of a donor heavy chain variable region, the donor variable region comprising i) framework regions and ii) three complementarity-determining regions as defined by the combined definitions of Kabat and Chothia; the second reference sequence comprising the sequence of an acceptor heavy chain variable region comprising framework regions; B) synthesizing a) a population of first oligonucleotides, each encoding at least a portion of a first complementarity-determining region selected from the group consisting of HCDR1, HCDR2 and HCDR3, wherein the modified first complementarity-determining region comprises a different amino acid at one or more positions when compared to the corresponding donor complementarity determining regions of the first reference sequence; and b) second oligonucleotides encoding i) portions of the framework regions of the acceptor heavy chain variable region, wherein the portions of the framework regions when compared to the reference sequence are unmodified and ii) one or more portions of a complementarity determining region which are capable of hybridizing to the population of first oligonucleotides; C) mixing the population of first oligonucleotides with the second oligonucleotides as to create overlapping oligonucleotides; and D) treating the overlapping oligonucleotides under conditions such that a population of altered heavy chain variable region encoding nucleic acids is constructed, wherein the framework regions encoded by the altered heavy chain variable region encoding nucleic acids are unmodified with respect to the second reference sequence.

In some embodiments, the representation of first and second reference sequences is in electronic form. In some embodiments, the method further comprises the step of (E) coexpressing the population of altered heavy chain variable region encoding nucleic acids with a light chain variable region encoding nucleic acid so as to produce a diverse population of altered heteromeric variable regions. In some embodiments, the synthesizing comprises chemically synthesizing. In some embodiments, the acceptor is human. In some embodiments, the treating of step D) comprises extension by a polymerase.

In other embodiments, a method of constructing a population of altered light chain variable region encoding nucleic acids is provided, comprising: A) providing a representation of first and second reference amino acid sequences, the first reference sequence comprising the sequence of a donor light chain variable region, the donor variable region comprising i) framework regions and ii) three complementarity-determining regions as defined by the combined definitions of Kabat and Chothia; the second reference sequence comprising the sequence of an acceptor light chain variable region comprising framework regions; B) synthesizing a) a population of first oligonucleotides, each encoding at least a portion of a first complementarity-determining region selected from the group consisting of LCDR1, LCDR2 and LCDR3, wherein the modified first complementarity-determining region comprises a different amino acid at one or more positions when compared to the corresponding donor complementarity determining regions of the first reference sequence; and b) second oligonucleotides encoding i) portions of the framework regions of the acceptor light chain variable region, wherein the portions of the framework regions when compared to the reference sequence are unmodified and ii) one or more portions of a complementarity determining region which are capable of hybridizing to the population of first oligonucleotides; C) mixing the population of first oligonucleotides with the second oligonucleotides as to create overlapping oligonucleotides; and D) treating the overlapping oligonucleotides under conditions such that a population of altered light chain variable region encoding nucleic acids is constructed, wherein the framework regions encoded by the altered light chain variable region encoding nucleic acids are unmodified with respect to the second reference sequence.

In some embodiments, the representation of first and second reference sequences is in electronic form. In some embodiments, the method further comprises the step of (E) coexpressing the population of altered light chain variable region encoding nucleic acids with a heavy chain variable region encoding nucleic acid so as to produce a diverse population of altered heteromeric variable regions. In some embodiments, the synthesizing comprises chemically synthesizing. In some embodiments, the acceptor is human. In some embodiments, the treating of step D) comprises extension by a polymerase.

In other embodiments, a method of constructing a population of altered heavy chain variable region encoding nucleic acids is contemplated, comprising: a) providing a representation of first and second reference amino acid sequences, the first reference sequence comprising the sequence of a donor heavy chain variable region, the donor variable region comprising i) framework regions and ii) three complementarity-determining regions, the second reference sequence comprising a heavy chain variable region; b) synthesizing a population of altered heavy chain variable region antibody gene sequences, wherein the framework regions of the altered heavy chain variable regions are identical to the framework regions of the second reference sequence and at least a first CDR of the altered antibody variable regions has been modified, wherein the modified first CDR comprises a different amino acid at one or more positions when compared to the corresponding donor CDR of the first reference sequence.

In some embodiments, the representation of first and second reference sequences is in electronic form. In some embodiments, the method further comprises the step of (E) coexpressing the population of altered heavy chain variable region encoding nucleic acids with a light chain variable region encoding nucleic acid so as to produce a diverse population of altered heteromeric variable regions. In some embodiments, the acceptor is human. In some embodiments, the synthesizing involves the use of overlapping oligonucleotides. In some embodiments, the CDRs are defined by the Kabat definition.

In some embodiments, a method of constructing a population of altered light chain variable region encoding nucleic acids is contemplated, comprising: a) providing a representation of first and second reference amino acid sequences, the first reference sequence comprising the sequence of a donor light chain variable region, the donor variable region comprising i) framework regions and ii) three complementarity-determining regions; the second reference sequence comprising the sequence of an acceptor light chain variable region comprising framework regions; b) synthesizing a population of altered light chain variable region antibody gene sequences, wherein the framework regions of the altered light chain variable regions are identical to the framework regions of the second reference sequence and at least a first CDR of the altered antibody light chain variable region has been modified, wherein the modified first CDR comprises a different amino acid at one or more positions when compared to the corresponding donor CDR of the first reference sequence.

In some embodiments, the representation of first and second reference sequences is in electronic form. In some embodiments, the method further comprisies the step of (E) coexpressing the population of altered light chain variable region encoding nucleic acids with a heavy chain variable region encoding nucleic acid so as to produce a diverse population of altered heteromeric variable regions. In some embodiments, the acceptor is human. In some embodiments, the synthesizing involves the use of overlapping oligonucleotides.

In yet other embodiments, a method of constructing a population of altered heavy chain variable region encoding nucleic acids is contemplated, comprising: a) providing a representation of a reference amino acid sequence, the reference sequence comprising the sequence of an acceptor heavy chain variable region comprising framework regions; b) synthesizing a population of altered heavy chain variable region antibody gene sequences, wherein the framework regions of the altered heavy chain variable regions are identical to the framework regions of the reference sequence and at least a first CDR of the altered antibody variable regions comprises a random amino acid sequence.

In some embodiments, the representation of the reference sequence is in electronic form. In some embodiments, the method further comprising the step of (E) coexpressing the population of altered heavy chain variable region encoding nucleic acids with a light chain variable region encoding nucleic acid so as to produce a diverse population of altered heteromeric variable regions. In some embodiments, the acceptor is human. In some embodiments, the synthesizing involves the use of overlapping oligonucleotides. In some embodiments, the CDRs are defined by the Kabat definition.

In other embodiments, a method of constructing a population of altered light chain variable region encoding nucleic acids is contemplated, comprising: a) providing a representation of a reference amino acid sequence, the reference sequence comprising the sequence of an acceptor light chain variable region comprising framework regions; b) synthesizing a population of altered light chain variable region antibody gene sequences, wherein the framework regions of the altered light chain variable regions are identical to the framework regions of the reference sequence and at least a first CDR of the altered antibody light chain variable regions comprises a random amino acid sequence.

In some embodiments, the representation of the reference sequence is in electronic form. In some embodiments, the method further comprises the step of (E) coexpressing the population of altered light chain variable region encoding nucleic acids with a heavy chain variable region encoding nucleic acid so as to produce a diverse population of altered heteromeric variable regions. In some embodiments, the acceptor is human. In some embodiments, the synthesizing involves the use of overlapping oligonucleotides. In some embodiments, the CDRs are defined by the Kabat definition.

In yet other embodiments, a method of constructing a population of altered heavy chain variable region encoding nucleic acids is contemplated, comprising: a) providing a representation of a reference amino acid sequence, the reference sequence comprising the sequence of a human acceptor heavy chain variable region comprising framework regions; b) synthesizing a population of altered heavy chain variable region antibody gene sequences, wherein the framework regions of the altered heavy chain variable regions are identical to the framework regions of the human reference sequence and at least a first CDR of the altered antibody variable regions comprises a random amino acid sequence. In some embodiments, the representation of the human reference sequence is in electronic form. In some embodiments, the method further comprises the step of (E) coexpressing the population of altered heavy chain variable region encoding nucleic acids with a light chain variable region encoding nucleic acid so as to produce a diverse population of altered heteromeric variable regions. In some embodiments, the synthesizing involves the use of overlapping oligonucleotides. In some embodiments, the CDRs are defined by the Kabat definition.

In other embodiments, a method of constructing a population of altered light chain variable region encoding nucleic acids is contemplated, comprising: a) providing a representation of a reference amino acid sequence, the reference sequence comprising the sequence of a human acceptor light chain variable region comprising framework regions; b) synthesizing a population of altered light chain variable region antibody gene sequences, wherein the framework regions of the altered light chain variable regions are identical to the framework regions of the human reference sequence and at least a first CDR of the altered antibody light chain variable regions comprises a random amino acid sequence.

In some embodiments, the representation of the reference sequence is in electronic form. In some embodiments, the method further comprises the step of (E) coexpressing the population of altered light chain variable region encoding nucleic acids with a heavy chain variable region encoding nucleic acid so as to produce a diverse population of altered heteromeric variable regions. In some embodiments, the synthesizing involves the use of overlapping oligonucleotides. In some embodiments, the CDRs are defined by the Kabat definition.

In some embodiments, one or more framework regions are modified simultaneously with the introduction of one or more modified CDRs. In other embodiments, the modified frameworks are adjacent to the modified CDRs.

In some embodiments, the present invention provides methods of constructing a population of altered heavy chain variable region encoding nucleic acids, comprising: a) providing a representation of first and second reference amino acid sequences, the first reference amino acid sequence comprising the sequence of a donor heavy chain variable region, the donor variable region comprising i) framework regions and ii) three complementarity-determining regions as defined by the combined definitions of Kabat and Chothia; the second reference amino acid sequence comprising the sequence of an acceptor heavy chain variable region comprising framework regions; b) synthesizing a) a first population of oligonucleotides, comprising oligonucleotides encoding a modified heavy chain variable region framework region, or portion thereof, wherein the heavy chain variable region framework region, or portion thereof, contains a plurality of changed amino acids at one or more positions when compared to the acceptor framework region reference sequence, wherein the framework positions that are changed are selected from among the acceptor framework positions of the second reference sequence that differ at the corresponding position compared to the donor framework positions of the first reference sequence; and b) a second population of oligonucleotides, each encoding i) at least one modified complementarity-determining region, or portion thereof, wherein the modified complementarity-determining region, or portion thereof, comprises a different amino acid at one or more positions when compared to the corresponding donor complementarity-determining region amino acid reference sequence and ii) one or more portions of adjacent framework regions which are capable of hybridizing to the first population of oligonucleotides; and c) mixing the first and second populations of oligonucleotides so as to create overlapping oligonucleotides; and d) treating the overlapping oligonucleotides under conditions such that a population of altered heavy chain variable region encoding nucleic acids is constructed. In certain embodiments, the representation of first and second reference sequences is in electronic form. In other embodiments, the methods further comprise the step of (e) coexpressing the population of altered heavy chain variable region encoding nucleic acids with a light chain variable region encoding nucleic acid so as to produce a diverse population of altered heteromeric variable regions. In additional embodiments, the synthesizing comprises chemically synthesizing. In some embodiments, the acceptor is human. In preferred embodiments, the one or more of the diverse population of altered heteromeric variable regions are part of an antibody comprising an Fc region, wherein the Fc region comprises at least one amino acid modification as compared to a parental polypeptide having an Fc region.

In other embodiments, the present invention provides methods of constructing a population of altered light chain variable region encoding nucleic acids, comprising: a) providing a representation of first and second reference amino acid sequences, the first reference amino acid sequence comprising the sequence of a donor light chain variable region, the donor variable region comprising i) framework regions and ii) three complementarity-determining regions as defined by the combined definitions of Kabat and Chothia; the second reference amino acid sequence comprising the sequence of an acceptor light chain variable region comprising framework regions; b) synthesizing a) a first population of oligonucleotides, comprising oligonucleotides encoding a modified light chain variable region framework region, or portion thereof, wherein the light chain variable region framework region, or portion thereof, contains a plurality of changed amino acids at one or more positions when compared to the acceptor framework region reference sequence, wherein the framework positions that are changed are selected from among the acceptor framework positions of the second reference sequence that differ at the corresponding position compared to the donor framework positions of the first reference sequence; and b) a second population of oligonucleotides, each encoding i) at least one modified complementarity-determining region, or portion thereof, wherein the modified complementarity-determining region, or portion thereof, comprises a different amino acid at one or more positions when compared to the corresponding donor complementarity-determining region amino acid reference sequence and ii) one or more portions of adjacent framework regions which are capable of hybridizing to the first population of oligonucleotides; and c) mixing the first and second populations of oligonucleotides so as to create overlapping oligonucleotides; and d) treating the overlapping oligonucleotides under conditions such that a population of altered light chain variable region encoding nucleic acids is constructed. In other embodiments, the representation of first and second reference sequences is in electronic form. In additional embodiments, the methods further comprise the step of (e) coexpressing the population of altered light chain variable region encoding nucleic acids with a heavy chain variable region encoding nucleic acid so as to produce a diverse population of altered heteromeric variable regions.

In some embodiments, the methods comprise a) providing a representation of first and second reference amino acid sequences, the first reference amino acid sequence comprising the sequence of a donor heavy chain variable region, the donor variable region comprising i) framework regions and ii) three complementarity-determining regions as defined by the combined definitions of Kabat and Chothia; the second reference amino acid sequence comprising the sequence of an acceptor heavy chain variable region comprising framework regions; b) synthesizing a) a first population of oligonucleotides, comprising oligonucleotides encoding a modified heavy chain variable region framework region, or portion thereof, wherein the heavy chain variable region framework region, or portion thereof, contains a plurality of changed amino acids at one or more positions when compared to the acceptor framework region reference sequence, wherein the framework positions that are changed are selected from among the acceptor framework positions of the second reference sequence that differ at the corresponding position compared to the donor framework positions of the first reference sequence; and b) a second population of oligonucleotides, each encoding i) at least one modified complementarity-determining region, or portion thereof, wherein the modified complementarity-determining region, or portion thereof, comprises a different amino acid at one or more positions when compared to the corresponding donor complementarity-determining region amino acid reference sequence and ii) one or more portions of adjacent framework regions which are capable of hybridizing to the first population of oligonucleotides; and c) mixing the first and second populations of oligonucleotides so as to create overlapping oligonucleotides; and d) extending the overlapping oligonucleotides with a DNA polymerase under conditions such that a population of altered heavy chain variable region encoding nucleic acids is constructed.

In still other embodiments, the present invention provides methods of constructing a population of altered light chain variable region encoding nucleic acids, comprising: a) providing a representation of first and second reference amino acid sequences, the first reference amino acid sequence comprising the sequence of a donor light chain variable region, the donor variable region comprising i) framework regions and ii) three complementarity-determining regions as defined by the combined definitions of Kabat and Chothia; the second reference amino acid sequence comprising the sequence of an acceptor light chain variable region comprising framework regions; b) synthesizing a) a first population of oligonucleotides, comprising oligonucleotides encoding a modified light chain variable region framework region, or portion thereof, wherein the light chain variable region framework region, or portion thereof, contains a plurality of changed amino acids at one or more positions when compared to the acceptor framework region reference sequence, wherein the framework positions that are changed are selected from among the acceptor framework positions of the second reference sequence that differ at the corresponding position compared to the donor framework positions of the first reference sequence; and b) a second population of oligonucleotides, each encoding i) at least one modified complementarity-determining region, or portion thereof, wherein the modified complementarity-determining region, or portion thereof, comprises a different amino acid at one or more positions when compared to the corresponding donor complementarity-determining region amino acid reference sequence and ii) one or more portions of adjacent framework regions which are capable of hybridizing to the first population of oligonucleotides; and c) mixing the first and second populations of oligonucleotides so as to create overlapping oligonucleotides; and d) extending the overlapping oligonucleotides with a DNA polymerase under conditions such that a population of altered light chain variable region encoding nucleic acids is constructed.

In some embodiments, one or more modifications are introduced into the framework, simultaneously with the introduction of one or more modified CDRs. The modified CDRs may comprise one or more amino acid alterations in comparison with the corresponding CDR of a reference sequence. In certain embodiments, the methods of constructing a population of altered heavy chain variable region encoding nucleic acids, comprises: a) providing a representation of first and second reference amino acid sequences, the first reference amino acid sequence comprising the sequence of a donor heavy chain variable region, the donor variable region comprising i) framework regions and ii) three complementarity-determining regions as defined by the combined definitions of Kabat and Chothia; the second reference amino acid sequence comprising the sequence of an acceptor heavy chain variable region comprising framework regions; b) synthesizing i) a first population of oligonucleotides, each encoding at least one modified complementarity-determining region, wherein the modified complementarity-determining region comprises a different amino acid at one or more positions when compared to the corresponding donor complementarity-determining region amino acid reference sequence; and ii) a second population of oligonucleotides, comprising oligonucleotides encoding modified portions of a heavy chain variable region framework, the modified portion containing a plurality of changed amino acids at one or more positions when compared to the acceptor framework region reference sequence, wherein the framework positions that are changed are selected from among the acceptor framework positions of the second reference sequence that differ at the corresponding position compared to the donor framework positions of the first reference sequence; c) mixing the first and second populations of oligonucleotides under conditions such that at least a portion of the oligonucleotides hybridize so as to create overlapping oligonucleotides; and d) treating the overlapping oligonucleotides under conditions such that a population of altered heavy chain variable region encoding nucleic acids is constructed. In certain embodiments, the representation of first and second reference sequences is in electronic form. In further embodiments, the methods further comprise the step of (e) coexpressing the population of altered heavy chain variable region encoding nucleic acids with a light chain variable region encoding nucleic acid so as to produce a diverse population of altered heteromeric variable regions. In other embodiments, the acceptor is human.

In other embodiments, the present invention provides methods constructing a population of altered light chain variable region encoding nucleic acids, comprising: a) providing a representation of first and second reference amino acid sequences, the first reference amino acid sequence comprising the sequence of a donor light chain variable region, the donor variable region comprising i) framework regions and ii) three complementarity-determining regions as defined by the combined definitions of Kabat and Chothia; the second reference amino acid sequence comprising the sequence of an acceptor light chain variable region comprising framework regions; b) synthesizing i) a first population of oligonucleotides, each encoding at least one modified complementarity-determining region, wherein the modified complementarity-determining region comprises a different amino acid at one or more positions when compared to the corresponding donor complementarity-determining region amino acid reference sequence; and ii) a second population of oligonucleotides, comprising oligonucleotides encoding modified portions of a light chain variable region framework, the modified portion containing a plurality of changed amino acids at one or more positions when compared to the acceptor framework region reference sequence, wherein the framework positions that are changed are selected from among the acceptor framework positions of the second reference sequence that differ at the corresponding position compared to the donor framework positions of the first reference sequence; c) mixing the first and second populations of oligonucleotides under conditions such that at least a portion of the oligonucleotides hybridize so as to create overlapping oligonucleotides; and d) treating the overlapping oligonucleotides under conditions such that a population of altered light chain variable region encoding nucleic acids is constructed.

In certain embodiments, the antibodies or antibody fragments comprising an Fc variant and an altered heavy chain variant region may be generated. For example, in some embodiments, the present invention provides methods of constructing a population of altered heavy chain variable region encoding nucleic acids, comprising: a) providing a representation of first and second reference amino acid sequences, the first reference sequence comprising the sequence of a donor heavy chain variable region, the donor variable region comprising i) framework regions and ii) three complementarity-determining regions as defined by the combined definitions of Kabat and Chothia; the second reference sequence comprising the sequence of an acceptor heavy chain variable region comprising framework regions; b) synthesizing A) first oligonucleotides encoding portions of the framework regions of the acceptor heavy chain variable region, wherein the portions of the framework regions when compared to the second reference sequence are unmodified; and B) a population of second oligonucleotides, each encoding i) at least a portion of a first complementarity-determining region that has been modified, the first complementarity-determining region selected from the group consisting of HCDR1, HCDR2 and HCDR3, wherein the modified first complementarity-determining region comprises a different amino acid at one or more positions when compared to the corresponding donor complementarity determining regions of the first reference sequence and ii) one or more portions of unmodified framework regions which are capable of hybridizing to the first oligonucleotides; c) mixing the first oligonucleotides with the population of second oligonucleotides as to create overlapping oligonucleotides; and d) treating the overlapping oligonucleotides under conditions such that a population of altered heavy chain variable region encoding nucleic acids is constructed, wherein the framework regions encoded by the altered heavy chain variable region encoding nucleic acids are unmodified with respect to the second reference sequence. In some embodiments, the methods further comprise the step of (E) coexpressing the population of altered heavy chain variable region encoding nucleic acids with a light chain variable region encoding nucleic acid so as to produce a diverse population of altered heteromeric variable regions.

In other embodiments, the present invention provides methods of constructing a population of altered light chain variable region encoding nucleic acids, comprising: a) providing a representation of first and second reference amino acid sequences, the first reference sequence comprising the sequence of a donor light chain variable region, the donor variable region comprising i) framework regions and ii) three complementarity-determining regions as defined by the combined definitions of Kabat and Chothia; the second reference sequence comprising the sequence of an acceptor light chain variable region comprising framework regions; b) synthesizing a) first oligonucleotides encoding portions of the framework regions of the acceptor light chain variable region, wherein the portions of the framework regions when compared to the second reference sequence are unmodified; and b) a population of second oligonucleotides, each encoding i) at least a portion of a first complementarity-determining region that has been modified, the first complementarity-determining region selected from the group consisting of LCDR1, LCDR2 and LCDR3, wherein the modified first complementarity-determining region comprises a different amino acid at one or more positions when compared to the corresponding donor complementarity determining regions of the first reference sequence and ii) one or more portions of unmodified framework regions which are capable of hybridizing to the first oligonucleotides; c) mixing the first oligonucleotides with the population of second oligonucleotides as to create overlapping oligonucleotides; and d) treating the overlapping oligonucleotides under conditions such that a population of altered light chain variable region encoding nucleic acids is constructed, wherein the framework regions encoded by the altered light chain variable region encoding nucleic acids are unmodified with respect to the second reference sequence.

In other embodiments, the present invention provides methods of constructing a population of altered heavy chain variable region encoding nucleic acids, comprising: A) providing a representation of first and second reference amino acid sequences, the first reference sequence comprising the sequence of a donor heavy chain variable region, the donor variable region comprising i) framework regions and ii) three complementarity-determining regions as defined by the combined definitions of Kabat and Chothia; the second reference sequence comprising the sequence of an acceptor heavy chain variable region comprising framework regions; B) synthesizing a) a population of first oligonucleotides, each encoding at least a portion of a first complementarity-determining region selected from the group consisting of HCDR1, HCDR2 and HCDR3, wherein the modified first complementarity-determining region comprises a different amino acid at one or more positions when compared to the corresponding donor complementarity determining regions of the first reference sequence; and b) second oligonucleotides encoding i) portions of the framework regions of the acceptor heavy chain variable region, wherein the portions of the framework regions when compared to the reference sequence are unmodified and ii) one or more portions of a complementarity determining region which are capable of hybridizing to the population of first oligonucleotides; C) mixing the population of first oligonucleotides with the second oligonucleotides as to create overlapping oligonucleotides; and D) treating the overlapping oligonucleotides under conditions such that a population of altered heavy chain variable region encoding nucleic acids is constructed, wherein the framework regions encoded by the altered heavy chain variable region encoding nucleic acids are unmodified with respect to the second reference sequence. In certain embodiments, the methods further comprise the step of (E) coexpressing the population of altered heavy chain variable region encoding nucleic acids with a light chain variable region encoding nucleic acid so as to produce a diverse population of altered heteromeric variable regions.

In other embodiments, the present invention provides methods of constructing a population of altered light chain variable region encoding nucleic acids, comprising: A) providing a representation of first and second reference amino acid sequences, the first reference sequence comprising the sequence of a donor light chain variable region, the donor variable region comprising i) framework regions and ii) three complementarity-determining regions as defined by the combined definitions of Kabat and Chothia; the second reference sequence comprising the sequence of an acceptor light chain variable region comprising framework regions; B) synthesizing a) a population of first oligonucleotides, each encoding at least a portion of a first complementarity-determining region selected from the group consisting of LCDR1, LCDR2 and LCDR3, wherein the modified first complementarity-determining region comprises a different amino acid at one or more positions when compared to the corresponding donor complementarity determining regions of the first reference sequence; and b) second oligonucleotides encoding i) portions of the framework regions of the acceptor light chain variable region, wherein the portions of the framework regions when compared to the reference sequence are unmodified and ii) one or more portions of a complementarity determining region which are capable of hybridizing to the population of first oligonucleotides; C) mixing the population of first oligonucleotides with the second oligonucleotides as to create overlapping oligonucleotides; and D) treating the overlapping oligonucleotides under conditions such that a population of altered light chain variable region encoding nucleic acids is constructed, wherein the framework regions encoded by the altered light chain variable region encoding nucleic acids are unmodified with respect to the second reference sequence.

In other embodiments, the present invention provides methods of improving the binding affinity of a mutated humanized antibody variable region, comprising: a) providing a nucleic acid sequence encoding a first mutated humanized antibody variable region, the mutated variable region comprising (i) a wild type human antibody framework, (ii) three non-human heavy chain complementarity determining regions, and (iii) three non-human light chain complementarity determining regions, wherein the complementarity determining regions are defined by the combined definitions of Kabat and Chothia, wherein at least one of the light chain complementarity determining regions is a mutation-containing light chain complementarity determining region comprising at least one different amino acid at at least one position when compared to the corresponding wild type non-human complementarity determining region, and wherein the first mutated antibody variable region has a higher binding affinity than the corresponding non-mutated antibody variable region; b) mutating the nucleic acid sequence encoding the first mutated antibody variable region under conditions such that a second mutated humanized antibody variable region is encoded, the second mutated humanized antibody variable region comprising at least one additional different amino acid at least one position in the mutation-containing light chain complementarity determining region, the additional mutation in combination with the first mutation resulting in higher binding affinity. In some embodiments, the mutation-containing light chain complementarity determining region of the first mutated humanized antibody variable region is complementarity determining region 3 (LCDR3). In other embodiments, at least one of the non-human heavy chain complementarity determining regions of the first mutated humanized antibody variable region comprises a mutation, such that a different amino acid is encoded at at least one position when compared to the corresponding wild type non-human complementarity determining region. In additional embodiments, the heavy chain complementarity determining region mutation is in HCDR3.

In some embodiments, the present invention provides methods of of simultaneously modifying at least one complementarity-determining region (CDR) and at least one framework region (FR) while constructing a population of altered heavy chain variable region encoding nucleic acids, comprising: a) providing a representation of first and second reference amino acid sequences, the first reference amino acid sequence comprising the sequence of a donor heavy chain variable region, the donor variable region comprising i) four framework regions and ii) three complementarity-determining regions as defined by the combined definitions of Kabat and Chothia; the second reference amino acid sequence comprising the sequence of an acceptor heavy chain variable region comprising four framework regions, as defmed by the combined definitions of Kabat and Chothia; b) synthesizing i) for every framework region to be modified, a population of oligonucleotides, each encoding a modified framework region, or portion thereof, the modified framework region, or portion thereof, containing a plurality of changed amino acids at one or more positions when compared to the corresponding framework region in the acceptor heavy chain variable region reference sequence, wherein the framework region positions that are changed are selected from among the acceptor framework positions of the second reference sequence that differ at the corresponding position compared to the donor framework region positions of the first reference sequence; and ii) for every complementarity-determining region to be modified, a population of oligonucleotides, each encoding a modified complementarity-determining region, or portion thereof, wherein the modified complementarity-determining region comprises a different amino acid at one or more positions when compared to the corresponding donor complementarity-determining region amino acid reference sequence; and iii) for each of any remaining and unmodified framework regions, oligonucleotides encoding the framework region, or portion thereof, having the same sequence as the corresponding framework region of the second reference acceptor sequence; and iv) for each of any remaining and unmodified complementarity-determining regions, oligonucleotides encoding the complementarity-determining region, or portion thereof, having the same sequence as the corresponding complementarity-determining region of the first reference donor sequence, wherein, individual oligonucleotides from (i) through (iv) which encode adjacent portions of the heavy chain variable region have overlapping sequences at their termini; and c) mixing the oligonucleotides and populations of oligonucleotides synthesized in step b) under conditions such that the overlapping sequences of individual oligonucleotides hybridize so as to create overlapping oligonucleotides; and d) treating the overlapping oligonucleotides under conditions such that a population of altered heavy chain variable region encoding nucleotides is formed. In certain emobodiments, the representation of first and second reference sequences is in electronic form. In further embodiments, the framework region to be modified is selected from the group consisting of HFR1, HFR2 and HFR3. In other embodiments, the complementarity-determining region to be modified is HCDR3. In other embodiments, the method further comprises the step of e) coexpressing the population of heavy chain variable region encoding nucleic acids with a light chain variable region encoding nucleic acid so as to produce a diverse population of altered heteromeric variable regions. In different embodiments, the method further comprises the step of e) coexpressing the population of heavy chain variable region encoding nucleic acids with a population of light chain variable region encoding nucleic acids so as to produce a diverse population of altered heteromeric variable regions.

In other embodiments, the methods of simultaneously modifying at least one complementarity-determining region (CDR) and at least one framework region (FR) while constructing a population of altered light chain variable region encoding nucleic acids are employed, wherein said method comprises: a) providing a representation of first and second reference amino acid sequences, the first reference amino acid sequence comprising the sequence of a donor light chain variable region, the donor variable region comprising i) four framework regions and ii) three complementarity-determining regions as defined by the combined definitions of Kabat and Chothia; the second reference amino acid sequence comprising the sequence of an acceptor light chain variable region comprising four framework regions, as defined by the combined definitions of Kabat and Chothia; b) synthesizing i) for every framework region to be modified, a population of oligonucleotides, each encoding a modified framework region, or portion thereof, the modified framework region, or portion thereof, containing a plurality of changed amino acids at one or more positions when compared to the corresponding framework region in the acceptor light chain variable region reference sequence, wherein the framework region positions that are changed are selected from among the acceptor framework positions of the second reference sequence that differ at the corresponding position compared to the donor framework region positions of the first reference sequence; and ii) for every complementarity-determining region to be modified, a population of oligonucleotides, each encoding a modified complementarity-determining region, or portion thereof, wherein the modified complementarity-determining region comprises a different amino acid at one or more positions when compared to the corresponding donor complementarity-determining region amino acid reference sequence; and iii) for each of any remaining and unmodified framework regions, oligonucleotides encoding the framework region, or portion thereof, having the same sequence as the corresponding framework region of the second reference acceptor sequence; and iv) for each of any remaining and unmodified complementarity-determining regions, oligonucleotides encoding the complementarity-determining region, or portion thereof, having the same sequence as the corresponding complementarity-determining region of the first reference donor sequence, wherein, individual oligonucleotides from (i) through (iv) which encode adjacent portions of the light chain variable region have overlapping sequences at their termini, and c) mixing the oligonucleotides and populations of oligonucleotides synthesized in step b) under conditions such that the overlapping sequences of individual oligonucleotides hybridize so as to create overlapping oligonucleotides; and d) treating the overlapping oligonucleotides under conditions such that a population of altered light chain variable region encoding nucleotides is formed. In certain embodiments, the methods further comprise the step of e) coexpressing the population of light chain variable region encoding nucleic acids with a population of heavy chain variable region encoding nucleic acids so as to produce a diverse population of altered heteromeric variable regions.

### (v) Multispecific antibodies

The present invention provides multispecific antibodies comprising a variant Fc region. Multispecific antibodies have binding specificities for at least two different antigens. While such molecules normally will only bind two antigens (i.e. bispecific antibodies, BsAbs), antibodies with additional specificities such as trispecific antibodies are encompassed by this expression when used herein. Examples of BsAbs include, but are not limited to, those with one arm directed against a tumor cell antigen and the other arm directed against a cytotoxic trigger molecule such as anti-FcγRI/anti- CD15, anti-p185HER2/FcyRIII (CD16), anti-CD3/anti-malignant B-cell (1D10), anti-CD3/antip185HER2, anti-CD3/anti-p97, anti-CD3/anti-renal cell carcinoma, anti-CD3/anti-OVCAR-3, antiCD3/L-D1 (anti-colon carcinoma), anti-CD3/anti-melanocyte stimulating hormone analog, anti EGF receptor/anti-CD3, anti-CD3/anti-CAMA1, anti-CD3/anti-CD19, anti-CD3/MoV18, anti-neural cell adhesion molecule (NCAM)/anti-CD3, anti-folate binding protein (FBP)/anti-CD3, anti-pan carcinoma associated antigen (AMOC-31)/anti-CD3; BsAbs with one arm which binds specifically to a tumor antigen and one arm which binds to a toxin such as anti-saporin/anti-id-1, antiCD22/anti-saporin, anti-CD7/anti-saporin, anti-CD38/anti-saporin, anti-CEA/anti-ricin A chain, anti-interferon-a (IFN-a)/anti-hybridoma idiotype, anti-CEA/anti-vinca alkaloid; BsAbs for converting enzyme activated prodrugs such as anti-CD30/anti-alkaline phosphatase (which catalyzes conversion of mitomycin phosphate prodrug to mitomycin alcool); BsAbs which can be used as fibrinolytic agents such as anti-fibrin/anti-tissue plasminogen activator (tPA), anti-fibrin/antiurokinase-type plasminogen activator (uPA); BsAbs for targeting immune complexes to cell surface receptors such as anti-low density lipoprotein (LDL)/anti-Fc receptor (e.g. FcγRI, FcγRII or FcγRIII); BsAbs for use in therapy of infectious diseases such as anti-CD3/anti-herpes simplex virus (HSV), anti-T-cell receptor: CD3 complex/anti-influenza, anti-FcyR/anti-HIV; BsAbs for tumor detection in vitro or in vivo such as anti-CEA/anti-EOTUBE, anti-CEA/anti-DPTA, anti-p185HER2/anti-hapten; BsAbs as vaccine adjuvants; and BsAbs as diagnostic tools such as anti-rabbit IgG/anti-ferritin, anti-horse radish peroxidase (HRP)/anti-hormone, anti-somatostatin/anti-substance P, anti-HRP/anti-FITC, anti-CEA/anti-p-galactosidase. Examples of trispecific antibodies include, but are not limited to, anti-CD3/anti-CD4/anti-CD37, anti-CD3/anti-CD5/anti-CD37 and anti-CD3/anti-CD8/anti-CD37. Bispecific antibodies can be prepared as full length antibodies or antibody fragments (e.g. F (ab') 2 bispecific antibodies).

Methods for making bispecific antibodies are known in the art. Traditional production of full length bispecific antibodies is based on the coexpression of two immunoglobulin heavy chain-light chain pairs, where the two chains have different specificities (e.g., Millstein et al., Nature, 305: 537-539 (1983), herein incorporated by reference). Because of the random assortment of immunoglobulin heavy and light chains, these hybridomas (quadromas) produce a potential mixture of 10 different antibody molecules, of which only one has the correct bispecific structure. Purification of the correct molecule may be performed by affinity chromatography steps. Similar procedures are disclosed in WO 93/08829, and in Traunecker et al., EMBO J., 10: 3655-3659 (1991), both of which are hereby incorporated by reference.

In another approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance. In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm (see, e.g., WO 94/04690, herein incorporated by reference). According to another approach described in W096/27011 (hereby incorporated by reference), the interface between a pair of antibody molecules can be engineered to maximize the percentage of heterodimers which are recovered from recombinant cell culture. Bispecific antibodies also include cross-linked or "heteroconjugate" antibodies. For example, one of the antibodies in the heteroconjugate can be coupled to avidin, the other to biotin.

### B. Immunoadhesin Molecules

The present invention also provides immunoadhesin molecules comprising a variant Fc region. One type of immunoadhesin design combines the binding domain(s) of the adhesin (e.g. the extracellular domain (ECD) of a receptor) with the Fc region of an immunoglobulin heavy chain (e.g., a variant Fc region). Ordinarily, when preparing the immunoadhesins of the present invention, nucleic acid encoding the binding domain of the adhesin will be fused C-terminally to nucleic acid encoding the N-terminus of an immunoglobulin constant domain sequence, however N-terminal fusions are also possible.

Typically, in such fusions the encoded chimeric polypeptide will retain at least functionally active hinge, CH2 and CH3 domains of the constant region of an immunoglobulin heavy chain. Fusions are also made to the C-terminus of the Fc portion of a constant domain, or immediately N-terminal to the CH1 of the heavy chain or the corresponding region of the light chain. The precise site at which the fusion is made is not critical; particular sites are well known and may be selected in order to optimize the biological activity, secretion, or binding characteristics of the immunoadhesin.

In some embodiments, the adhesin sequence is fused to the N-terminus of the variant Fc region of immunoglobulin G₁. It is possible to fuse the entire heavy chain constant region to the adhesin sequence. However, in preferred embodiments, a sequence beginning in the hinge region just upstream of the papain cleavage site which defines IgG Fc chemically (i.e. residue 216, taking the first residue of heavy chain constant region to be 114), or analogous sites of other immunoglobulins is used in the fusion. In certain preferred embodiments, the adhesin amino acid sequence is fused to (a) the hinge region and CH2 and CH3 or (b) the CH1, hinge, CH2 and CH3 domains, of an IgG heavy chain. In some embodiments, the immunoadhesins are bispecific.

Alternatively, the adhesin sequences can be inserted between immunoglobulin heavy chain and light chain sequences, such that an immunoglobulin comprising a chimeric heavy chain is obtained. In such embodiments, the adhesin sequences may be fused to the 3' end of an immunoglobulin heavy chain in each arm of an immunoglobulin, either between the hinge and the CH2 domain, or between the CH2 and CH3 domains (see, e.g., Hoogenboom et al., Mol. Immunol. 28:1027-1037 (1991), herein incorporated by reference).

Although the presence of an immunoglobulin light chain is not required in the immunoadhesins of the present invention, an immunoglobulin light chain might be present either covalently associated to an adhesin-immunoglobulin heavy chain fusion polypeptide, or directly fused to the adhesin. In the former case, DNA encoding an immunoglobulin light chain is typically coexpressed with the DNA encoding the adhesin-immunoglobulin heavy chain fusion protein. Upon secretion, the hybrid heavy chain and the light chain will be covalently associated to provide an immunoglobulin-like structure comprising two disulfide-linked immunoglobulin heavy chain-light chain pairs. Methods suitable for the preparation of such structures are, for example, disclosed in U. S. Patent No. 4,816,567, herein incorporated by reference.

In preferred embodiments, immunoadhesins are constructed by fusing the cDNA sequence encoding the adhesin portion in-frame to an immunoglobulin cDNA sequence. However, fusion to genomic immunoglobulin fragments can also be used. Generally, the latter type of fusion requires the presence of Ig regulatory sequences for expression. cDNAs encoding IgG heavy chain constant regions can be isolated based on published sequences from cDNA libraries derived from spleen or peripheral blood lymphocytes, by hybridization or by polymerase chain reaction (PCR) techniques. The cDNAs encoding the "adhesin" and the immunoglobulin parts of the immunoadhesin may be inserted in tandem into a plasmid vector that directs efficient expression in the chosen host cells.

### VI. Nucleic Sequences Encoding Fc Region Variants

The present invention also provides nucleic acid sequences encoding Fc region variants, as well as compositions, vectors, and host cells comprising nucleic acid sequences encoding Fc region variants. The present invention also provides recombinant methods for producing Fc region variants.

Generally, for recombinant production of variants, nucleic acid encoding the variant is isolated and inserted into a vector. Host cells may be transfected with the vector, thereby allowing the nucleic acid sequence to be amplified, and/or the variant peptide produced. Nucleic acid sequences encoding the peptide variants of the present invention may be isolated and sequenced using conventional procedures (e.g. using oligonucleotide probes that are capable of binding specifically to nucleic acid encoding the variant). Generally, the nucleic acid sequence encoding the variant is operably linked to other elements, such as a signal sequence (e.g. secretory signal sequences), an origin of replication, at least one marker gene, an enhancer, a promoter, or a transcription terminator. In certain embodiments, host cells are stably transfected with nucleic acid encoding a variant to generate a cell line expressing a particular variant. In preferred embodiments, the variants are expressed in CHO, NSO, Sp2/0, PER.C6, or HEK293 cells. Recombinant methods are well known in the art.

Nucleic acid sequences may be mutated such that variant Fc regions may be produced. For example, a nucleic acid sequence encoding a parental Fc region (e.g. SEQ ID NO:32) may be mutated such that at least one amino acid change results when the nucleic acid sequence is expressed. Also, nucleic acid sequences encoding at least a portion of a parental Fc region may mutated to produce amino acid sequences comprising at least a portion of an Fc region variant. For example, SEQ ID NO:32 may be mutated, such that at least one amino acid sequence results (See, e.g., SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, and SEQ ID NO:56).

In certain embodiments, codon-based synthesis is employed to generate mutated sequences. Examples of codon-based synthesis include, for example, those described in U.S. Patents 5,264,563, 5,523,388 and 5,808,022, all of which are hereby incorporated by reference. Briefly, codon-based synthesis may be performed by sequentially coupling monomers on separate supports to form at least two different tuplets. The coupling may be performed in separate reaction vessels, then mixing the supports from the reaction vessels, and dividing the mixed supports into two or more separate reaction vessels, and repeating the coupling, mixing and dividing steps one or more times in the reaction vessels, ending with a mixing or dividing step. Additionally, the oligonucleotides can be cleaved from the supports.

### VII. Therapeutic Uses and Formulations

In some embodiments, the present invention provides therapeutic formulations comprising the variants described herein. It is not intended that the present invention be limited by the particular nature of the therapeutic composition. For example, such compositions can include a polypeptide variant (or portion thereof), provided together with physiologically tolerable liquids, gels, solid carriers, diluents, adjuvants and excipients, and combinations thereof (See, e.g, Remington's Pharmaceutical Sciences 16th edition, Osol, A. Ed. (1980), herein incorporated by reference).

In addition, polypeptide variants may be used together with other therapeutic agents, including, but not limited to, salicylates, steroids, immunosuppressants, antibodies or antibiotics. Particular therapeutic agents which may be used with the variants of the present invention include, but are not limited to, the following agents: azobenzene compounds (US Pat. No. 4,312,806, incorporated herein by reference), benzyl-substituted rhodamine derivatives (US Pat. No. 5,216,002, incorporated herein by reference), zinc L-carnosine salts (US Pat. No. 5,238,931, incorporated herein by reference), 3-phenyl-5-carboxypyrazoles and isothiazoles (US Pat. No. 5,294,630, incorporated herein by reference), IL-10 (US Pat. No. 5,368,854, incorporated herein by reference), quinoline leukotriene synthesis inhibitors (US Pat. No. 5,391,555, incorporated herein by reference), 2'-halo-2'deoxy adenosine (US Pat. No. 5,506,213, incorporated herein by reference), phenol and benzamide compounds (US Pat. No. 5,552,439, incorporated herein by reference), tributyrin (US Pat. No. 5,569,680, incorporated herein by reference), certain peptides (US Pat. No. 5,756,449, incorporated herein by reference), omega-3 polyunsaturated acids (US Pat. No. 5,792,795, incorporated herein by reference), VLA-4 blockers (US Pat. No. 5,932,214, incorporated herein by reference), prednisolone metasulphobenzoate (US Pat. No. 5,834,021, incorporated herein by reference), cytokine restraining agents (US Pat. No. 5,888,969, incorporated herein by reference), and nicotine (US Pat. No. 5,889,028, incorporated herein by reference).

Polypeptide variants may be used together with agents which reduce the viability or proliferative potential of a cell. Agents which reduce the viability or proliferative potential of a cell can function in a variety of ways including, for example, inhibiting DNA synthesis, inhibiting cell division, inducing apoptosis, or inducing non-apoptotic cell killing. Specific examples of cytotoxic and cytostatic agents, include but are not limited to, pokeweed antiviral protein, abrin, ricin, and each of their A chains, doxorubicin, cisplastin, iodine-131, yttrium-90, rhenium-188, bismuth-212, taxol, 5-fluorouracil VP-16, bleomycin, methotrexate, vindesine, adriamycin, vincristine, vinblastine, BCNU, mitomycin and cyclophosphamide and certain cytokines such as TNF-α and TNF-β. Thus, cytotoxic or cytostatic agents can include, for example, radionuclides, chemotherapeutic drugs, proteins, and lectins.

Therapeutic compositions may contain, for example, such normally employed additives as binders, fillers, carriers, preservatives, stabilizing agents, emulsifiers, buffers and excipients as, for example, pharmaceutical grades of mannitol, lactose, starch, magnesium stearate, sodium saccharin, cellulose, magnesium carbonate, and the like. These compositions typically contain 1%-95% of active ingredient, preferably 2%-70% active ingredient.

The polypeptide variants of the present invention can also be mixed with diluents or excipients which are compatible and physiologically tolerable. Suitable diluents and excipients are, for example, water, saline, dextrose, glycerol, or the like, and combinations thereof. In addition, if desired, the compositions may contain minor amounts of auxiliary substances such as wetting or emulsifying agents, stabilizing or pH buffering agents.

In some embodiments, the therapeutic compositions of the present invention are prepared either as liquid solutions or suspensions, as sprays, or in solid forms. Oral formulations usually include such normally employed additives such as binders, fillers, carriers, preservatives, stabilizing agents, emulsifiers, buffers and excipients as, for example, pharmaceutical grades of mannitol, lactose, starch, magnesium stearate, sodium saccharin, cellulose, magnesium carbonate, and the like. These compositions take the form of solutions, suspensions, tablets, pills, capsules, sustained release formulations, or powders, and typically contain 1%-95% of active ingredient, preferably 2%-70%. One example of an oral composition useful for delivering the therapeutic compositions of the present invention is described in US Pat. No. 5,643,602 (incorporated herein by reference).

Additional formulations which are suitable for other modes of administration, such as topical administration, include salves, tinctures, creams, lotions, transdermal patches, and suppositories. For salves and creams, traditional binders, carriers and excipients may include, for example, polyalkylene glycols or triglycerides. One example of a topical delivery method is described in U.S. Pat. No. 5,834,016 (incorporated herein by reference). Other liposomal delivery methods may also be employed (See, e.g., US Pat. Nos. 5,851,548 and 5,711,964, both of which are herein incorporated by reference).

The formulations may also contain more than one active compound as necessary for the particular indication being treated, preferably those with complementary activities that do not adversely affect each other. Such molecules are suitably present in combination in amounts that are effective for the purpose intended.

Sustained-release preparations may also be prepared. Suitable examples of sustained release preparations include semipermeable matrices of solid hydrophobic polymers containing the polypeptide variant, which matrices are in the form of shaped articles, e.g., films, or microcapsule. Examples of sustained-release matrices include, but are not limited to, polyesters, hydrogels (for example, poly (2-hydroxyethylmethacrylate), or poly (vinylalcohol)), polylactides, copolymers of L-glutamic acid and y ethyl-L-glutamate, non-degradable ethylene-vinyl acetate, degradable lactic acid-glycolic acid copolymers such as the LUPRON DEPOT (injectable microspheres composed of lactic acid-glycolic acid copolymer and leuprolide acetate), and poly-D- (-)-3-hydroxybutyric acid. While polymers such as ethylene-vinyl acetate and lactic acid-glycolic acid enable release of molecules for over 100 days, certain hydrogels release proteins for shorter time periods.

The polypeptide variants of the present invention may be used to treat a subject. Such treatment may be administered to a subject with a disease, or may be administered prophylactically to a subject (e.g. to a subject predisposed to a disease). Example of conditions that may be treated include, but are not limited to, cancer (e.g. where the polypeptide variant binds the HER2 receptor, CD20 or vascular endothelial growth factor (VEGF)); allergic conditions such as asthma (with an anti-IgE antibody); and LFA-1-mediated disorders (e.g. where the polypeptide variant is an anti-LFA-1 or anti-ICAM-1 antibody) etc.

In preferred embodiments, the variants used to treat subjects comprise antibodies or immunoadhesins. Also in preferred embodiments, the diseases treated are antibody or immunoadhesin responsive diseases. Examples of antibody responsive diseases include diseases and medical conditions such as: lymphoma (shown to be treatable with RITUXAN, an anti-CD20 antibody), infectious disease (shown to be treatable with SYNAGIS, an antibody directed to the F protein of respiratory syncytial virus), kidney transplant (ZENAPAX, an anti-IL-2 receptor antibody, has shown to be helpful), Crohn's disease and rheumatoid arthritis (shown to be treatable with REMICADE, an anti-TNF alpha antibody), breast carcinoma (shown to be treatable with HERCEPTIN, an anti-HER2 antibody), and colon cancer (shown to be treatable with EDRECOLOMAB, an anti-17-1A antibody).

In some embodiments, a polypeptide variant with improved ADCC activity (e.g. the P247L or I332E variant) is employed in the treatment of diseases or disorders where destruction or elimination of tissue or foreign microorganisms is desired. For example, the variant may be used to treat cancer; inflammatory disorders; infections (e.g. bacterial, viral, fungal or yeast infections); and other conditions (such as goiter) where removal of tissue is desired. In other embodiments, the polypeptide variant has diminished ADCC activity (e.g. the I332R or I332K variant). Such variants may be used to treat diseases or disorders where an Fc region-containing polypeptide with long half-life is desired, but the polypeptide preferably does not have undesirable effector function(s). For example, the Fc region-containing polypeptide may be an anti-tissue factor (TF) antibody; anti-IgE antibody; and anti-integrin antibody (e.g. an anti-α4β7 antibody). The desired mechanism of action of such Fc region-containing polypeptides may be to block ligand-receptor binding pairs. Moreover, the Fc-region containing polypeptide with diminished ADCC activity may be an agonist antibody.

The polypeptide variants of the present invention may be administered by any suitable means, including parenteral, subcutaneous, topical, intraperitoneal, intrapulmonary, and intranasal, and, intralesional administration (e.g. for local immunosuppressive treatment). Parenteral infusions include intramuscular, intravenous, intraarterial, intraperitoneal, or subcutaneous administration. In addition, the polypeptide variant is suitably administered by pulse infusion, particularly with declining doses of the polypeptide variant. Preferably, the dosing is given by injections, most preferably intravenous or subcutaneous injections, depending in part on whether the administration is brief or chronic.

For the prevention or treatment of disease, the appropriate dosage of polypeptide variant will depend on the type of disease to be treated, the severity and course of the disease, whether the polypeptide variant is administered for preventive or therapeutic purposes, previous therapy, the patient's clinical history and response to the polypeptide variant, and the discretion of the attending physician. The polypeptide variant is suitably administered to the patient at one time or over a series of treatments.

For example, depending on the type and severity of the disease, about 1 µg/kg to 15 mg/kg (e.g., 0.120 mg/kg) of polypeptide variant is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until the symptoms are sufficiently reduced or eliminated. The progress of this therapy is easily monitored by conventional techniques and assays, and may be used to adjust dosage to achieve a therapeutic effect.

A therapeutically effective amount of a polypeptide variant to be administered is the dosage level required for a patient such that the symptoms of the disease being treated are reduced. The polypeptide variant need not be, but is optionally formulated with one or more agents currently used to prevent or treat the disorder in question. The effective amount of such other agents depends on the amount of polypeptide variant present in the formulation, the type of disorder or treatment, and other factors discussed above.

### VIII. Additional Variant Fc Region Uses

The variants, and nucleic acid sequences encoding variants, of the present invention may be used in many ways. For example, variants of the present invention may be used in drug screening assays. For example, candidate compounds may be evaluated for their ability to alter or interfere with Fc effector functions by contacting a variant with the candidate compound and determining binding of the candidate compound to the variant. The variant may be immobilized using methods known in the art such as binding a GST-variant fusion protein to a polymeric bead containing glutathione. A chimeric gene encoding a GST fusion protein is constructed by fusing DNA encoding the variant of interest to the DNA encoding the carboxyl terminus of GST (See e.g., Smith et al., Gene 67:31 [1988]). The fusion construct is then transformed into a suitable expression system (e.g., *E*.*coli* XA90) in which the expression of the GST fusion protein can be induced with isopropyl-β-D-thiogalactopyranoside (IPTG). Induction with IPTG should yield the fusion protein as a major constituent of soluble, cellular proteins. The fusion proteins can be purified by methods known to those skilled in the art, including purification by glutathione affinity chromatography. Binding of the candidate compound to the variant is correlated with the ability of the compound to disrupt the one or more effector functions.

In another screening method, either the variant or a selected FcR is immobilized using methods known in the art, such as adsorption onto a plastic microtiter plate or specific binding of a GST-fusion protein to a polymeric bead containing glutathione. For example, GST-variant is bound to glutathione-Sepharose beads. The immobilized variant is then contacted with an Fc receptor and a candidate compound. Unbound peptide is then removed and the complex solubilized and analyzed to determine the amount of bound labeled peptide. A decrease in binding is an indication that the candidate compound inhibits the interaction of variant with the Fc receptor. This screening method is particularly useful with variants of the present invention that show an increased level of Fc receptor binding (e.g. since many parental Fc receptors are low affinity receptors, such as FcγRIII, FcγRIIb, and FcγRIIa). A variation of this method allows for the screening of compounds that are capable of disrupting a previously-formed variant/Fc receptor complex. For example, in some embodiments a complex comprising a variant bound to an Fc receptor is immobilized as described above and contacted with a candidate compound. The dissolution of the complex by the candidate compound correlates with the ability of the compound to disrupt or inhibit the interaction between the variant being tested and the Fc receptor being. In this regard, compounds with therapeutic potential (e.g. in humans) may be identified (e.g. compounds useful in treating human disease, such as autoimmune diseases).

Another technique for drug screening provides high throughput screening for compounds having suitable binding affinity to variant peptides and is described in detail in WO 84/03564, incorporated herein by reference. Briefly, large numbers of different small peptide test compounds are synthesized on a solid substrate, such as plastic pins or some other surface. The peptide test compounds are then reacted with variant peptides and washed. Bound variant peptides are then detected by methods well known in the art.

Another technique uses antibodies directed to variant peptides. Such antibodies capable of specifically binding to variant peptides compete with a test compound for binding to a given variant. In this manner, the antibodies can be used to detect the presence of any peptide that shares one or more antigenic determinants of the variant peptide.

The present invention contemplates many other means of screening compounds. The examples provided above are presented merely to illustrate a range of techniques available. One of ordinary skill in the art will appreciate that many other screening methods can be used.

In particular, the present invention contemplates the use of cell lines transfected with nucleic acid encoding at least one Fc region variant for screening compounds for activity, and in particular to high throughput screening of compounds from combinatorial libraries (e.g., libraries containing greater than 10⁴ compounds). The cell lines of the present invention can be used in a variety of screening methods.

The variants of the present invention may be used as an affinity purification agent. For example, the variant may be immobilized on a solid phase such a Sephadex resin or filter paper, using methods well known in the art. The immobilized variant is then contacted with a sample containing the antigen to be purified, and thereafter the support is washed with a suitable solvent that will remove substantially all the material in the sample except the antigen to be purified, which is bound to the immobilized polypeptide variant. Finally, the support is washed with another suitable solvent, such as glycine buffer, pH 5.0, that will release the antigen from the polypeptide variant.

The polypeptide variant may also be useful in diagnostic assays (e.g., for detecting expression of an antigen of interest in specific cells, tissues, or serum). For diagnostic applications, the variant will typically be labeled with a detectable moiety (such labels are also useful in the Fc region assays described above). Numerous labels are available, including, but not limited to, radioisotopes (e.g., ³⁵S, ¹⁴C, ¹²⁵I, ³H, and ¹³¹I), Fluorescent labels (e.g. rare earth chelates (europium chelates) or fluorescein and its derivatives, rhodamine and its derivatives, dansyl, Lissamine, phycoerythrin and Texas Red), and various enzyme-substrate labels (see, e.g., U. S. Patent No. 4,275,149, herein incorporated by reference, and luciferase, luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like). Examples of enzyme-substrate combinations include, for example: (i) horseradish peroxidase (HRPO) with hydrogen peroxidase as a substrate, wherein the hydrogen peroxidase oxidizes a dye precursor (e.g., orthophenylene diamine (OPD) or 3,3', 5,5'-tetramethyl benzidine hydrochioride (TMB)); (ii) alkaline phosphatase (AP) with para-Nitrophenyl phosphate as chromogenic substrate; and (iii) -D-galactosidase (R-D-Gai) with a chromogenic substrate or fluorogenic substrate.

The variants of the present invention may also be used for in vivo diagnostic assays. For example, the polypeptide variant is labeled with a radionuclide so that the antigen or cells expressing it can be localized using immunoscintiography.

### EXPERIMENTAL

The following examples are provided in order to demonstrate and further illustrate certain preferred embodiments and aspects of the present invention and are not to be construed as limiting the scope thereof.

In the experimental disclosure which follows, the following abbreviations apply: N (normal); M (molar); mM (millimolar); µM (micromolar); mol (moles); mmol (millimoles); µmol (micromoles); nmol (nanomoles); pmol (picomoles); g (grams); mg (milligrams); µg (micrograms); ng (nanograms); 1 or L (liters); ml (milliliters); µl (microliters); cm (centimeters); mm (millimeters); µm (micrometers); nm (nanometers); DS (dextran sulfate); and C (degrees Centigrade)..

### EXAMPLE 1

### Screening Variants in ADCC Assays

This example describes how variants were screened in an ADCC assay. All variants screened were anti-CD20 antibodies (based on variable region specificity).

### i. PBMC (peripheral blood mononuclear cell) isolation

About 50 mL of peripheral blood is obtained from a healthy donor and diluted 1:2 with phosphate buffered saline (PBS), pH 7.0. The solutions are mixed by gently swirling the tube. About 12 mL of Histopaque-1077 (Sigma Cat. No. 1077-1) is carefully layered underneath the diluted blood sample followed by centrifugation in a Sorvall RT6000B centrifuge with swinging bucket rotor at 1000 rpm for 10 minutes with the brake turned off. The upper phase of the gradient is discarded by aspiration and the white-colored, PBMC-containing interphase collected and washed 3 times with Hanks' Balanced Salt Solution (Gibco Cat. No. 14025-092). The washed cell pellet is suspended in about 20 mL RPMI 1640 media containing 10% Fetal Bovine Serum (FBS) (Omega Scientific Cat. No. FB-01). The resuspended PBMCs are split into two T-175 culture flasks, and 30 mL of RPMI containing 10% FBS is added to each followed by incubation overnight in a 37°C 5% CO₂ incubator. The following day the nonadherent PBMCs are collected in 50 mL Falcon tubes, centrifuged as above and resuspended in RPMI containing 1% FBS lacking phenol red. A small portion of the resuspended cells are diluted 10-fold and counted using a hemocytometer. The remaining PBMCs are placed in the incubator until needed.

### ii. Target cell line (these are specific for anti-CD20 ADCC assays)

Wil.2 and SKW6.4 CD20-expressing B-cell lines were obtained from ATCC and grown as recommended. One day before use, the cells are split 2-fold. The next day the cell number is adjusted to 4 X 10⁵ cells/mL and 50 µL aliquots (20,000 cells/well) added to a 96-well tissue culture plate.

### iii. IgG dilutions

Prior to screening, IgG variants are expressed and quantitated using a standard enzyme-linked immunosorbent assay (ELISA). For primary single-point ADCC screening IgG variants are diluted to 40 ng/mL in RPMI media containing 1% FBS lacking phenol red. The final IgG concentration in the assay will be diluted by 4-fold (i.e. 10 ng/mL final concentration). Fifty microliter aliquots of IgG are added to the target cells and incubated for about 15 minutes at 37°C prior to adding the effector cells to the opsonized target cells.

When IgG titrations are performed, IgG concentration is varied in the range from about .0001 to 1 µg/mL. IgG dilutions are prepared using a 96-well microtiter plate by diluting the samples in RPMI containing 1% FBS lacking phenol red. The diluted IgG samples are then added to the assay plate containing the target cells.

### iv. Effector cells

The concentration of the PBMCs are adjusted so that the effector-to-target ratio is in the range of 10-20:1 (i.e. 2-4 X 10⁶ cells/mL). One hundred microliters of the resuspended PBMCs are added to each well of the opsonized target cells. The plates are incubated at 37°C in the presence of 5% CO₂ for 3-4 hours.

### v. Lactate-dehydrogenase (LDH)-release detection

Target cell lysis is measured by detecting the release of lactate dehydrogenase enzyme from the cytoplasm of damaged cells into the culture supernatant. Following incubation of the opsonized target cells with the effector cells, the assay plates are centrifuged at 2000 rpm for 5 minutes. About 75 µL of the cell culture supernatant is carefully removed while the pelleted cells and debris are avoided. This supernatant is added directly to a microtiter plate and to this is added 75 µL of LDH detection reagent (Roche Cat. No. 1 644 793). The plate is then incubated for approximately 15-30 minutes and the absorbance read at 490 nm using a Molecular Devices Vmax Kinetic Microplate Reader.

### vi. Data Analysis

All single-point ADCC screening assays are performed in duplicate. Each assay plate contains controls for spontaneous target lysis, spontaneous effector plus target lysis in the absence of IgG and target cell total lysis. Target cell total lysis is achieved by addition of 1% Triton X-100 to the target cells. Three wild type controls are included on each assay plate and the ADCC assay signal averaged. The background value, obtained from the spontaneous lysis controls, is subtracted from each sample. The background value, obtained from the diluted IgG, is subtracted from each sample. The data are converted from absorbance values to percentage of specific-lysis based upon the spontaneous and total lysis controls. The percentage of specific-lysis is calculated from the following equation: percentage specific lysis = (experimental A490 - background A490)/(maximal A490 - background A490) X 100, where background A490 is the sum of the A490 obtained from the effector and target cells in the absence of IgG and the IgG background due to contaminating LDH present in the crude IgG supernatants. The percentage of the Fc variant activity is normalized relative to the averaged wild type controls. The percentage of the normalized activity for duplicate assay plates are averaged and the standard deviation between individual assay plates calculated for each sample.

### vii. Results

The relative ADCC specific activity are shown in Table 2. The CDC values reported in this table were generated as described in Example 4 and the FcRn binding assay value reported in this table were generated as described in Example 5.

**TABLE 2**

| **Variant** | **ADCC** | **FcRn 6.0** | **FcRn 7.0** | **CDC** |
|---|---|---|---|---|
| Parental | 100 | 100 | 100 | 100 |
| P247H | 161 | 79 | 73 | 81 |
| P2471 | 163 | 89 | 74 | 42 |
| P247L | 166 | 92 | 78 | 49 |
| L251F | 123 | 102 | 100 | 138 |
| T256M | 126 | 72 | 82 | 167 |
| T256P | 115 | 132 | 121 | 315 |
| H268D | 127 | 87 | 84 | 137 |
| H268E | 129 | 90 | 81 | 108 |
| D280A | 144 | 91 | 84 | 141 |
| D280K | 140 | 103 | 93 | 144 |
| A330K | 129 | 104 | 99 | 43 |
| A330R | 123 | 77 | 101 | 31 |
| I332D | 145 | 91 | 102 | 146 |
| I332E | 161 | 99 | 97 | 150 |
| I332K | 12 | 109 | 97 | 67 |
| I332R | 12 | 97 | 92 | 88 |
| A339T | 130 | 115 | 109 | 189 |
| A378D | 101 | 122 | 93 | 28 |
| S440Y | 116 | 106 | 102 | 199 |

Figure 7A shows representative ADCC data obtained following titration of IgG variants. In this experiment, SKW6.4 cells were opsonized using both single and double amino acid Fc-region IgG variants followed by addition of PBMCs at a ratio of 15:1. The assay plate was incubated at 37°C in the presence of 5% CO₂ for 3 hours, followed by centrifugation. A portion of the supernatant was collected and transferred to a microtiter plate and the LDH activity in the supernatant detected by addition of an equal volume of LDH substrate. Following color development the absorbance was read at 490 nm. The background due to contaminating LDH in the crude IgG supernatants was measured and these background values were subtracted from the raw A490 yielding the data shown in Figure 7A. These data reveal that compared to the wild-type anti-CD20 antibody the ADCC activity of the A330K, A330R and I332E single amino acid variants are enhanced. The combination of either A330K or A330R with the I332E variant further improves the ADCC activity. A non-specific IgG shows no activity under these assay conditions. The results of a similar assay where glycosylation is altered is shown in Figure 7B. In particular, 7B (sample labeled GnTIII) shows that the activity of the 1332 variant can be further enhanced with altered glycosylation (by increasing bisecting GlcNAc content, for eg)..

Figure 8 shows representative ADCC data obtained using purified IgG. The plot shows percentage cytotoxicity versus IgG concentration for a non-specific IgG, wild type anti-CD20 and Fc-region variants I332D and I332E. The percentage cytotoxicity was calculated based upon the following equation: % cytotoxicity = (experimental A490-background A490)/(maximal A490 - background A490) X 100, where background A490 is obtained from the effector and target cells in the absence of IgG. The data show that the ADCC activity of the I332D and I332E variants are enhanced compared to the wild type. The target cells used in this experiment were SKW6.4 and an effector-to-target ratio of 15:1 was used in a standard 3 hour ADCC assay.

Figure 9 shows the same type of ADCC activity, except a lower affinity variable region is employed (AME 6F1 instead of AME 33). This figure demonstrates that ADCC activity with this particular anti-CD20 specificity and the Wil-2 and SKW6.4 cell lines is independent of variable region affinity.

Figure 10 shows Fc gamma RI receptor binding. The extracellular portion of the human Fc gamma RI receptor (CD64) with a C-terminal 6-his tag added in place of the putative transmembrane domain was expressed in HEK-293A cells and purified using a Ni-column. The receptor was coated overnight at 4°C on an ELISA plate and the plate was subsequently blocked using Superblock. After blocking and washing, IgG dilutions were prepared and added to the receptor-coated plate followed by incubation at 37°C for 2 hours. Unbound IgG was removed by washing and bound antibody detected using a 1:1000 of an anti-human Kappa alkaline phosphatase conjugated secondary antibody. Following washing, bound IgG was detected by addition of the phosphatase substrate (phenolphthalein monophosphate) (PPMP) (JBL Scientific) with absorbance determined colorimetrically at 560 nm with a VMAX microplate reader (Molecular Devices, Sunnyvale, CA). Data are plotted as log IgG concentration versus absorbance.

### Example 2

### Screening Variants for B Cell Depletion in Whole Blood Assay

This example describes screening variants for B cell depletion in a whole blood assay.

### i. FcγRILA Genotyping

Genomic DNA was isolated from peripheral blood samples using the QiaAmp kit (Qiagen, Cat No. 51104). FcγRIIA was amplified by PCR using the primers (5' *GGAAAATCCCAGAAATTCTCGC 3' -* SEQ ID NO:61) and (5' *CAACAGCCTGAC TACCTATTACGCGGG* 3' *-* SEQ ID NO:62). PCR products were purified using a MinElute PCR purification kit (Qiagen, Cat No. 28006) and digested with the restriction enzyme, BstUI, at 60°C for 12 hours. Digested samples were analyzed on 3% agarose gels. All PCR products cut at an internal BstUI site independent of the H or R genotype. Products that possess the R allele cut at an additional BstUI site. This results in the H/H genotype being identified by one fragment of 337bp, H/R being identified by two fragments of 337 bp and 316 bp and R/R identified by one fragment of 316bp.

### ii. FcγRIILA Genotyping

Genomic DNA was isolated from peripheral blood samples using the QiaAmp kit (Qiagen, Cat No. 51104). FcγRIIIA was amplified from genomic DNA by PCR using the primers (5' *ATATTTACAGAATGGCA* 3' - SEQ ID NO:63) and (5' *GGTGATGT TCACAGTCTCTGAAGACACATTTTTACTGTCAA 3*' - SEQ ID NO:64). PCR products were purified using a MinElute PCR purification kit (Qiagen, Cat No. 28006) and digested with the restriction enzyme, HincII, at 37°C for 16 hours. Digested samples were analyzed on 3% NuSieve agarose gels. HincII cuts the V allele but not the F allele. Therefore F/F gives one fragment of 148bp, F/V gives three fragments of 148, 109, and 39bp and V/V gives two fragments of 109 and 39bp.

### iii. B cell depletion assay (using whole blood)

Heparinized peripheral blood was drawn from healthy volunteers. Blood was mixed with variant, Rituxan or a non-specific IgG (negative control) diluted in FACS buffer (phosphate buffered saline +2% fetal bovine serum). Tubes were incubated for four hours at 37°C and then stained with fluorescein-anti-CD45 (to identify leucocytes) (BD, cat#555482) and phycoerythrin-anti-CD19 (to identify B cells) (BD, cat#555413) for 30 minutes at room temperature. Red cells were lysed by the addition of BD PharmLyse, (BD, cat#555899), samples were washed and resuspended in FACS buffer for analysis on a Becton Dickinson FACSort flow cytometer. Immediately before analysis, propidium iodide (PI), 0.1 µg/ml final concentration, was added to the samples to discriminate between live and dead cells. Controls included cells that had been incubated with FACS buffer alone, unstained and single-color stained samples. Each sample was set up in triplicate and 10,000 PI-negative events falling within the lymphocyte forward/side scatter (FSCSSC) gate were collected. Data was analyzed using WinMDI software. For analysis, live lymphocytes were identified on the basis of positive staining with CD45, PI negativity and FSC/SSC characteristics. In each sample the percentage of CD 19+ cells with these characteristics was identified. Data was expressed as mean % CD 19+ cells plus/minus 1 standard deviation. Alternatively, to facilitate comparison between donors, data was expressed as % of initial CD19+ cells remaining i.e. (%CD19+ lymphocytes remaining after treatment)/(%CD19+ lymphocytes in FACS buffer treated sample) x 100%.

Figure 12 shows representative B cell depletion data demonstrating that the I332E variant depeletes B cells more potently and to a greater extent than Rituxan in a whole blood sample containing the VV (high affinity) genotype of the FcγRIIIa receptor.

Figure 13 shows representative B cell depletion data demonstrating that the I332E variant depeletes B cells more potently and to a greater extent than Rituxan in a whole blood sample containing the FF (low affinity) genotype of the FcγRIIIa receptor. Similar results are obtained when the assay is performed with whole blood displaying the VF (heterozygous) genotype for the FcγRIIIa receptor or with HH, HR, or RR genotypes of the FcγRIIa receptor (Table 3).

**Table 3. Percent Remaining B Cells at Maximum Depletion**

| **Receptor** | **Genotype** | **I332E** | **RITUXAN** | **Non-specific IgG** | **n** |
|---|---|---|---|---|---|
| FcγRIIIa | VV | 40 | 60 | 98 | 5 |
| | VF | 51 | 70 | 94 | 6 |
| | FF | 52 | 73 | 95 | 6 |
| | | | | | |
| FcγRIIa | RR | 53 | 77 | 96 | 5 |
| | RH | 49 | 69 | 95 | 9 |
| | HH | 38 | 53 | 95 | 3 |

Figure 14 shows representative B cell depletion data demonstrating that variant A378D unexpectedly depeletes B cells more potently and to a greater extent than variants with equal or greater activity in in vitro ADCC and CDC assays.

### Example 3

### Affinity Determination of Variant

This examples describes how the the affinity (Kd) of the interaction between FcγRIIIa (FF genotype) and wild type, or parental, Fc and variant I332E was determined.

### i. FcγRIIIa/IgG Kd analysis

Human FcγRIIIa(158F) soluble extracellular domain was expressed in 293 cells and purified by Ni-NTA resin binding to its engineered 6-histidine tag.

One tube of Azlactone beads (Sapidyne) was coupled with 1mg of FcγRIII(158F) in sodium bicarbonate buffer pH 9.3. The slurry was tumbled overnight at 4°C, washed twice with PBS and blocked with blocking buffer (1M Tris pH 7.4 + 1% BSA) for 1 hour at room temperature. The beads were then washed three times with PBS and re-suspended in 50 mL of PBS with 0.01% azide. Twelve equilibrations all containing 7.5 nM IgG and FcγRIII titrated from 4 µM to 1.95 nM were prepared in running buffer (PBS pH 7.4, 0.1% BSA 0.01% azide) and allowed to equilibrate for 16 hours at room temperature. All experiments were run in running buffer at room temperature with flow rates at 0.5 ml/min. Following packing of the FcγRIII beads in the flow cell of the instrument, free IgG was captured from the first equilibration. The beads were then washed and bound IgG detected using a goat anti-human Fc (Fab')2-FITC conjugate (Jackson Immunochemicals). The process was then repeated for the remaining 11 equilibrations and the Kd determined following analysis with the Kinexa Pro software. Using this approach, the affinity (Kd) of the interaction between FcγRIIIa (FF genotype) and wild type, or parental, Fc was determined to be 387 nM while the affinity of variant I332E was 52 nM. Glycoengineered antibody (GnTIII) displayed a Kd of 83 nM while a combinatorial variant (L256P, I332E) displayed a Kd of 21 nM.

### Example 4

### Characterization of CDC Activity of Variants

This example descibes how the CDC activity of various Fc region variants was determined.

### i. Assay

This assay was carried out using human complement (Quidel Corp., cat#A113) on Ramos (RA #1) cell (ATCC Catalog No. CRL-1596). Ramos cells were cultured in Gibco RPMI1640 media containing10% FBS at 37°C and 5% CO2. The day before the assay, cells were seeded at 1 x 10⁶ cells in a T175 flask. The following day, the cells were resuspended to 3.57 x 10⁵ cells/ml in RPMI1640 without phenol red containing 1% FBS. Distribute 70 µl cells per well to a Costar 3917 flat bottom plate. For titration curve, variant IgG was prepared in a 3-fold serial dilution in RPMI1640 media. For single-point library screening assay, transiently expressed IgG variant in culture supernatant was normalized to 1 µg/ml in mock media. Thirty microliter of variant IgG (i.e. 200 ng/ml final concentration) and 50 µl of human complement (Quidel Corp., cat#A113) 1:5 diluted in RPMI1640 + 1% FBS was added to the target cell andmixed well by gentle pipetting. The plates were incubated at 37°C in the presence of 5% CO₂ for 1.5 hours. After addition of 15 µl/well of Alamar Blue (Serotec, cat#BUF012B) the incubation continued overnight. The following morning, the fluorescence signal was measured by PerkinElmer's EnVision 2100 multilabel reader with excitation at 560 nm and emission at 590 nm.

### ii. Data Analysis

All single-point assays were performed in duplicate. Each assay plate contained controls for spontaneous target cell lysis by human complement in the absence of IgG and target cell maximal lysis in the presence of 1% Triton X-100. Three wild type controls were included on each assay plate and the CDC assay signal averaged. The background value, obtained from the spontaneous lysis control, was subtracted from each sample. The data were converted from fluorescence signals to percentage of specific-lysis based upon spontaneous and maximal lysis controls. The percentage of specific-lysis was calculated from the following equation: percentage specific lysis = (experimental fluorescence signal - spontaneous signal)/(maximal lysis signal - spontaneous signal) X 100. The percentage of Fc variant activity over the average of three wild type controls was then calculated. The percentage activities over wild type for duplicate assay plates were averaged and the standard deviation between individual assay plates calculated.

Figure 11A, B, and C shows representative CDC data obtained following titration of IgG variants. In this experiment, Ramos cells were lysed using both single and double amino acid Fc-region IgG variants and human complement. The assay plate was incubated at 37°C in the presence of 5% CO₂ for 1.5 hours, followed by addition of 15 µl of Alamar blue and overnight incubation. The fluorescence signal was measured and plotted against the IgG concentrations. These data reveal that compared to the wild-type anti-CD20 antibody the CDC activities are slightly enhanced in cases of single amino acid variants I332D, I332E (Figure 11A), and significantly enhanced in cases of T256P and S440Y (Figure 11 B) and the combination of T256P with I332E (Figure 11C). In the case of variant A378D, the CDC activity is diminished (Figure 11C and Table 2).

### Example 5

### Characterization of Binding to FcRn

This Example describes assays for Fc neonatal receptor (FcRn) binding to variant IgG. A U-bottom 96-well ELISA plate was coated (Costar) with 50 µl/well of 2 µg/ml Neutravidin (Pierce Biotechnology, Cat#31000) in 50 mM Carbonate buffer (pH 9.3) at 4°C overnight. Unbound NeutraAvidin was removed and the plate was washed three times with PBST (PBS containing 0.1% Tween 20). 50 µl/well biotin-labeled soluble FcRn at 2.5 µg/ml in PBS was applied and incubated at room temperature for 1 hr. 75 µl of casein blocking buffer (Pierce Biotechnology, Cat#37528) was then added to the plate for 1 hour. The ELISA plate was then washed with PBST and incubated with 50 µl/well of variant IgG. For titration curve, variant IgG was prepared by a 3-fold serial dilution in FcRn-binding buffer (100 mM NaPO₄, 0.05% Tween-20 at different pH (from 6.0 to 7.4). For single-point screening, transient expressed variant IgG in culture supernatant was normalized to final concentration of 50 ng/ml (200 ng/ml for pH 7.4 binding) and pH adjusted with FcRn binding buffer to 6.0. In the following steps, FcRn-binding buffer at correspondingly pH was used to wash the plate and to dilute reagents. The binding reaction was carried out at room temperature for 1 hr. After three washes, bound IgG was detected by goat (Fab')2 anti-human-Fab-HRP conjugate for 1 hr. HRP activity was developed in Pierce's HRP substrate (Turbo TMB-ELISA, Cat#34022) for 5-30 minutes. Reaction was stopped by addition of 50 µl of 2M H₂SO₄ and the absorbance at 450nm was read with a VMAX microplate reader (Molecular Devices).

### EXAMPLE 6

### Anti-CD20-I332E Fc Variant Human Therapy

*In vitro* studies and murine tumor models (Clynes RA, et al. Nat Med. 6:443 (2000)) provide evidence that ADCC plays a role in the anti-tumor effects of anti-CD20 antibodies, such as RITUXAN. Human patients may be treated with anti-CD20-I332E Fc variant antibodies (Figs. 15 and 16), or RITUXAN, in a manner similar to that disclosed in Cartron *et al*., Blood 99:754 (2002), herein incorporated by reference. For example, patients presenting with stage II to IV disease according to the Ann-Arbor classification, having at least one measurable disease site, and low tumor burden according to the GELF criteria, could be treated with a total of four approximately 375 mg/m² doses of an anti-CD20-I332E Fc variant or with RITUXAN administered by intravenous infusion (days 1, 8, 15, and 22). The primary efficacy end point is the objective response rate, ie, the proportion of patients achieving either complete remission (CR), unconfirmed CR (Cru), or partial response (PR) according to criteria recently proposed by an international expert committee. Clinical response may be evaluated at month two (M2). Patients may also be evaluated for progression at 1 year (M12).

The objective response rates at M2 and M12 for patients treated with RITUXAN or anti-CD20-I332E can be compared such that the improved ADCC activities provided by I332E variants may be quantified. This same example could be repeated with other anti-CD20 variants (eg., I332D, P247L A330K, A339T, A378D, or combinatorial as shown in Table 1).

Additionally, the enhanced potency of the variants may permit different routes of administration, less frequent injections, and/or administration of smaller doses.

All publications and patents mentioned in the above specification are herein incorporated by reference. Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in chemistry, and molecular biology or related fields are intended to be within the scope of the following claims.

## Claims

1. An antibody comprising a variant of a parent human Fc region, or portion thereof, wherein the variant comprises at least one amino acid substitution compared to the parent Fc region, and wherein the amino acid substitution is at a position corresponding to a position of the human Fc sequence selected from the group consisting of: 378, 251, 256, 280, 330, 332, 339, and 440 according to EU index format as in Kabat et al Sequence of Proteins of Immunological Interest, 1991.

2. The antibody of claim 1, wherein the substitution is selected from the group consisting of 378D, 251F, 256P, 280K, 330K, 332K, 339T, and 440Y.

3. The antibody of any of claims 1-2, wherein the antibody comprising the variant of a parent Fc region at least one amino acid modification that increases neonatal Fc receptor (FcRn) binding affinity at pH 6.0, but not at pH 7.0, as compared to the antibody comprising the parent Fc region.

4. The antibody of claim 1-2, wherein the antibody comprising the variant of a parent Fc region displays relative FcRn binding affinity values greater than 100 when the antibody comprising the parent Fc region displays relative FcRn binding affinity values of 100 when measured with the same assay at pH 6.0.

5. The antibody of claim 1 or claim 2, wherein the antibody comprising the variant of a parent Fc region mediates antibody-dependent cell-mediated cytotoxicity (ADCC) in the presence of effector cells more effectively than the antibody comprising the parent Fc region.

6. The antibody of claim 5, wherein the antibody comprising the variant Fc region displays relative ADCC specific activity values greater than 100 when the antibody comprising the parent Fc region displays relative ADCC specific activity of 100 when measured with the same assay.

7. The antibody of claim 5, wherein the antibody comprising the variant Fc region displays relative ADCC specific activity values less than 100 when the antibody comprising the parent Fc region displays relative ADCC specific activity of 100 when measured with the same assay.

8. The antibody of claim 1 or claim 2, wherein the antibody comprising the variant of a parent Fc region mediates complement-dependent cytotoxicity (CDC) in the presence of effector cells more effectively than the antibody comprising the parent Fc region.

9. The antibody of claim 8, wherein the antibody comprising the variant of a parent Fc region displays relative CDC specific activity values greater than 100 when the antibody comprising the parent Fc region displays relative CDC specific activity of 100 when measured with the same assay.

10. The antibody of claim 8, wherein the antibody comprising the variant of a parent Fc region displays relative CDC specific activity values less than 100 when the antibody comprising the parent Fc region displays relative CDC specific activity of 100 when measured with the same assay

11. The antibody of any of claims 1-10, wherein the parent Fc region is a class of IgG.

12. The antibody of any of claims 1-10, wherein the parent Fc region is a subclass selected from the group consisting of IgG1, IgG2, IgG3 and IgG4.

13. The antibody of any of claims 1-12, wherein the antibody is a monoclonal antibody.

14. The monoclonal antibody of claim 13, wherein the monoclonal antibody comprises two identical heavy chain polypeptides and two identical light chain polypeptides.

15. A pharmaceutical composition comprising the antibody of any one of claims 1-14.

16. An antibody according to any one of claims 1-14 for use in therapy.


SEQUENCE LISTING

<110>  Applied Molecular Evolution

<120>  Fc Region Variants

<130>  X-16757

<150>  60/535,764
<151>  2004-01-12

<160>  56

<170>  PatentIn version 3.3

<210>  1
<211>  218
<212>  PRT
<213>  Human

<400>  1

Pro Ala Pro Glu Leu Leu Gly Gly Pro Ser Val Phe Leu Phe Pro Pro
1               5                   10                  15

Lys Pro Lys Asp Thr Leu Met Ile Ser Arg Thr Pro Glu Val Thr Cys
            20                  25                  30

Val Val Val Asp Val Ser His Glu Asp Pro Glu Val Lys Phe Asn Trp
        35                  40                  45

Tyr Val Asp Gly Val Glu Val His Asn Ala Lys Thr Lys Pro Arg Glu
    50                  55                  60

Glu Gln Tyr Asn Ser Thr Tyr Arg Val Val Ser Val Leu Thr Val Leu
65                  70                  75                  80

His Gln Asp Trp Leu Asn Gly Lys Glu Tyr Lys Cys Lys Val Ser Asn
                85                  90                  95

Lys Ala Leu Pro Ala Pro Ile Glu Lys Thr Ile Ser Lys Ala Lys Gly
            100                 105                 110

Gln Pro Arg Glu Pro Gln Val Tyr Thr Leu Pro Pro Ser Arg Glu Glu
        115                 120                 125

Met Thr Lys Asn Gln Val Ser Leu Thr Cys Leu Val Lys Gly Phe Tyr
    130                 135                 140

Pro Ser Asp Ile Ala Val Glu Trp Glu Ser Asn Gly Gln Pro Glu Asn
145                 150                 155                 160

Asn Tyr Lys Thr Thr Pro Pro Val Leu Asp Ser Asp Gly Ser Phe Phe
                165                 170                 175

Leu Tyr Ser Lys Leu Thr Val Asp Lys Ser Arg Trp Gln Gln Gly Asn

180                 185                 190

Val Phe Ser Cys Ser Val Met His Glu Ala Leu His Asn His Tyr Thr
        195                 200                 205

Gln Lys Ser Leu Ser Leu Ser Pro Gly Lys
    210                 215

<210>  2
<211>  217
<212>  PRT
<213>  Human

<400>  2

Pro Ala Pro Pro Val Ala Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Phe Arg Val Val Ser Val Leu Thr Val Val His
65                  70                  75                  80

Gln Asp Trp Leu Asn Gly Lys Glu Tyr Lys Cys Lys Val Ser Asn Lys
                85                  90                  95

Gly Leu Pro Ala Pro Ile Glu Lys Thr Ile Ser Lys Thr Lys Gly Gln
            100                 105                 110

Pro Arg Glu Pro Gln Val Tyr Thr Leu Pro Pro Ser Arg Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Met Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln

195                    200                    205

Lys Ser Leu Ser Leu Ser Pro Gly Lys
    210                 215

<210>  3
<211>  218
<212>  PRT
<213>  Human

<400>  3

Pro Ala Pro Glu Leu Leu Gly Gly Pro Ser Val Phe Leu Phe Pro Pro
1               5                   10                  15

Lys Pro Lys Asp Thr Leu Met Ile Ser Arg Thr Pro Glu Val Thr Cys
            20                  25                  30

Val Val Val Asp Val Ser His Glu Asp Pro Glu Val Gln Phe Lys Trp
        35                  40                  45

Tyr Val Asp Gly Val Glu Val His Asn Ala Lys Thr Lys Pro Arg Glu
    50                  55                  60

Glu Gln Phe Asn Ser Thr Phe Arg Val Val Ser Val Leu Thr Val Leu
65                  70                  75                  80

His Gln Asp Trp Leu Asn Gly Lys Glu Tyr Lys Cys Lys Val Ser Asn
                85                  90                  95

Lys Ala Leu Pro Ala Pro Ile Glu Lys Thr Ile Ser Lys Thr Lys Gly
            100                 105                 110

Gln Pro Arg Glu Pro Gln Val Tyr Thr Leu Pro Pro Ser Arg Glu Glu
        115                 120                 125

Met Thr Lys Asn Gln Val Ser Leu Thr Cys Leu Val Lys Gly Phe Tyr
    130                 135                 140

Pro Ser Asp Ile Ala Val Glu Trp Glu Ser Ser Gly Gln Pro Glu Asn
145                 150                 155                 160

Asn Tyr Asn Thr Thr Pro Pro Met Leu Asp Ser Asp Gly Ser Phe Phe
                165                 170                 175

Leu Tyr Ser Lys Leu Thr Val Asp Lys Ser Arg Trp Gln Gln Gly Asn
            180                 185                 190

Ile Phe Ser Cys Ser Val Met His Glu Ala Leu His Asn Arg Phe Thr
        195                 200                 205

Gln Lys Ser Leu Ser Leu Ser Pro Gly Lys

210                    215

<210>  4
<211>  218
<212>  PRT
<213>  Human

<400>  4

Pro Ala Pro Glu Phe Leu Gly Gly Pro Ser Val Phe Leu Phe Pro Pro
1               5                   10                  15

Lys Pro Lys Asp Thr Leu Met Ile Ser Arg Thr Pro Glu Val Thr Cys
            20                  25                  30

Val Val Val Asp Val Ser Gln Glu Asp Pro Glu Val Gln Phe Asn Trp
        35                  40                  45

Tyr Val Asp Gly Val Glu Val His Asn Ala Lys Thr Lys Pro Arg Glu
    50                  55                  60

Glu Gln Phe Asn Ser Thr Tyr Arg Val Val Ser Val Leu Thr Val Leu
65                  70                  75                  80

His Gln Asp Trp Leu Asn Gly Lys Glu Tyr Lys Cys Lys Val Ser Asn
                85                  90                  95

Lys Gly Leu Pro Ser Ser Ile Glu Lys Thr Ile Ser Lys Ala Lys Gly
            100                 105                 110

Gln Pro Arg Glu Pro Gln Val Tyr Thr Leu Pro Pro Ser Gln Glu Glu
        115                 120                 125

Met Thr Lys Asn Gln Val Ser Leu Thr Cys Leu Val Lys Gly Phe Tyr
    130                 135                 140

Pro Ser Asp Ile Ala Val Glu Trp Glx Ser Asn Gly Gln Pro Glu Asn
145                 150                 155                 160

Asn Tyr Lys Thr Thr Pro Pro Val Leu Asp Ser Asp Gly Ser Phe Phe
                165                 170                 175

Leu Tyr Ser Arg Leu Thr Val Asp Lys Ser Arg Trp Gln Glu Gly Asn
            180                 185                 190

Val Phe Ser Cys Ser Val Met His Glu Ala Leu His Asn His Tyr Thr
        195                 200                 205

Gln Lys Ser Leu Ser Leu Ser Leu Gly Lys
    210                 215

<210>  5

<211>  215
<212>  PRT
<213>  Murine

<400>  5

Thr Val Pro Glu Val Ser Ser Val Phe Ile Phe Pro Pro Lys Pro Lys
1               5                   10                  15

Asp Val Leu Thr Ile Thr Leu Thr Pro Lys Val Thr Cys Val Val Val
            20                  25                  30

Asp Ile Ser Lys Asp Asp Pro Glu Val Gln Phe Ser Trp Phe Val Asp
        35                  40                  45

Asp Val Glu Val His Thr Ala Gln Thr Gln Pro Arg Glu Glu Gln Phe
    50                  55                  60

Asn Ser Thr Phe Arg Ser Val Ser Glu Leu Pro Ile Met His Gln Asp
65                  70                  75                  80

Cys Leu Asn Gly Lys Glu Phe Lys Cys Arg Val Asn Ser Ala Ala Phe
                85                  90                  95

Pro Ala Pro Ile Glu Lys Thr Ile Ser Lys Thr Lys Gly Arg Pro Lys
            100                 105                 110

Ala Pro Gln Val Tyr Thr Ile Pro Pro Pro Lys Glu Gln Met Ala Lys
        115                 120                 125

Asp Lys Val Ser Leu Thr Cys Met Ile Thr Asp Phe Phe Pro Glu Asp
    130                 135                 140

Ile Thr Val Glu Trp Gln Trp Asn Gly Gln Pro Ala Glu Asn Tyr Lys
145                 150                 155                 160

Asn Thr Gln Pro Ile Met Asp Thr Asp Gly Ser Tyr Phe Val Tyr Ser
                165                 170                 175

Lys Leu Asn Val Gln Lys Ser Asn Trp Glu Ala Gly Asn Thr Phe Thr
            180                 185                 190

Cys Ser Val Leu His Glu Gly Leu His Asn His His Thr Glu Lys Ser
        195                 200                 205

Leu Ser His Ser Pro Gly Lys
    210                 215

<210>  6
<211>  218
<212>  PRT
<213>  Murine

<400> 6

Pro Ala Pro Asn Leu Leu Gly Gly Pro Ser Val Phe Ile Phe Pro Pro
1               5                   10                  15

Lys Ile Lys Asp Val Leu Met Ile Ser Leu Ser Pro Ile Val Thr Cys
            20                  25                  30

Val Val Val Asp Val Ser Glu Asp Asp Pro Asp Val Gln Ile Ser Trp
        35                  40                  45

Phe Val Asn Asn Val Glu Val His Thr Ala Gln Thr Gln Thr His Arg
    50                  55                  60

Glu Asp Tyr Asn Ser Thr Leu Arg Val Val Ser Ala Leu Pro Ile Gln
65                  70                  75                  80

His Gln Asp Trp Met Ser Gly Lys Glu Phe Lys Cys Lys Val Asn Asn
                85                  90                  95

Lys Asp Leu Pro Ala Pro Ile Glu Arg Thr Ile Ser Lys Pro Lys Gly
            100                 105                 110

Ser Val Arg Ala Pro Gln Val Tyr Val Leu Pro Pro Pro Glu Glu Glu
        115                 120                 125

Met Thr Lys Lys Gln Val Thr Leu Thr Cys Met Val Thr Asp Phe Met
    130                 135                 140

Pro Glu Asp Ile Tyr Val Glu Trp Thr Asn Asn Gly Lys Thr Glu Leu
145                 150                 155                 160

Asn Tyr Lys Asn Thr Glu Pro Val Leu Asp Ser Asp Gly Ser Tyr Phe
                165                 170                 175

Met Tyr Ser Lys Leu Arg Val Glu Lys Lys Asn Trp Val Glu Arg Asn
            180                 185                 190

Ser Tyr Ser Cys Ser Val Val His Glu Gly Leu His Asn His His Thr
        195                 200                 205

Thr Lys Ser Phe Ser Arg Thr Pro Gly Lys
    210                 215

<210>  7
<211>  218
<212>  PRT
<213>  Murine

<400>  7

Pro Ala Pro Asn Leu Glu Gly Gly Pro Ser Val Phe Ile Phe Pro Pro
1               5                   10                  15

Asn Ile Lys Asp Val Leu Met Ile Ser Leu Thr Pro Lys Val Thr Cys
            20                  25                  30

Val Val Val Asp Val Ser Glu Asp Asp Pro Asp Val Gln Ile Ser Trp
        35                  40                  45

Phe Val Asn Asn Val Glu Val His Thr Ala Gln Thr Gln Thr His Arg
    50                  55                  60

Glu Asp Tyr Asn Ser Thr Ile Arg Val Val Ser His Leu Pro Ile Gln
65                  70                  75                  80

His Gln Asp Trp Met Ser Gly Lys Glu Phe Lys Cys Lys Val Asn Asn
                85                  90                  95

Lys Asp Leu Pro Ser Pro Ile Glu Arg Thr Ile Ser Lys Pro Lys Gly
            100                 105                 110

Leu Val Arg Ala Pro Gln Val Tyr Thr Leu Pro Pro Pro Ala Glu Gln
        115                 120                 125

Leu Ser Arg Lys Asp Val Ser Leu Thr Cys Leu Val Val Gly Phe Asn
    130                 135                 140

Pro Gly Asp Ile Ser Val Glu Trp Thr Ser Asn Gly His Thr Glu Glu
145                 150                 155                 160

Asn Tyr Lys Asp Thr Ala Pro Val Leu Asp Ser Asp Gly Ser Tyr Phe
                165                 170                 175

Ile Tyr Ser Lys Leu Asn Met Lys Thr Ser Lys Trp Glu Lys Thr Asp
            180                 185                 190

Ser Phe Ser Cys Asn Val Arg His Glu Gly Leu Lys Asn Tyr Tyr Leu
        195                 200                 205

Lys Lys Thr Ile Ser Arg Ser Pro Gly Lys
    210                 215

<210>  8
<211>  218
<212>  PRT
<213>  Murine

<400>  8

Pro Pro Gly Asn Ile Leu Gly Gly Pro Ser Val Phe Ile Phe Pro Pro
1               5                   10                  15

Lys Pro Lys Asp Ala Leu Met Ile Ser Leu Thr Pro Lys Val Thr Cys
            20                  25                  30

Val Val Val Asp Val Ser Glu Asp Asp Pro Asp Val His Val Ser Trp
        35                  40                  45

Phe Val Asp Asn Lys Glu Val His Thr Ala Trp Thr Gln Pro Arg Glu
    50                  55                  60

Ala Gln Tyr Asn Ser Thr Phe Arg Val Val Ser Ala Leu Pro Ile Gln
65                  70                  75                  80

His Gln Asp Trp Met Arg Gly Lys Glu Phe Lys Cys Lys Val Asn Asn
                85                  90                  95

Lys Ala Leu Pro Ala Pro Ile Glu Arg Thr Ile Ser Lys Pro Lys Gly
            100                 105                 110

Arg Ala Gln Thr Pro Gln Val Tyr Thr Ile Pro Pro Pro Arg Glu Gln
        115                 120                 125

Met Ser Lys Lys Lys Val Ser Leu Thr Cys Leu Val Thr Asn Phe Phe
    130                 135                 140

Ser Glu Ala Ile Ser Val Glu Trp Glu Arg Asn Gly Glu Leu Glu Gln
145                 150                 155                 160

Asp Tyr Lys Asn Thr Pro Pro Ile Leu Asp Ser Asp Gly Thr Tyr Phe
                165                 170                 175

Leu Tyr Ser Lys Leu Thr Val Asp Thr Asp Ser Trp Leu Gln Gly Glu
            180                 185                 190

Ile Phe Thr Cys Ser Val Val His Glu Ala Leu His Asn His His Thr
        195                 200                 205

Gln Lys Asn Leu Ser Arg Ser Pro Gly Lys
    210                 215

<210>  9
<211>  110
<212>  PRT
<213>  Human

<400>  9

Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys
            100                 105                 110

<210>  10
<211>  107
<212>  PRT
<213>  Human

<400>  10

Gly Gln Pro Arg Glu Pro Gln Val Tyr Thr Leu Pro Pro Ser Arg Glu
1               5                   10                  15

Glu Met Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly Lys
            100                 105

<210>  11
<211>  330
<212>  PRT
<213>  Human

<400>  11

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  12
<211>  330
<212>  PRT
<213>  Human

<400>  12

<210>  13
<211>  31
<212>  PRT
<213>  Human

<400>  13

Ala Pro Glu Leu Leu Gly Gly Pro Ser Val Phe Leu Phe Pro Pro Lys
1               5                   10                  15

His Lys Asp Thr Leu Met Ile Ser Arg Thr Pro Glu Val Thr Cys
            20                  25                  30

<210>  14
<211>  31
<212>  PRT
<213>  Human

<400>  14

Ala Pro Glu Leu Leu Gly Gly Pro Ser Val Phe Leu Phe Pro Pro Lys
1               5                   10                  15

Ile Lys Asp Thr Leu Met Ile Ser Arg Thr Pro Glu Val Thr Cys
            20                  25                  30

<210>  15
<211>  31
<212>  PRT

<213>   Human

<400>   15

Ala Pro Glu Leu Leu Gly Gly Pro Ser Val Phe Leu Phe Pro Pro Lys
1               5                   10                  15

Leu Lys Asp Thr Leu Met Ile Ser Arg Thr Pro Glu Val Thr Cys
            20                  25                  30

<210>   16
<211>   31
<212>   PRT
<213>   Human

<400>   16

Ala Pro Glu Leu Leu Gly Gly Pro Ser Val Phe Leu Phe Pro Pro Lys
1               5                   10                  15

Pro Lys Asp Thr Phe Met Ile Ser Arg Thr Pro Glu Val Thr Cys
            20                  25                  30

<210>   17
<211>   31
<212>   PRT
<213>   Human

<400>   17

Ala Pro Glu Leu Leu Gly Gly Pro Ser Val Phe Leu Phe Pro Pro Lys
1               5                   10                  15

Pro Lys Asp Thr Leu Met Ile Ser Arg Met Pro Glu Val Thr Cys
            20                  25                  30

<210>   18
<211>   31
<212>   PRT
<213>   Human

<400>   18

Ala Pro Glu Leu Leu Gly Gly Pro Ser Val Phe Leu Phe Pro Pro Lys
1               5                   10                  15

Pro Lys Asp Thr Leu Met Ile Ser Arg Pro Pro Glu Val Thr Cys
            20                  25                  30

<210>   19
<211>   29
<212>   PRT
<213>   Human

<400>   19

Val Val Val Asp Val Ser Asp Glu Asp Pro Glu Val Lys Phe Asn Trp
1               5                   10                  15

Tyr Val Asp Gly Val Glu Val His Asn Ala Lys Thr Lys
            20                  25

<210> 20
<211> 29
<212> PRT
<213> Human

<400> 20

Val Val Val Asp Val Ser Glu Glu Asp Pro Glu Val Lys Phe Asn Trp
1               5                   10                  15

Tyr Val Asp Gly Val Glu Val His Asn Ala Lys Thr Lys
            20                  25

<210> 21
<211> 29
<212> PRT
<213> Human

<400> 21

Val Val Val Asp Val Ser His Glu Asp Pro Glu Val Lys Phe Asn Trp
1               5                   10                  15

Tyr Val Ala Gly Val Glu Val His Asn Ala Lys Thr Lys
            20                  25

<210> 22
<211> 29
<212> PRT
<213> Human

<400> 22

Val Val Val Asp Val Ser His Glu Asp Pro Glu Val Lys Phe Asn Trp
1               5                   10                  15

Tyr Val Lys Gly Val Glu Val His Asn Ala Lys Thr Lys
            20                  25

<210> 23
<211> 30
<212> PRT
<213> Human

<400> 23

Cys Lys Val Ser Asn Lys Ala Leu Pro Lys Pro Ile Glu Lys Thr Ile
1               5                   10                  15

Ser Lys Ala Lys Gly Gln Pro Arg Glu Pro Gln Val Tyr Thr
            20                  25                  30

<210> 24
<211> 30

<212>   PRT
<213>   Human

<400>   24

Cys Lys Val Ser Asn Lys Ala Leu Pro Arg Pro Ile Glu Lys Thr Ile
1               5                   10                  15

Ser Lys Ala Lys Gly Gln Pro Arg Glu Pro Gln Val Tyr Thr
            20                  25                  30

<210>   25
<211>   30
<212>   PRT
<213>   Human

<400>   25

Cys Lys Val Ser Asn Lys Ala Leu Pro Ala Pro Asp Glu Lys Thr Ile
1               5                   10                  15

Ser Lys Ala Lys Gly Gln Pro Arg Glu Pro Gln Val Tyr Thr
            20                  25                  30

<210>   26
<211>   30
<212>   PRT
<213>   Human

<400>   26

Cys Lys Val Ser Asn Lys Ala Leu Pro Ala Pro Glu Glu Lys Thr Ile
1               5                   10                  15

Ser Lys Ala Lys Gly Gln Pro Arg Glu Pro Gln Val Tyr Thr
            20                  25                  30

<210>   27
<211>   30
<212>   PRT
<213>   Human

<400>   27

Cys Lys Val Ser Asn Lys Ala Leu Pro Ala Pro Lys Glu Lys Thr Ile
1               5                   10                  15

Ser Lys Ala Lys Gly Gln Pro Arg Glu Pro Gln Val Tyr Thr
            20                  25                  30

<210>   28
<211>   30
<212>   PRT
<213>   Human

<400>   28

Cys Lys Val Ser Asn Lys Ala Leu Pro Ala Pro Arg Glu Lys Thr Ile
1               5                   10                  15

Ser Lys Ala Lys Gly Gln Pro Arg Glu Pro Gln Val Tyr Thr
            20                  25                  30

<210>  29
<211>  30
<212>  PRT
<213>  Human

<400>  29

Cys Lys Val Ser Asn Lys Ala Leu Pro Ala Pro Ile Glu Lys Thr Ile
1               5                   10                  15

Ser Lys Thr Lys Gly Gln Pro Arg Glu Pro Gln Val Tyr Thr
            20                  25                  30

<210>  30
<211>  22
<212>  PRT
<213>  Human

<400>  30

Cys Leu Val Lys Gly Phe Tyr Pro Ser Asp Ile Asp Val Glu Trp Glu
1               5                   10                  15

Ser Asn Gly Gln Pro Glu
            20

<210>  31
<211>  23
<212>  PRT
<213>  Human

<400>  31

Cys Ser Val Met His Glu Ala Leu His Asn His Tyr Thr Gln Lys Tyr
1               5                   10                  15

Leu Ser Leu Ser Pro Gly Lys
            20

<210>  32
<211>  990
<212>  DNA
<213>  HUMAN

<400>  32
gcctccacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa ggttgagccc     300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggacgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctat agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 33
<211> 93
<212> DNA
<213> HUMAN

<400> 33
gcacctgaac tcctgggggg accgtcagtc ttcctcttcc ccccaaaacc caaggacacc 60

ctcatgatct cccggacccc tgaggtcaca tgc 93

<210> 34
<211> 87
<212> DNA
<213> HUMAN

<400> 34
gtggtggtgg acgtgagcca cgaagaccct gaggtcaagt tcaactggta cgtggacggc 60

gtggaggtgc ataatgccaa gacaaag 87

<210> 35
<211> 90
<212> DNA
<213> HUMAN

<400> 35
tgcaaggtct ccaacaaagc cctcccagcc cccatcgaga aaaccatctc caaagccaaa 60

gggcagcccc gagaaccaca ggtgtacacc 90

<210> 36
<211> 69
<212> DNA
<213> HUMAN

<400> 36
tgctccgtga tgcatgaggc tctgcacaac cactacacgc agaagagcct ctccctgtct 60

ccgggtaaa 69

<210> 37
<211> 66
<212> DNA
<213> HUMAN

<400> 37
tgcctggtca aaggcttcta tcccagcgac atcgccgtgg agtgggagag caatgggcag 60

ccggag 66

<210> 38
<211> 93
<212> DNA
<213> HUMAN

<400> 38
gcacctgaac tcctgggggg accgtcagtc ttcctcttcc ccccaaaaca caaggacacc 60

ctcatgatct cccggacccc tgaggtcaca tgc 93

<210> 39
<211> 93
<212> DNA
<213> HUMAN

<400> 39
gcacctgaac tcctgggggg accgtcagtc ttcctcttcc ccccaaaaat caaggacacc 60

ctcatgatct cccggacccc tgaggtcaca tgc 93

<210> 40
<211> 93
<212> DNA
<213> HUMAN

<400> 40
gcacctgaac tcctgggggg accgtcagtc ttcctcttcc ccccaaaact gaaggacacc 60

ctcatgatct cccggacccc tgaggtcaca tgc 93

<210> 41
<211> 93
<212> DNA
<213> HUMAN

<400> 41
gcacctgaac tcctgggggg accgtcagtc ttcctcttcc ccccaaaacc caaggacacc 60

ttcatgatct cccggacccc tgaggtcaca tgc 93

<210> 42
<211> 93
<212> DNA
<213> HUMAN

<400> 42
gcacctgaac tcctgggggg accgtcagtc ttcctcttcc ccccaaaacc caaggacacc 60

ctcatgatct cccggatgcc tgaggtcaca tgc 93

<210> 43
<211> 93
<212> DNA
<213> HUMAN

<400> 43
gcacctgaac tcctgggggg accgtcagtc ttcctcttcc ccccaaaacc caaggacacc 60

ctcatgatct cccggaccac tgaggtcaca tgc 93

<210> 44
<211> 87
<212> DNA
<213> HUMAN

<400> 44
gtggtggtgg acgtgagcga cgaagaccct gaggtcaagt tcaactggta cgtggacggc 60

gtggaggtgc ataatgccaa gacaaag 87

<210> 45
<211> 87
<212> DNA
<213> HUMAN

<400> 45
gtggtggtgg acgtgagcga ggaagaccct gaggtcaagt tcaactggta cgtggacggc 60

gtggaggtgc ataatgccaa gacaaag 87

<210> 46
<211> 87
<212> DNA
<213> HUMAN

<400> 46
gtggtggtgg acgtgagcca cgaagaccct gaggtcaagt tcaactggta cgtggccggc 60

gtggaggtgc ataatgccaa gacaaag 87

<210> 47
<211> 87
<212> DNA
<213> HUMAN

<400> 47
gtggtggtgg acgtgagcca cgaagaccct gaggtcaagt tcaactggta cgtgaaaggc 60

gtggaggtgc ataatgccaa gacaaag 87

<210> 48
<211> 90
<212> DNA
<213> HUMAN

<400> 48
tgcaaggtct ccaacaaagc cctcccaaaa cccatcgaga aaaccatctc caaagccaaa 60

gggcagcccc gagaaccaca ggtgtacacc 90

<210>  49
<211>  90
<212>  DNA
<213>  HUMAN

<400>  49
tgcaaggtct ccaacaaagc cctcccacgc cccatcgaga aaaccatctc caaagccaaa      60

gggcagcccc gagaaccaca ggtgtacacc                                      90


<210>  50
<211>  90
<212>  DNA
<213>  HUMAN

<400>  50
tgcaaggtct ccaacaaagc cctcccagcc cccgacgaga aaaccatctc caaagccaaa      60

gggcagcccc gagaaccaca ggtgtacacc                                      90


<210>  51
<211>  90
<212>  DNA
<213>  HUMAN

<400>  51
tgcaaggtct ccaacaaagc cctcccagcc cccgaggaga aaaccatctc caaagccaaa      60

gggcagcccc gagaaccaca ggtgtacacc                                      90


<210>  52
<211>  90
<212>  DNA
<213>  HUMAN

<400>  52
tgcaaggtct ccaacaaagc cctcccagcc cccaaagaga aaaccatctc caaagccaaa      60

gggcagcccc gagaaccaca ggtgtacacc                                      90


<210>  53
<211>  90
<212>  DNA
<213>  HUMAN

<400>  53
tgcaaggtct ccaacaaagc cctcccagcc ccccgcgaga aaaccatctc caaagccaaa      60

gggcagcccc gagaaccaca ggtgtacacc                                      90


<210>  54
<211>  90
<212>  DNA
<213>  HUMAN

<400>  54
tgcaaggtct ccaacaaagc cctcccagcc cccatcgaga aaaccatctc caaaaccaaa      60

gggcagcccc gagaaccaca ggtgtacacc                                      90


<210>  55

<211>  66
<212>  DNA
<213>  HUMAN

<400>  55
tgcctggtca aaggcttcta tcccagcgac atcgacgtgg agtgggagag caatgggcag     60

ccggag                                                                66


<210>  56
<211>  69
<212>  DNA
<213>  HUMAN

<400>  56
tgctccgtga tgcatgaggc tctgcacaac cactacacgc agaagtacct ctccctgtct     60

ccgggtaaa                                                             69

SEQUENCE LISTING

<110>  Applied Molecular Evolution

<120>  Fc Region Variants

<130>  X-16757

<150>  60/535,764
<151>  2004-01-12

<160>  56

<170>  PatentIn version 3.3

<210>  1
<211>  218
<212>  PRT
<213>  Human

<400>  1

Pro Ala Pro Glu Leu Leu Gly Gly Pro Ser Val Phe Leu Phe Pro Pro
1               5                   10                  15

Lys Pro Lys Asp Thr Leu Met Ile Ser Arg Thr Pro Glu Val Thr Cys
            20                  25                  30

Val Val Val Asp Val Ser His Glu Asp Pro Glu Val Lys Phe Asn Trp
        35                  40                  45

Tyr Val Asp Gly Val Glu Val His Asn Ala Lys Thr Lys Pro Arg Glu
    50                  55                  60

Glu Gln Tyr Asn Ser Thr Tyr Arg Val Val Ser Val Leu Thr Val Leu
65                  70                  75                  80

His Gln Asp Trp Leu Asn Gly Lys Glu Tyr Lys Cys Lys Val Ser Asn
                85                  90                  95

Lys Ala Leu Pro Ala Pro Ile Glu Lys Thr Ile Ser Lys Ala Lys Gly
            100                 105                 110

Gln Pro Arg Glu Pro Gln Val Tyr Thr Leu Pro Pro Ser Arg Glu Glu
        115                 120                 125

Met Thr Lys Asn Gln Val Ser Leu Thr Cys Leu Val Lys Gly Phe Tyr
    130                 135                 140

Pro Ser Asp Ile Ala Val Glu Trp Glu Ser Asn Gly Gln Pro Glu Asn
145                 150                 155                 160

Asn Tyr Lys Thr Thr Pro Pro Val Leu Asp Ser Asp Gly Ser Phe Phe
                165                 170                 175

Leu Tyr Ser Lys Leu Thr Val Asp Lys Ser Arg Trp Gln Gln Gly Asn
            180                 185                 190

Val Phe Ser Cys Ser Val Met His Glu Ala Leu His Asn His Tyr Thr
        195                 200                 205

Gln Lys Ser Leu Ser Leu Ser Pro Gly Lys
    210                 215

<210>  2
<211>  217
<212>  PRT
<213>  Human

<400>  2

Pro Ala Pro Pro Val Ala Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Phe Arg Val Val Ser Val Leu Thr Val Val His
65                  70                  75                  80

Gln Asp Trp Leu Asn Gly Lys Glu Tyr Lys Cys Lys Val Ser Asn Lys
                85                  90                  95

Gly Leu Pro Ala Pro Ile Glu Lys Thr Ile Ser Lys Thr Lys Gly Gln
            100                 105                 110

Pro Arg Glu Pro Gln Val Tyr Thr Leu Pro Pro Ser Arg Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn

145                 150                 155                 160

Tyr Lys Thr Thr Pro Pro Met Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys
    210                 215

<210>  3
<211>  218
<212>  PRT
<213>  Human

<400>  3

Pro Ala Pro Glu Leu Leu Gly Gly Pro Ser Val Phe Leu Phe Pro Pro
1               5                   10                  15

Lys Pro Lys Asp Thr Leu Met Ile Ser Arg Thr Pro Glu Val Thr Cys
            20                  25                  30

Val Val Val Asp Val Ser His Glu Asp Pro Glu Val Gln Phe Lys Trp
        35                  40                  45

Tyr Val Asp Gly Val Glu Val His Asn Ala Lys Thr Lys Pro Arg Glu
    50                  55                  60

Glu Gln Phe Asn Ser Thr Phe Arg Val Val Ser Val Leu Thr Val Leu
65                  70                  75                  80

His Gln Asp Trp Leu Asn Gly Lys Glu Tyr Lys Cys Lys Val Ser Asn
                85                  90                  95

Lys Ala Leu Pro Ala Pro Ile Glu Lys Thr Ile Ser Lys Thr Lys Gly
            100                 105                 110

Gln Pro Arg Glu Pro Gln Val Tyr Thr Leu Pro Pro Ser Arg Glu Glu
        115                 120                 125

Met Thr Lys Asn Gln Val Ser Leu Thr Cys Leu Val Lys Gly Phe Tyr
    130                 135                 140

Pro Ser Asp Ile Ala Val Glu Trp Glu Ser Ser Gly Gln Pro Glu Asn
145                 150                 155                 160

Asn Tyr Asn Thr Thr Pro Pro Met Leu Asp Ser Asp Gly Ser Phe Phe
                165                 170                 175

Leu Tyr Ser Lys Leu Thr Val Asp Lys Ser Arg Trp Gln Gln Gly Asn
            180                 185                 190

Ile Phe Ser Cys Ser Val Met His Glu Ala Leu His Asn Arg Phe Thr
        195                 200                 205

Gln Lys Ser Leu Ser Leu Ser Pro Gly Lys
    210                 215

<210>  4
<211>  218
<212>  PRT
<213>  Human

<400>  4

Pro Ala Pro Glu Phe Leu Gly Gly Pro Ser Val Phe Leu Phe Pro Pro
1               5                   10                  15

Lys Pro Lys Asp Thr Leu Met Ile Ser Arg Thr Pro Glu Val Thr Cys
            20                  25                  30

Val Val Val Asp Val Ser Gln Glu Asp Pro Glu Val Gln Phe Asn Trp
        35                  40                  45

Tyr Val Asp Gly Val Glu Val His Asn Ala Lys Thr Lys Pro Arg Glu
    50                  55                  60

Glu Gln Phe Asn Ser Thr Tyr Arg Val Val Ser Val Leu Thr Val Leu
65                  70                  75                  80

His Gln Asp Trp Leu Asn Gly Lys Glu Tyr Lys Cys Lys Val Ser Asn
                85                  90                  95

Lys Gly Leu Pro Ser Ser Ile Glu Lys Thr Ile Ser Lys Ala Lys Gly
            100                 105                 110

Gln Pro Arg Glu Pro Gln Val Tyr Thr Leu Pro Pro Ser Gln Glu Glu
        115                 120                 125

Met Thr Lys Asn Gln Val Ser Leu Thr Cys Leu Val Lys Gly Phe Tyr

130                 135                 140

Pro Ser Asp Ile Ala Val Glu Trp Glx Ser Asn Gly Gln Pro Glu Asn
145                 150                 155                 160

Asn Tyr Lys Thr Thr Pro Pro Val Leu Asp Ser Asp Gly Ser Phe Phe
                165                 170                 175

Leu Tyr Ser Arg Leu Thr Val Asp Lys Ser Arg Trp Gln Glu Gly Asn
            180                 185                 190

Val Phe Ser Cys Ser Val Met His Glu Ala Leu His Asn His Tyr Thr
        195                 200                 205

Gln Lys Ser Leu Ser Leu Ser Leu Gly Lys
    210                 215

<210>  5
<211>  215
<212>  PRT
<213>  Murine

<400>  5

Thr Val Pro Glu Val Ser Ser Val Phe Ile Phe Pro Pro Lys Pro Lys
1               5                   10                  15

Asp Val Leu Thr Ile Thr Leu Thr Pro Lys Val Thr Cys Val Val Val
            20                  25                  30

Asp Ile Ser Lys Asp Asp Pro Glu Val Gln Phe Ser Trp Phe Val Asp
        35                  40                  45

Asp Val Glu Val His Thr Ala Gln Thr Gln Pro Arg Glu Glu Gln Phe
    50                  55                  60

Asn Ser Thr Phe Arg Ser Val Ser Glu Leu Pro Ile Met His Gln Asp
65                  70                  75                  80

Cys Leu Asn Gly Lys Glu Phe Lys Cys Arg Val Asn Ser Ala Ala Phe
                85                  90                  95

Pro Ala Pro Ile Glu Lys Thr Ile Ser Lys Thr Lys Gly Arg Pro Lys
            100                 105                 110

Ala Pro Gln Val Tyr Thr Ile Pro Pro Pro Lys Glu Gln Met Ala Lys
        115                 120                 125

Asp Lys Val Ser Leu Thr Cys Met Ile Thr Asp Phe Phe Pro Glu Asp
    130                 135                 140

Ile Thr Val Glu Trp Gln Trp Asn Gly Gln Pro Ala Glu Asn Tyr Lys
145                 150                 155                 160

Asn Thr Gln Pro Ile Met Asp Thr Asp Gly Ser Tyr Phe Val Tyr Ser
                165                 170                 175

Lys Leu Asn Val Gln Lys Ser Asn Trp Glu Ala Gly Asn Thr Phe Thr
            180                 185                 190

Cys Ser Val Leu His Glu Gly Leu His Asn His His Thr Glu Lys Ser
        195                 200                 205

Leu Ser His Ser Pro Gly Lys
    210                 215

<210>  6
<211>  218
<212>  PRT
<213>  Murine

<400>  6

Pro Ala Pro Asn Leu Leu Gly Gly Pro Ser Val Phe Ile Phe Pro Pro
1               5                   10                  15

Lys Ile Lys Asp Val Leu Met Ile Ser Leu Ser Pro Ile Val Thr Cys
            20                  25                  30

Val Val Val Asp Val Ser Glu Asp Asp Pro Asp Val Gln Ile Ser Trp
        35                  40                  45

Phe Val Asn Asn Val Glu Val His Thr Ala Gln Thr Gln Thr His Arg
    50                  55                  60

Glu Asp Tyr Asn Ser Thr Leu Arg Val Val Ser Ala Leu Pro Ile Gln
65                  70                  75                  80

His Gln Asp Trp Met Ser Gly Lys Glu Phe Lys Cys Lys Val Asn Asn
                85                  90                  95

Lys Asp Leu Pro Ala Pro Ile Glu Arg Thr Ile Ser Lys Pro Lys Gly
            100                 105                 110

Ser Val Arg Ala Pro Gln Val Tyr Val Leu Pro Pro Pro Glu Glu Glu

115                 120                 125

Met Thr Lys Lys Gln Val Thr Leu Thr Cys Met Val Thr Asp Phe Met
    130                 135                 140

Pro Glu Asp Ile Tyr Val Glu Trp Thr Asn Asn Gly Lys Thr Glu Leu
145                 150                 155                 160

Asn Tyr Lys Asn Thr Glu Pro Val Leu Asp Ser Asp Gly Ser Tyr Phe
                165                 170                 175

Met Tyr Ser Lys Leu Arg Val Glu Lys Lys Asn Trp Val Glu Arg Asn
            180                 185                 190

Ser Tyr Ser Cys Ser Val Val His Glu Gly Leu His Asn His His Thr
        195                 200                 205

Thr Lys Ser Phe Ser Arg Thr Pro Gly Lys
    210                 215

<210>  7
<211>  218
<212>  PRT
<213>  Murine

<400>  7

Pro Ala Pro Asn Leu Glu Gly Gly Pro Ser Val Phe Ile Phe Pro Pro
1               5                   10                  15

Asn Ile Lys Asp Val Leu Met Ile Ser Leu Thr Pro Lys Val Thr Cys
            20                  25                  30

Val Val Val Asp Val Ser Glu Asp Asp Pro Asp Val Gln Ile Ser Trp
        35                  40                  45

Phe Val Asn Asn Val Glu Val His Thr Ala Gln Thr Gln Thr His Arg
    50                  55                  60

Glu Asp Tyr Asn Ser Thr Ile Arg Val Val Ser His Leu Pro Ile Gln
65                  70                  75                  80

His Gln Asp Trp Met Ser Gly Lys Glu Phe Lys Cys Lys Val Asn Asn
                85                  90                  95

Lys Asp Leu Pro Ser Pro Ile Glu Arg Thr Ile Ser Lys Pro Lys Gly
            100                 105                 110

Leu Val Arg Ala Pro Gln Val Tyr Thr Leu Pro Pro Pro Ala Glu Gln
        115                 120                 125

Leu Ser Arg Lys Asp Val Ser Leu Thr Cys Leu Val Val Gly Phe Asn
    130                 135                 140

Pro Gly Asp Ile Ser Val Glu Trp Thr Ser Asn Gly His Thr Glu Glu
145                 150                 155                 160

Asn Tyr Lys Asp Thr Ala Pro Val Leu Asp Ser Asp Gly Ser Tyr Phe
                165                 170                 175

Ile Tyr Ser Lys Leu Asn Met Lys Thr Ser Lys Trp Glu Lys Thr Asp
            180                 185                 190

Ser Phe Ser Cys Asn Val Arg His Glu Gly Leu Lys Asn Tyr Tyr Leu
        195                 200                 205

Lys Lys Thr Ile Ser Arg Ser Pro Gly Lys
    210                 215

<210>  8
<211>  218
<212>  PRT
<213>  Murine

<400>  8

Pro Pro Gly Asn Ile Leu Gly Gly Pro Ser Val Phe Ile Phe Pro Pro
1               5                   10                  15

Lys Pro Lys Asp Ala Leu Met Ile Ser Leu Thr Pro Lys Val Thr Cys
            20                  25                  30

Val Val Val Asp Val Ser Glu Asp Asp Pro Asp Val His Val Ser Trp
        35                  40                  45

Phe Val Asp Asn Lys Glu Val His Thr Ala Trp Thr Gln Pro Arg Glu
    50                  55                  60

Ala Gln Tyr Asn Ser Thr Phe Arg Val Val Ser Ala Leu Pro Ile Gln
65                  70                  75                  80

His Gln Asp Trp Met Arg Gly Lys Glu Phe Lys Cys Lys Val Asn Asn
                85                  90                  95

Lys Ala Leu Pro Ala Pro Ile Glu Arg Thr Ile Ser Lys Pro Lys Gly

            100                 105                 110

Arg Ala Gln Thr Pro Gln Val Tyr Thr Ile Pro Pro Pro Arg Glu Gln
        115                 120                 125

Met Ser Lys Lys Lys Val Ser Leu Thr Cys Leu Val Thr Asn Phe Phe
    130                 135                 140

Ser Glu Ala Ile Ser Val Glu Trp Glu Arg Asn Gly Glu Leu Glu Gln
145                 150                 155                 160

Asp Tyr Lys Asn Thr Pro Pro Ile Leu Asp Ser Asp Gly Thr Tyr Phe
                165                 170                 175

Leu Tyr Ser Lys Leu Thr Val Asp Thr Asp Ser Trp Leu Gln Gly Glu
            180                 185                 190

Ile Phe Thr Cys Ser Val Val His Glu Ala Leu His Asn His His Thr
        195                 200                 205

Gln Lys Asn Leu Ser Arg Ser Pro Gly Lys
    210                 215

<210> 9
<211> 110
<212> PRT
<213> Human

<400> 9

Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys
            100                 105                 110

<210>  10
<211>  107
<212>  PRT
<213>  Human

<400>  10

Gly Gln Pro Arg Glu Pro Gln Val Tyr Thr Leu Pro Pro Ser Arg Glu
1               5                   10                  15

Glu Met Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly Lys
            100                 105

<210>  11
<211>  330
<212>  PRT
<213>  Human

<400>  11

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  12
<211>  330
<212>  PRT
<213>  Human

<400>  12

<210>  13
<211>  31
<212>  PRT
<213>  Human

<400>  13

Ala Pro Glu Leu Leu Gly Gly Pro Ser Val Phe Leu Phe Pro Pro Lys
1               5                   10                  15

His Lys Asp Thr Leu Met Ile Ser Arg Thr Pro Glu Val Thr Cys
            20                  25                  30

<210>  14
<211>  31
<212>  PRT
<213>  Human

<400>  14

Ala Pro Glu Leu Leu Gly Gly Pro Ser Val Phe Leu Phe Pro Pro Lys
1               5                   10                  15

Ile Lys Asp Thr Leu Met Ile Ser Arg Thr Pro Glu Val Thr Cys
            20                  25                  30

<210>  15
<211>  31
<212>  PRT
<213>  Human

<400>  15

Ala Pro Glu Leu Leu Gly Gly Pro Ser Val Phe Leu Phe Pro Pro Lys
1               5                   10                  15

Leu Lys Asp Thr Leu Met Ile Ser Arg Thr Pro Glu Val Thr Cys
            20                  25                  30

<210>  16
<211>  31
<212>  PRT
<213>  Human

<400>  16

Ala Pro Glu Leu Leu Gly Gly Pro Ser Val Phe Leu Phe Pro Pro Lys
1               5                   10                  15

Pro Lys Asp Thr Phe Met Ile Ser Arg Thr Pro Glu Val Thr Cys
            20                  25                  30

<210>  17
<211>  31
<212>  PRT
<213>  Human

<400>  17

Ala Pro Glu Leu Leu Gly Gly Pro Ser Val Phe Leu Phe Pro Pro Lys
1               5                   10                  15

Pro Lys Asp Thr Leu Met Ile Ser Arg Met Pro Glu Val Thr Cys
            20                  25                  30

<210>  18
<211>  31
<212>  PRT
<213>  Human

<400>  18

Ala Pro Glu Leu Leu Gly Gly Pro Ser Val Phe Leu Phe Pro Pro Lys

1               5                   10                  15

Pro Lys Asp Thr Leu Met Ile Ser Arg Pro Pro Glu Val Thr Cys
            20                  25                  30

<210>  19
<211>  29
<212>  PRT
<213>  Human

<400>  19

Val Val Val Asp Val Ser Asp Glu Asp Pro Glu Val Lys Phe Asn Trp
1               5                   10                  15

Tyr Val Asp Gly Val Glu Val His Asn Ala Lys Thr Lys
            20                  25

<210>  20
<211>  29
<212>  PRT
<213>  Human

<400>  20

Val Val Val Asp Val Ser Glu Glu Asp Pro Glu Val Lys Phe Asn Trp
1               5                   10                  15

Tyr Val Asp Gly Val Glu Val His Asn Ala Lys Thr Lys
            20                  25

<210>  21
<211>  29
<212>  PRT
<213>  Human

<400>  21

Val Val Val Asp Val Ser His Glu Asp Pro Glu Val Lys Phe Asn Trp
1               5                   10                  15

Tyr Val Ala Gly Val Glu Val His Asn Ala Lys Thr Lys
            20                  25

<210>  22
<211>  29
<212>  PRT
<213>  Human

<400>  22

Val Val Val Asp Val Ser His Glu Asp Pro Glu Val Lys Phe Asn Trp
1               5                   10                  15

Tyr Val Lys Gly Val Glu Val His Asn Ala Lys Thr Lys
            20                  25

<210>  23
<211>  30
<212>  PRT
<213>  Human

<400>  23

Cys Lys Val Ser Asn Lys Ala Leu Pro Lys Pro Ile Glu Lys Thr Ile
1               5                   10                  15

Ser Lys Ala Lys Gly Gln Pro Arg Glu Pro Gln Val Tyr Thr
            20                  25                  30

<210>  24
<211>  30
<212>  PRT
<213>  Human

<400>  24

Cys Lys Val Ser Asn Lys Ala Leu Pro Arg Pro Ile Glu Lys Thr Ile
1               5                   10                  15

Ser Lys Ala Lys Gly Gln Pro Arg Glu Pro Gln Val Tyr Thr
            20                  25                  30

<210>  25
<211>  30
<212>  PRT
<213>  Human

<400>  25

Cys Lys Val Ser Asn Lys Ala Leu Pro Ala Pro Asp Glu Lys Thr Ile
1               5                   10                  15

Ser Lys Ala Lys Gly Gln Pro Arg Glu Pro Gln Val Tyr Thr
            20                  25                  30

<210>  26
<211>  30
<212>  PRT
<213>  Human

<400>  26

Cys Lys Val Ser Asn Lys Ala Leu Pro Ala Pro Glu Glu Lys Thr Ile
1               5                   10                  15

Ser Lys Ala Lys Gly Gln Pro Arg Glu Pro Gln Val Tyr Thr
            20                  25                  30

<210>  27
<211>  30
<212>  PRT
<213>  Human

<400>  27

Cys Lys Val Ser Asn Lys Ala Leu Pro Ala Pro Lys Glu Lys Thr Ile
1               5                   10                  15

Ser Lys Ala Lys Gly Gln Pro Arg Glu Pro Gln Val Tyr Thr
            20                  25                  30

<210>  28
<211>  30
<212>  PRT
<213>  Human

<400>  28

Cys Lys Val Ser Asn Lys Ala Leu Pro Ala Pro Arg Glu Lys Thr Ile
1               5                   10                  15

Ser Lys Ala Lys Gly Gln Pro Arg Glu Pro Gln Val Tyr Thr
            20                  25                  30

<210>  29
<211>  30
<212>  PRT
<213>  Human

<400>  29

Cys Lys Val Ser Asn Lys Ala Leu Pro Ala Pro Ile Glu Lys Thr Ile
1               5                   10                  15

Ser Lys Thr Lys Gly Gln Pro Arg Glu Pro Gln Val Tyr Thr
            20                  25                  30

<210>  30
<211>  22
<212>  PRT
<213>  Human

<400>  30

Cys Leu Val Lys Gly Phe Tyr Pro Ser Asp Ile Asp Val Glu Trp Glu
1               5                   10                  15

Ser Asn Gly Gln Pro Glu
            20

<210>  31
<211>  23
<212>  PRT
<213>  Human

<400>  31

Cys Ser Val Met His Glu Ala Leu His Asn His Tyr Thr Gln Lys Tyr
1               5                   10                  15

Leu Ser Leu Ser Pro Gly Lys
            20

<210>  32
<211>  990
<212>  DNA
<213>  HUMAN

<400>  32
gcctccacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa ggttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacaac     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggacgag     720

ctgaccaaga accaggtcag cctgacctgc ctggtcaaag gcttctatcc cagcgacatc     780

gccgtggagt gggagagcaa tgggcagccg gagaacaact acaagaccac gcctcccgtg     840

ctggactccg acggctcctt cttcctctat agcaagctca ccgtggacaa gagcaggtgg     900

cagcagggga acgtcttctc atgctccgtg atgcatgagg ctctgcacaa ccactacacg     960

cagaagagcc tctccctgtc tccgggtaaa                                      990

<210>  33

<211>  93
<212>  DNA
<213>  HUMAN

<400>  33
gcacctgaac tcctgggggg accgtcagtc ttcctcttcc ccccaaaacc caaggacacc     60

ctcatgatct cccggacccc tgaggtcaca tgc                                  93


<210>  34
<211>  87
<212>  DNA
<213>  HUMAN

<400>  34
gtggtggtgg acgtgagcca cgaagaccct gaggtcaagt tcaactggta cgtggacggc     60

gtggaggtgc ataatgccaa gacaaag                                         87


<210>  35
<211>  90
<212>  DNA
<213>  HUMAN

<400>  35
tgcaaggtct ccaacaaagc cctcccagcc cccatcgaga aaaccatctc caaagccaaa     60

gggcagcccc gagaaccaca ggtgtacacc                                      90


<210>  36
<211>  69
<212>  DNA
<213>  HUMAN

<400>  36
tgctccgtga tgcatgaggc tctgcacaac cactacacgc agaagagcct ctccctgtct     60

ccgggtaaa                                                             69


<210>  37
<211>  66
<212>  DNA
<213>  HUMAN

<400>  37
tgcctggtca aaggcttcta tcccagcgac atcgccgtgg agtgggagag caatgggcag     60

ccggag                                                                66


<210>  38
<211>  93
<212>  DNA
<213>  HUMAN

<400>  38
gcacctgaac tcctgggggg accgtcagtc ttcctcttcc ccccaaaaca caaggacacc     60

ctcatgatct cccggacccc tgaggtcaca tgc                                93

<210>  39
<211>  93
<212>  DNA
<213>  HUMAN

<400>  39
gcacctgaac tcctgggggg accgtcagtc ttcctcttcc ccccaaaaat caaggacacc      60

ctcatgatct cccggacccc tgaggtcaca tgc                                93

<210>  40
<211>  93
<212>  DNA
<213>  HUMAN

<400>  40
gcacctgaac tcctgggggg accgtcagtc ttcctcttcc ccccaaaact gaaggacacc      60

ctcatgatct cccggacccc tgaggtcaca tgc                                93

<210>  41
<211>  93
<212>  DNA
<213>  HUMAN

<400>  41
gcacctgaac tcctgggggg accgtcagtc ttcctcttcc ccccaaaacc caaggacacc      60

ttcatgatct cccggacccc tgaggtcaca tgc                                93

<210>  42
<211>  93
<212>  DNA
<213>  HUMAN

<400>  42
gcacctgaac tcctgggggg accgtcagtc ttcctcttcc ccccaaaacc caaggacacc      60

ctcatgatct cccggatgcc tgaggtcaca tgc                                93

<210>  43
<211>  93
<212>  DNA
<213>  HUMAN

<400>  43
gcacctgaac tcctgggggg accgtcagtc ttcctcttcc ccccaaaacc caaggacacc      60

ctcatgatct cccggccacc tgaggtcaca tgc                                93

<210>  44
<211>  87

<212>  DNA
<213>  HUMAN

<400>  44
gtggtggtgg acgtgagcga cgaagaccct gaggtcaagt tcaactggta cgtggacggc      60

gtggaggtgc ataatgccaa gacaaag                                          87


<210>  45
<211>  87
<212>  DNA
<213>  HUMAN

<400>  45
gtggtggtgg acgtgagcga ggaagaccct gaggtcaagt tcaactggta cgtggacggc      60

gtggaggtgc ataatgccaa gacaaag                                          87


<210>  46
<211>  87
<212>  DNA
<213>  HUMAN

<400>  46
gtggtggtgg acgtgagcca cgaagaccct gaggtcaagt tcaactggta cgtggccggc      60

gtggaggtgc ataatgccaa gacaaag                                          87


<210>  47
<211>  87
<212>  DNA
<213>  HUMAN

<400>  47
gtggtggtgg acgtgagcca cgaagaccct gaggtcaagt tcaactggta cgtgaaaggc      60

gtggaggtgc ataatgccaa gacaaag                                          87


<210>  48
<211>  90
<212>  DNA
<213>  HUMAN

<400>  48
tgcaaggtct ccaacaaagc cctcccaaaa cccatcgaga aaaccatctc caaagccaaa      60

gggcagcccc gagaaccaca ggtgtacacc                                       90


<210>  49
<211>  90
<212>  DNA
<213>  HUMAN

<400>  49
tgcaaggtct ccaacaaagc cctcccacgc cccatcgaga aaaccatctc caaagccaaa      60

gggcagcccc gagaaccaca ggtgtacacc                                    90


<210>  50
<211>  90
<212>  DNA
<213>  HUMAN

<400>  50
tgcaaggtct ccaacaaagc cctcccagcc cccgacgaga aaaccatctc caaagccaaa      60

gggcagcccc gagaaccaca ggtgtacacc                                    90


<210>  51
<211>  90
<212>  DNA
<213>  HUMAN

<400>  51
tgcaaggtct ccaacaaagc cctcccagcc cccgaggaga aaaccatctc caaagccaaa      60

gggcagcccc gagaaccaca ggtgtacacc                                    90


<210>  52
<211>  90
<212>  DNA
<213>  HUMAN

<400>  52
tgcaaggtct ccaacaaagc cctcccagcc cccaaagaga aaaccatctc caaagccaaa      60

gggcagcccc gagaaccaca ggtgtacacc                                    90


<210>  53
<211>  90
<212>  DNA
<213>  HUMAN

<400>  53
tgcaaggtct ccaacaaagc cctcccagcc ccccgcgaga aaaccatctc caaagccaaa      60

gggcagcccc gagaaccaca ggtgtacacc                                    90


<210>  54
<211>  90
<212>  DNA
<213>  HUMAN

<400>  54
tgcaaggtct ccaacaaagc cctcccagcc cccatcgaga aaaccatctc caaaaccaaa      60

gggcagcccc gagaaccaca ggtgtacacc                                    90


<210>  55
<211>  66
<212>  DNA

<213>  HUMAN

<400>  55
tgcctggtca aaggcttcta tcccagcgac atcgacgtgg agtgggagag caatgggcag      60

ccggag                                                                  66


<210>  56
<211>  69
<212>  DNA
<213>  HUMAN

<400>  56
tgctccgtga tgcatgaggc tctgcacaac cactacacgc agaagtacct ctccctgtct      60

ccgggtaaa                                                               69